# EUROPEAN PATENT APPLICATION

(11) **EP 4 617 272 A1**
(43) Date of publication of application: **17.09.2025**
(21) Application number: 23888071.0
(22) Date of filing: 09.11.2023
(51) Int. Cl.: C07D 487/04, C07D 513/04, A61K 31/4985, A61K 31/498, A61K 31/429, A61P 35/00

(54) **FUSED BICYCLIC COMPOUND**

(30) Priority: 10.11.2022 CN 202211404183; 30.06.2023 CN 202310799109; 01.11.2023 CN 202311448787
(71) Applicant: Chia Tai Tianqing Pharmaceutical Group Co., Ltd., Lianyungang, Jiangsu 222062 (CN)
(72) Inventor: LIU, Xin, Lianyungang, Jiangsu 222062 (CN); QIN, Hui, Lianyungang, Jiangsu 222062 (CN); LI, Ke, Lianyungang, Jiangsu 222062 (CN); CHEN, Puzhou, Lianyungang, Jiangsu 222062 (CN); ZHANG, Yinsheng, Lianyungang, Jiangsu 222062 (CN)
(74) Representative: Grünecker Patent- und Rechtsanwälte PartG mbB
(86) International application number: PCT/CN2023/130659
(87) International publication number: WO 2024/099386

(57) **Abstract**

The present application relates to the field of pharmaceutical chemistry, relates to a fused bicyclic compound, and in particular relates to a compound represented by formula (II), a stereoisomer or a pharmaceutically acceptable salt thereof, a preparation method therefor, a pharmaceutical composition containing the compound, and a use thereof in treating diseases.

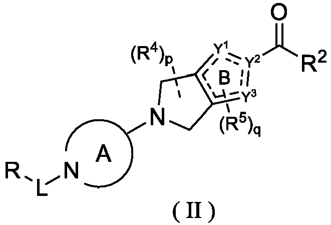

## Description

### CROSS-REFERENCE TO RELATED APPLICATION

The present application claims the benefit and priority to the Chinese Patent Application Nos. 202211404183.4, 202310799109.5, and 202311448787.3 filed with National Intellectual Property Administration, PRC on November 10, 2022, June 30, 2023, and November 1, 2023, which are incorporated herein in their entireties.

### TECHNICAL FIELD

The present application relates to a fused bicyclic compound, a preparation method therefor, a pharmaceutical composition containing the compound, and use thereof for treating a disease.

### BACKGROUND

Poly(ADP-ribose) polymerase (PARP) is a class of nuclear enzymes that catalyze ADP-ribosylation. The PARP family consists of 18 members and plays a crucial role in a wide range of cellular metabolic processes, including DNA damage repair, inflammation regulation, transcriptional regulation, signal transduction, genomic stability, cell cycle regulation, and mitosis. PARP1 is the most significant PARP enzyme, accounting for 85%-90% of total intracellular PARP activity, and is primarily involved in DNA damage repair. PARP inhibitors can selectively kill tumor cells with homologous recombination repair (HR) deficiencies caused by BRCA gene defects while sparing normal cells with intact BRCA function, a phenomenon known as synthetic lethality.

Since the approval of olaparib in 2014, multiple PARP inhibitors have been developed and have achieved widespread success. However, adverse effects of the drugs have limited their ability to be combined with chemotherapeutic drugs. Therefore, PARP inhibitors with enhanced PARP1 selectivity may offer improved efficacy and reduced toxicity.

### SUMMARY

In one aspect, the present application relates to a compound of formula (II), a stereoisomer thereof, or a pharmaceutically acceptable salt thereof, 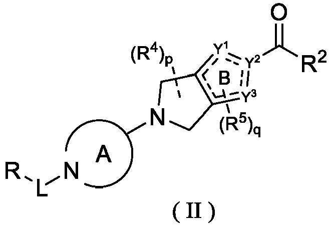 wherein,
R is selected from the group consisting of 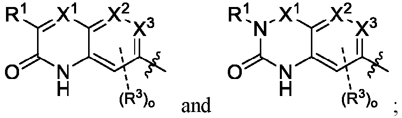
X¹ is selected from the group consisting of CR^{a}, CHR^{a}, N, and NR^{a};
X² is selected from the group consisting of CH and N;
X³ is selected from the group consisting of CH and N;
R¹ is selected from the group consisting of halogen, C₁₋₆ alkyl, C₁₋₆ alkoxy, C₃₋₈ cycloalkyl, and 3-8 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R^{a} is selected from the group consisting of H, halogen, and C₁₋₆ alkyl;
or R^{a} and R¹ are linked to each other to form 5-7 membered heterocycloalkyl, 5-7 membered cycloalkenyl, phenyl, 5-7 membered heterocycloalkenyl, or 5-6 membered heteroaryl;
R² is selected from the group consisting of C₁₋₆ alkyl, -OH, -OC₁₋₆ alkyl, -OC₃₋₆ cycloalkyl, -SH, -SC₁₋₆ alkyl, -SC₃₋₆ cycloalkyl, -NH₂, -NH(C₁₋₆ alkyl), -NH(C₃₋₆ cycloalkyl), -NH(3-8 membered heterocycloalkyl), -N(C₁₋₆ alkyl)₂, -N(C₁₋₆ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₆ alkyl, -SH, -SC₁₋₆ alkyl, -NH₂,
-NH(C₁₋₆ alkyl), -N(C₁₋₆ alkyl)₂, C₃₋₆ cycloalkyl, and 3-8 membered heterocycloalkyl;
R³, R⁴, and R⁵ are each independently selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂, wherein the C₁₋₆ alkyl, -OC₁₋₆ alkyl, -SC₁₋₆ alkyl, -NH(C₁₋₆ alkyl), or -N(C₁₋₆ alkyl)₂ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂; o, p, and q are each independently selected from the group consisting of 0, 1, and 2;
L is selected from the group consisting of -NH- and -CH₂-, wherein L is optionally substituted with one or more groups selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂;
ring A is selected from the group consisting of 3-8 membered heterocycloalkyl, wherein ring A optionally contains 1-3 heteroatoms independently selected from the group consisting of N, O, and S in addition to the N atom linked to L, and ring A is optionally substituted with one or more groups selected from the group consisting of D, halogen, -OH, -C₁₋₆ alkyl, -OC₁₋₆ alkyl, -SH, -SC₁₋₆ alkyl, -NH₂, -NH(C₁₋₆ alkyl), -N(C₁₋₆ alkyl)₂, -C₁₋₄ alkylene-OC₁₋₆ alkyl, -C₁₋₄ alkylene-SC₁₋₆ alkyl, -C₁₋₄ alkylene-NH(C₁₋₆ alkyl), and -C₁₋₄ alkylene-N(C₁₋₆ alkyl)₂; ring B is selected from the group consisting of an aromatic ring or a partially saturated ring;
Y¹, Y², and Y³ are each independently selected from the group consisting of C, CH, N, O, and S.

In some embodiments, the compound of formula (II) is selected from the group consisting of a compound of formula (I), a stereoisomer thereof, or a pharmaceutically acceptable salt thereof, 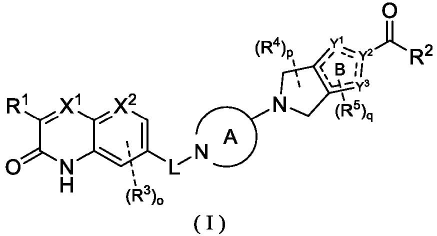 wherein,
X¹ is selected from the group consisting of CR^{a} and N;
X² is selected from the group consisting of CH and N;
R¹ is selected from the group consisting of C₁₋₆ alkyl, C₃₋₈ cycloalkyl, and 3-8 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R^{a} is selected from the group consisting of H, halogen, and C₁₋₆ alkyl;
or R^{a} and R¹ are linked to each other to form 5-7 membered cycloalkenyl, or 5-7 membered heterocycloalkenyl containing 1-3 heteroatoms independently selected from the group consisting of N, O, and S;
R² is selected from the group consisting of C₁₋₆ alkyl, -OH, -OC₁₋₆ alkyl, -OC₃₋₆ cycloalkyl, -SH, -SC₁₋₆ alkyl, -SC₃₋₆ cycloalkyl, -NH₂, -NH(C₁₋₆ alkyl), -NH(C₃₋₆ cycloalkyl), -N(C₁₋₆ alkyl)₂, -N(C₁₋₆ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R³, R⁴, and R⁵ are each independently selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂, wherein the C₁₋₆ alkyl, -OC₁₋₆ alkyl, -SC₁₋₆ alkyl, -NH(C₁₋₆ alkyl), or -N(C₁₋₆ alkyl)₂ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂; o, p, and q are each independently selected from the group consisting of 0, 1, and 2;
L is selected from the group consisting of -NH- and -CH₂-, wherein L is optionally substituted with one or more groups selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂;
ring A is selected from the group consisting of 3-8 membered heterocycloalkyl, wherein ring A optionally contains 1-3 heteroatoms independently selected from the group consisting of N, O, and S in addition to the N atom linked to L, and ring A is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
ring B is selected from the group consisting of an aromatic ring or a partially saturated ring;
Y¹, Y², and Y³ are each independently selected from the group consisting of C, N, O, and S.

In some embodiments, the compound of formula (II) is selected from the group consisting of a compound of formula (I), a stereoisomer thereof, or a pharmaceutically acceptable salt thereof, 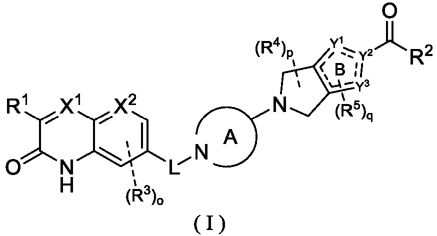 wherein,
X¹ is selected from the group consisting of CR^{a} and N;
X² is selected from the group consisting of CH and N;
R¹ is selected from the group consisting of halogen, C₁₋₆ alkyl, C₁₋₆ alkoxy, C₃₋₈ cycloalkyl, and 3-8 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R^{a} is selected from the group consisting of H, halogen, and C₁₋₆ alkyl;
or R^{a} and R¹ are linked to each other to form 5-7 membered cycloalkenyl, phenyl, 5-7 membered heterocycloalkenyl, or 5-6 membered heteroaryl;
R² is selected from the group consisting of C₁₋₆ alkyl, -OH, -OC₁₋₆ alkyl, -OC₃₋₆ cycloalkyl, -SH, -SC₁₋₆ alkyl, -SC₃₋₆ cycloalkyl, -NH₂, -NH(C₁₋₆ alkyl), -NH(C₃₋₆ cycloalkyl), -NH(3-8 membered heterocycloalkyl), -N(C₁₋₆ alkyl)₂, -N(C₁₋₆ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R³, R⁴, and R⁵ are each independently selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂, wherein the C₁₋₆ alkyl, -OC₁₋₆ alkyl, -SC₁₋₆ alkyl, -NH(C₁₋₆ alkyl), or -N(C₁₋₆ alkyl)₂ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂; o, p, and q are each independently selected from the group consisting of 0, 1, and 2;
L is selected from the group consisting of -NH- and -CH₂-, wherein L is optionally substituted with one or more groups selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂;
ring A is selected from the group consisting of 3-8 membered heterocycloalkyl, wherein ring A optionally contains 1-3 heteroatoms independently selected from the group consisting of N, O, and S in addition to the N atom linked to L, and ring A is optionally substituted with one or more groups selected from the group consisting of D, halogen, -OH, -C₁₋₆ alkyl, -OC₁₋₆ alkyl, -SH, -SC₁₋₆ alkyl, -NH₂, -NH(C₁₋₆ alkyl), and -N(C₁₋₆ alkyl)₂;
ring B is selected from the group consisting of an aromatic ring or a partially saturated ring;
Y¹, Y², and Y³ are each independently selected from the group consisting of C, CH, N, O, and S.

In some embodiments, X¹ is selected from the group consisting of CR^{a} and N.

In other embodiments, X¹ is selected from the group consisting of CHR^{a} and NR^{a}.

In other embodiments, X¹ is selected from the group consisting of CHR^{a}.

In some embodiments, X¹ is selected from the group consisting of CR^{a}, and X² is selected from the group consisting of CH.

In some embodiments, X¹ is selected from the group consisting of CH, and X² is selected from the group consisting of N.

In some embodiments, X¹ is selected from the group consisting of N, and X² is selected from the group consisting of CH.

In some embodiments, X¹ is selected from the group consisting of NR^{a}, and X² is selected from the group consisting of CH.

In some embodiments, X³ is selected from the group consisting of CH.

In some embodiments, X¹ is selected from the group consisting of CR^{a}, X² is selected from the group consisting of CH, and X³ is selected from the group consisting of CH.

In some embodiments, X¹ is selected from the group consisting of CH, X² is selected from the group consisting of N, and X³ is selected from the group consisting of CH.

In some embodiments, X¹ is selected from the group consisting of N, X² is selected from the group consisting of CH, and X³ is selected from the group consisting of CH.

In some embodiments, X¹ is selected from the group consisting of CH, X² is selected from the group consisting of CH, and X³ is selected from the group consisting of N.

In some embodiments, X¹ is selected from the group consisting of NR^{a}, X² is selected from the group consisting of CH, and X³ is selected from the group consisting of CH.

In some embodiments, R¹ is selected from the group consisting of halogen, C₁₋₄ alkyl, C₁₋₄ alkoxy, C₃₋₆ cycloalkyl, and 3-6 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R¹ is selected from the group consisting of C₁₋₄ alkyl, C₃₋₆ cycloalkyl, and 3-6 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂.

In some embodiments, R¹ is selected from the group consisting of halogen, C₁₋₄ alkyl, and C₃₋₆ cycloalkyl, wherein R¹ is optionally substituted with one or more groups selected from the group consisting of D, halogen, -OH, -OC₁₋₆ alkyl, -SH, -SC₁₋₃ alkyl, -NH₂, -NH(C₁₋₃ alkyl), and -N(C₁₋₃ alkyl)₂.

In some embodiments, R¹ is selected from the group consisting of halogen, C₁₋₄ alkyl, and C₃₋₆ cycloalkyl, wherein R¹ is optionally substituted with one or more groups selected from the group consisting of D and halogen.

In some embodiments, R¹ is selected from the group consisting of halogen, C₁₋₃ alkyl, and C₃₋₆ cycloalkyl, wherein R¹ is optionally substituted with one or more groups selected from the group consisting of halogen.

In some embodiments, R¹ is selected from the group consisting of methyl, ethyl, propyl, cyclopropyl, cyclobutyl, cyclopentyl, cyclohexyl, and 3-6 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R¹ is selected from the group consisting of halogen. In some embodiments, R¹ is selected from the group consisting of methyl, ethyl, propyl, cyclopropyl, cyclobutyl, cyclopentyl, cyclohexyl, and 3-6 membered heterocycloalkyl containing 1-3 heteroatoms independently selected from the group consisting of N, O, and S, wherein R¹ is optionally substituted with one or more groups selected from the group consisting of D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂. In some embodiments, R¹ is selected from the group consisting of halogen. In some embodiments, R¹ is selected from the group consisting of methyl, ethyl, propyl, cyclopropyl, cyclobutyl, cyclopentyl, cyclohexyl, oxetanyl, azetidinyl, thietanyl, tetrahydropyrrolyl, tetrahydrofuranyl, tetrahydrothienyl, piperidinyl, tetrahydropyranyl, tetrahydrothiopyranyl, morpholinyl, and piperazinyl, wherein R¹ is optionally substituted with one or more groups selected from the group consisting of D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂. In some embodiments, R¹ is selected from the group consisting of F, Cl, Br, and I.

In some embodiments, R¹ is selected from the group consisting of methyl, ethyl, propyl, cyclopropyl, cyclobutyl, oxetanyl, azetidinyl, tetrahydropyrrolyl, tetrahydrofuranyl, and tetrahydropyranyl, wherein R¹ is optionally substituted with one or more groups selected from the group consisting of D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂. In some embodiments, R¹ is selected from the group consisting of F, Cl, Br, and I.

In some embodiments, R¹ is selected from the group consisting of methyl, ethyl, propyl, 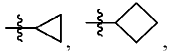 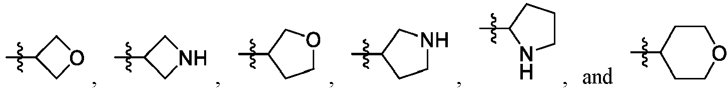 wherein R¹ is optionally substituted with one or more groups selected from the group consisting of D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂. In some embodiments, R¹ is selected from the group consisting of Cl.

In some embodiments, R¹ is selected from the group consisting of Cl, methyl, ethyl, propyl, 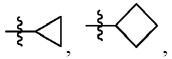 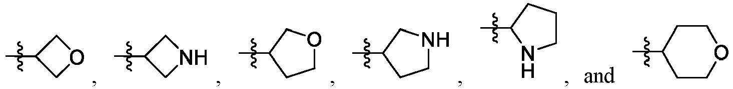 wherein R¹ is optionally substituted with one or more groups selected from the group consisting of D, F, Cl, and Br.

In some embodiments, R¹ is selected from the group consisting of Cl, methyl, ethyl, propyl, 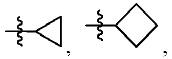 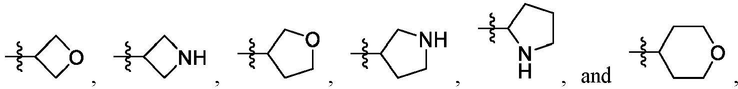 wherein R¹ is optionally substituted with one or more F.

In some embodiments, R¹ is selected from the group consisting of chloro, methyl, ethyl, propyl, trifluoromethyl,

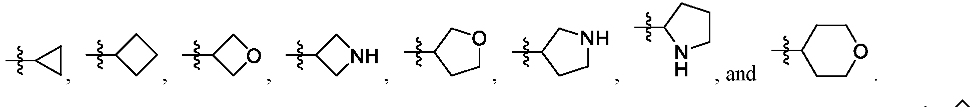

In some embodiments, R¹ is selected from the group consisting of methyl, ethyl, propyl,

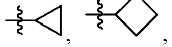

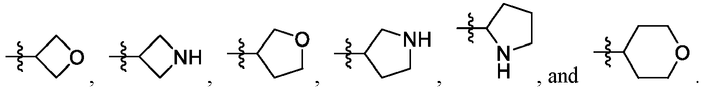

In some embodiments, R¹ is selected from the group consisting of chloro, methyl, ethyl, trifluoromethyl, and

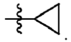

In some embodiments, R¹ is selected from the group consisting of ethyl and

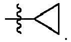

In some embodiments, R^{a} is selected from the group consisting of H, F, Cl, Br, and C₁₋₄ alkyl.

In some embodiments, R^{a} is selected from the group consisting of H, F, Cl, and C₁₋₃ alkyl.

In some embodiments, R^{a} is selected from the group consisting of H, F, Cl, methyl, ethyl, and propyl.

In other embodiments, R^{a} is selected from the group consisting of H and methyl.

In some embodiments, R^{a} is selected from the group consisting of H and F.

In some embodiments, R^{a} is selected from the group consisting of H.

In some embodiments, R^{a} and R¹ are linked to each other to form 5-6 membered heterocycloalkyl, 5-6 membered cycloalkenyl, phenyl, or 5-6 membered heterocycloalkenyl or 5-6 membered heteroaryl containing 1-3 heteroatoms independently selected from the group consisting of N, O, and S.

In some embodiments, R^{a} and R¹ are linked to each other to form 5-6 membered cycloalkenyl, phenyl, or 5-6 membered heterocycloalkenyl or 5-6 membered heteroaryl containing 1-3 heteroatoms independently selected from the group consisting of N, O, and S.

In some embodiments, R^{a} and R¹ are linked to each other to form 5-6 membered cycloalkenyl, or 5-membered heterocycloalkenyl or 5-membered heteroaryl containing 1 heteroatom independently selected from the group consisting of N and O.

In some embodiments, R^{a} and R¹ are linked to each other to form 5-6 membered cycloalkenyl, or 5-6 membered heterocycloalkenyl containing 1-3 heteroatoms independently selected from the group consisting of N, O, and S. In some embodiments, R^{a} and R¹ are linked to each other to form 5-6 membered cycloalkenyl, or 5-membered heterocycloalkenyl containing 1 heteroatom independently selected from the group consisting of N and O.

In other embodiments, R^{a} and R¹ are linked to each other to form 5-6 membered heteroaryl containing 1-3 heteroatoms independently selected from the group consisting of N, O, and S.

In other embodiments, R^{a} and R¹ are linked to each other to form 5-membered heteroaryl containing 1-3 heteroatoms independently selected from the group consisting of N, O, and S.

In other embodiments, R^{a} and R¹ are linked to each other to form 5-membered heteroaryl containing 1-2 N atoms.

In some embodiments, R^{a} and R¹ are linked to each other to form cyclopentenyl, cyclohexenyl, dihydrofuranyl, furanyl, pyrrolyl, pyrazolyl, imidazolyl, thienyl, or thiazolyl.

In some embodiments, R^{a} and R¹ are linked to each other to form cyclopentenyl, cyclohexenyl, or dihydrofuranyl.

In other embodiments, R is selected from the group consisting of

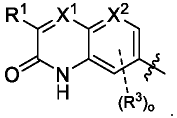

In other embodiments, R is selected from the group consisting of

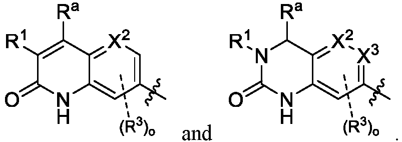

In other embodiments, R is selected from the group consisting of and

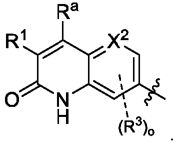

In other embodiments, R is selected from the group consisting of 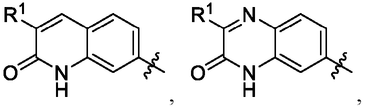 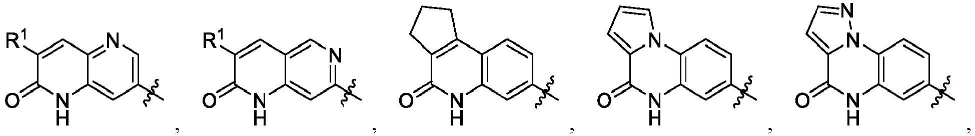 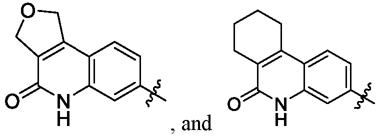 wherein R is substituted with 0, 1, or 2 R³.

In other embodiments, R is selected from the group consisting of 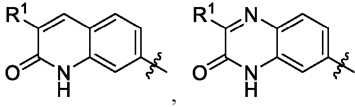 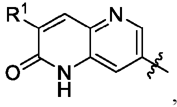 wherein R is substituted with 0, 1, or 2 R³

In other embodiments, R is selected from the group consisting of . . 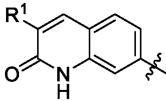 wherein R is substituted with 0, 1, or 2 R³

In some embodiments, R is selected from the group consisting of 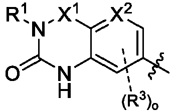 In some embodiments, R is selected from the group consisting of

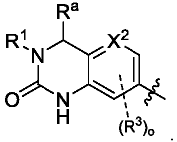

In some embodiments, R is selected from the group consisting of 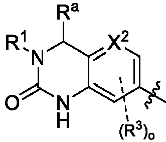 and R^{a} and R¹ are linked to each other to form 5-6 membered heteroaryl containing 1-3 heteroatoms independently selected from the group consisting of N, O, and S.

In some embodiments, R is selected from the group consisting of 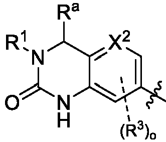 and R^{a} and R¹ are linked to each other to form 5-membered heteroaryl containing 1-2 N atoms.

In some embodiments, R is selected from the group consisting of 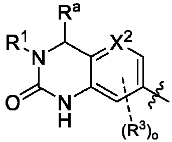 and R^{a} and R¹ are linked to each other to form pyrrolyl, pyrazolyl, or imidazolyl.

In some embodiments, R is selected from the group consisting of 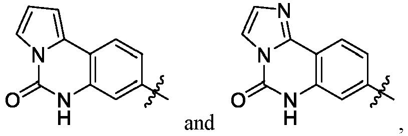 wherein R is substituted with 0, 1, or 2 R³.

In some embodiments, R is selected from the group consisting of 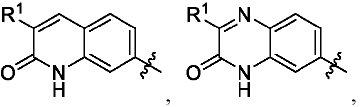 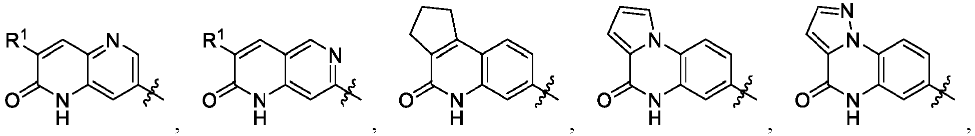 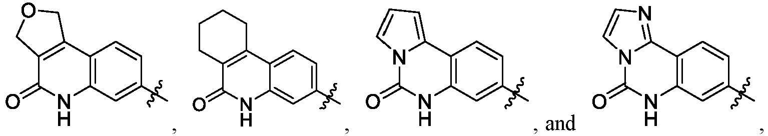 and R is substituted with 0, 1, or 2 R³.

In some embodiments, R is selected from the group consisting of 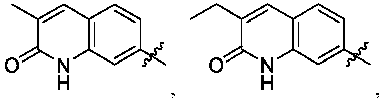 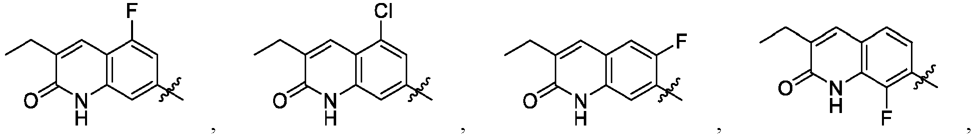 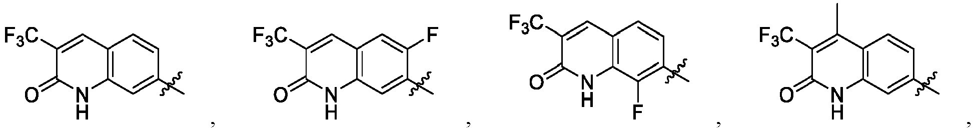 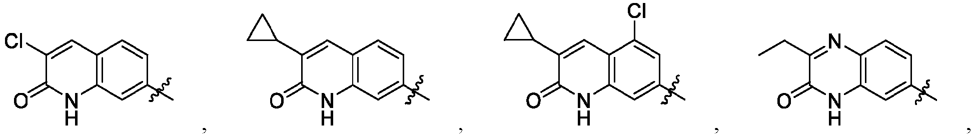 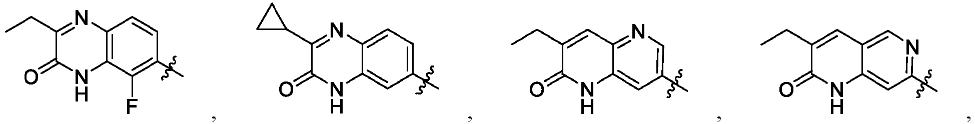 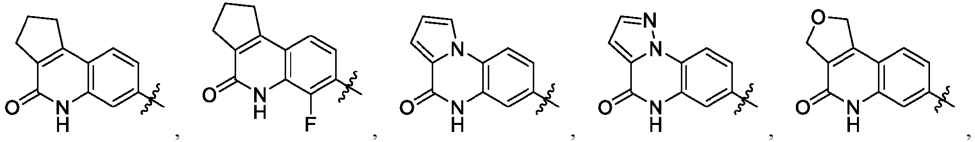 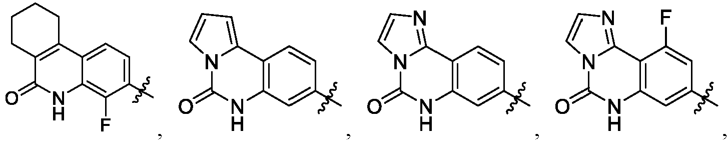 and

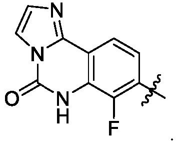

In some embodiments, R is selected from the group consisting of

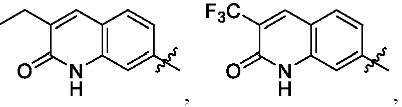

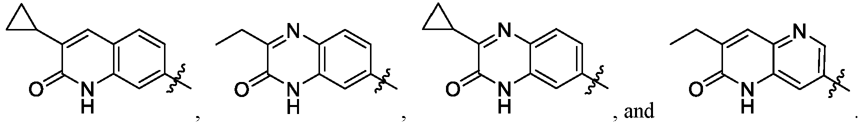

In some embodiments, R is selected from the group consisting of 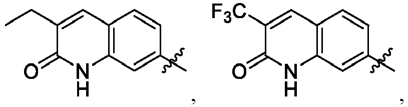 and

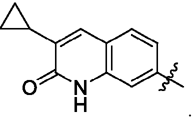

In other embodiments, R² is selected from the group consisting of C₁₋₄ alkyl, -OH, -OC₁₋₄ alkyl, -OC₃₋₆ cycloalkyl, -SH, -SC₁₋₄ alkyl, -SC₃₋₆ cycloalkyl, -NH₂, -NH(C₁₋₄ alkyl), -NH(C₃₋₆ cycloalkyl), -NH(3-8 membered heterocycloalkyl), -N(C₁₋₄ alkyl)₂, -N(C₁₋₄ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-8 membered heterocycloalkyl.

In some embodiments, R² is selected from the group consisting of C₁₋₄ alkyl, -OH, -OC₁₋₄ alkyl, -OC₃₋₆ cycloalkyl, -SH, -SC₁₋₄ alkyl, -SC₃₋₆ cycloalkyl, -NH₂, -NH(C₁₋₄ alkyl), -NH(C₃₋₆ cycloalkyl), -N(C₁₋₄ alkyl)₂, -N(C₁₋₄ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is selected from the group consisting of -NH(3-6 membered heterocycloalkyl), wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-6 membered heterocycloalkyl.

In some embodiments, R² is selected from the group consisting of C₁₋₄ alkyl, -OH, -OC₁₋₄ alkyl, -SH, -SC₁₋₄ alkyl, -NH₂, -NH(C₁₋₄ alkyl), -NH(C₃₋₆ cycloalkyl), -NH(3-6 membered heterocycloalkyl), and -N(C₁₋₄ alkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-6 membered heterocycloalkyl.

In some embodiments, R² is selected from the group consisting of C₁₋₄ alkyl, -OH, -OC₁₋₄ alkyl, -NH₂, -NH(C₁₋₄ alkyl), -NH(C₃₋₆ cycloalkyl), and -N(C₁₋₄ alkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is selected from the group consisting of -NH(3-6 membered heterocycloalkyl), wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-6 membered heterocycloalkyl.

In some embodiments, R² is selected from the group consisting of -NH₂, -NH(C₁₋₄ alkyl), -NH(C₃₋₆ cycloalkyl), and -N(C₁₋₄ alkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is selected from the group consisting of -NH(3-6 membered heterocycloalkyl), wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂. In some embodiments, R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-6 membered heterocycloalkyl.

In some embodiments, R² is selected from the group consisting of -NH₂, -NH(C₁₋₄ alkyl), -NH(C₃₋₆ cycloalkyl), and -N(C₁₋₄ alkyl)₂, wherein R² is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂. In some embodiments, R² is selected from the group consisting of -NH(3-6 membered heterocycloalkyl), wherein R² is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂. In some embodiments, R² is selected from the group consisting of -NH(C₁₋₄ alkyl), -NH(C₃₋₆ cycloalkyl), and -NH(3-6 membered heterocycloalkyl), wherein R² is optionally substituted with one or more D.

In some embodiments, R² is selected from the group consisting of -NH(C₁₋₄ alkyl), -NH(C₃₋₆ cycloalkyl), and -NH(3-8 membered heterocycloalkyl), wherein R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-6 membered heterocycloalkyl.

In some embodiments, R² is selected from the group consisting of -NH(C₁₋₄ alkyl), -NH(C₃₋₆ cycloalkyl), and -NH(3-6 membered heterocycloalkyl), wherein R² is optionally substituted with one or more groups selected from the group consisting of D, halogen, C₃₋₆ cycloalkyl, and 3-6 membered heterocycloalkyl.

In some embodiments, R² is selected from the group consisting of -NH(C₁₋₄ alkyl), -NH(C₃₋₆ cycloalkyl), and -NH(3-6 membered heterocycloalkyl), wherein R² is optionally substituted with one or more groups selected from the group consisting of D, F, Cl, cyclopropyl, cyclobutyl, cyclopentyl, cyclohexyl, aziridinyl, oxiranyl, azetidinyl, oxetanyl, tetrahydropyrrolyl, and tetrahydrofuranyl.

In some embodiments, R² is selected from the group consisting of -NHCH₃, -NHCH(CH₃)₂, 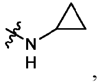 -NHCD₃, -NHCF₃, -NH-O-CH₃, and -NH-N(CH₃)₂. In some embodiments, R² is selected from the group consisting of -NHCH₃, -NHCH₂CH₃, 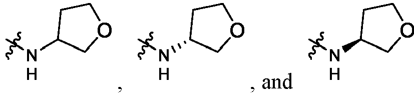 In some embodiments, R² is selected from the group consisting of -NHCH₂CF₃ and

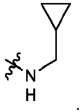

In some embodiments, R² is selected from the group consisting of -NHCH₃, -NHCH(CH₃)₂, 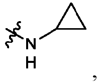 -NHCD₃, -NHCH₂CH₃,

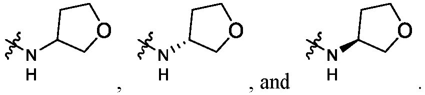

In some embodiments, R² is selected from the group consisting of -NHCH₃, -NHCH(CH₃)₂, 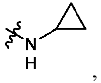 -NHCD₃, -NHCH₂CH₃, -NHCH₂CF₃,

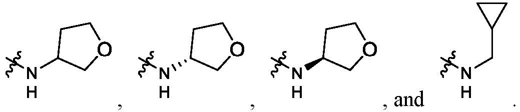

In some embodiments, R² is selected from the group consisting of -NHCH₃, 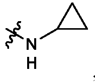 and -NHCH₂CH₃.

In some embodiments, R³ is selected from the group consisting of C₁₋₄ alkyl, D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂, wherein the C₁₋₄ alkyl, -OC₁₋₄ alkyl, -SC₁₋₄ alkyl, -NH(C₁₋₄ alkyl), or -N(C₁₋₄ alkyl)₂ is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂.

In some embodiments, R³ is selected from the group consisting of C₁₋₃ alkyl, D, F, Cl, -OH, -OC₁₋₃ alkyl, -NH₂, -NH(C₁₋₃ alkyl), and -N(C₁₋₃ alkyl)₂, wherein the C₁₋₃ alkyl, -OC₁₋₃ alkyl, -NH(C₁₋₃ alkyl), or -N(C₁₋₃ alkyl)₂ is optionally substituted with one or more groups selected from the group consisting of D, F, Cl, -OH, and -NH₂.

In some embodiments, R³ is selected from the group consisting of methyl, ethyl, propyl, F, and Cl, wherein the methyl, ethyl, or propyl is optionally substituted with one or more groups selected from the group consisting of D, F, Cl, -OH, and -NH₂.

In some embodiments, R³ is selected from the group consisting of methyl, ethyl, and halogen.

In some embodiments, R³ is selected from the group consisting of halogen.

In some embodiments, R³ is selected from the group consisting of F and Cl.

In some embodiments, R³ is selected from the group consisting of methyl, F and Cl.

In some embodiments, R⁴ is selected from the group consisting of C₁₋₄ alkyl, D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂, wherein the C₁₋₄ alkyl, -OC₁₋₄ alkyl, -SC₁₋₄ alkyl, -NH(C₁₋₄ alkyl), or -N(C₁₋₄ alkyl)₂ is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂.

In some embodiments, R⁴ is selected from the group consisting of C₁₋₃ alkyl, D, F, Cl, -OH, -OC₁₋₃ alkyl, -NH₂, -NH(C₁₋₃ alkyl), and -N(C₁₋₃ alkyl)₂, wherein the C₁₋₃ alkyl, -OC₁₋₃ alkyl, -NH(C₁₋₃ alkyl), or -N(C₁₋₃ alkyl)₂ is optionally substituted with one or more groups selected from the group consisting of D, F, Cl, -OH, and -NH₂.

In some embodiments, R⁴ is selected from the group consisting of methyl, ethyl, propyl, F, and Cl, wherein the methyl, ethyl, or propyl is optionally substituted with one or more groups selected from the group consisting of D, F, Cl, -OH, and -NH₂.

In some embodiments, R⁵ is selected from the group consisting of C₁₋₄ alkyl, D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂, wherein the C₁₋₄ alkyl, -OC₁₋₄ alkyl, -SC₁₋₄ alkyl, -NH(C₁₋₄ alkyl), or -N(C₁₋₄ alkyl)₂ is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂.

In some embodiments, R⁵ is selected from the group consisting of C₁₋₃ alkyl, D, F, Cl, -OH, -OC₁₋₃ alkyl, -NH₂, -NH(C₁₋₃ alkyl), and -N(C₁₋₃ alkyl)₂, wherein the C₁₋₃ alkyl, -OC₁₋₃ alkyl, -NH(C₁₋₃ alkyl), or -N(C₁₋₃ alkyl)₂ is optionally substituted with one or more groups selected from the group consisting of D, F, Cl, -OH, and -NH₂.

In some embodiments, R⁵ is selected from the group consisting of methyl, ethyl, propyl, F, and Cl, wherein the methyl, ethyl, or propyl is optionally substituted with one or more groups selected from the group consisting of D, F, Cl, -OH, and -NH₂.

In some embodiments, o is selected from the group consisting of 0, 1, and 2.

In some embodiments, o is selected from the group consisting of 0 and 1.

In some embodiments, p is selected from the group consisting of 0, 1, and 2.

In some embodiments, p is selected from the group consisting of 0 and 1. In some embodiments, p is selected from the group consisting of 0.

In some embodiments, q is selected from the group consisting of 0, 1, and 2.

In some embodiments, q is selected from the group consisting of 0 and 1. In some embodiments, q is selected from the group consisting of 0.

In some embodiments, L is selected from the group consisting of -NH- and -CH₂-, wherein L is optionally substituted with one or more groups selected from the group consisting of C₁₋₄ alkyl, D, F, Cl, -OH, and -NH₂.

In some embodiments, L is selected from the group consisting of -NH- and -CH₂-, wherein L is optionally substituted with one or more groups selected from the group consisting of methyl, D, and F.

In some embodiments, L is selected from the group consisting of -CH₂-.

In some embodiments, ring A is selected from the group consisting of 3-6 membered heterocycloalkyl, wherein ring A optionally contains 1-3 heteroatoms independently selected from the group consisting of N, O, and S in addition to the N atom linked to L, and ring A is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂; or ring A is optionally substituted with one or more groups selected from the group consisting of -C₁₋₆ alkyl; or ring A is optionally substituted with one or more groups selected from the group consisting of -C₁₋₄ alkylene-OC₁₋₄ alkyl, -C₁₋₄ alkylene-SC₁₋₄ alkyl, -C₁₋₄ alkylene-NH(C₁₋₄ alkyl), and -C₁₋₄ alkylene-N(C₁₋₄ alkyl)₂. In some embodiments, ring A is selected from the group consisting of 3-6 membered heterocycloalkyl, wherein ring A optionally contains 1-3 heteroatoms independently selected from the group consisting of N and O in addition to the N atom linked to L, and ring A is optionally substituted with one or more groups selected from the group consisting of D, F, Cl, -OH, -C₁₋₄ alkyl, -OC₁₋₄ alkyl, -SH, -SC₁₋₄ alkyl, -NH₂, -NH(C₁₋₄ alkyl), and -N(C₁₋₄ alkyl)₂; or ring A is optionally substituted with one or more groups selected from the group consisting of -C₁₋₃ alkylene-OC₁₋₄ alkyl, -C₁₋₃ alkylene-NH(C₁₋₄ alkyl) and -C₁₋₃ alkylene-N(C₁₋₄ alkyl)₂.

In some embodiments, ring A is selected from the group consisting of 3-6 membered heterocycloalkyl, wherein ring A optionally contains 1-3 heteroatoms independently selected from the group consisting of N and O in addition to the N atom linked to L, and ring A is optionally substituted with one or more groups selected from the group consisting of -OH and -C₁₋₄ alkyl; or ring A is optionally substituted with one or more groups selected from the group consisting of -C₁₋₃ alkylene-OC₁₋₄ alkyl, -C₁₋₃ alkylene-NH(C₁₋₄ alkyl) and -C₁₋₃ alkylene-N(C₁₋₄ alkyl)₂.

In some embodiments, ring A is selected from the group consisting of 3-6 membered heterocycloalkyl, wherein ring A optionally contains 1-3 heteroatoms independently selected from the group consisting of N and O in addition to the N atom linked to L, and ring A is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂; or ring A is optionally substituted with one or more groups selected from the group consisting of -C₁₋₃ alkylene-OC₁₋₄ alkyl.

In some embodiments, ring A is selected from the group consisting of azetidinyl, tetrahydropyrrolyl, piperidinyl, diazetidinyl, imidazolinyl, piperazinyl, oxazolinyl, and morpholinyl, wherein ring A is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂; or ring A is optionally substituted with one or more -C₁₋₄ alkyl; or ring A is optionally substituted with one or more groups selected from the group consisting of -C₁₋₃ alkylene-OC₁₋₄ alkyl.

In some embodiments, ring A is selected from the group consisting of azetidinyl and tetrahydropyrrolyl, wherein ring A is optionally substituted with one or more groups selected from the group consisting of -OH, -C₁₋₄ alkyl, and -C₁₋₃ alkylene-OC₁₋₄ alkyl.

In some embodiments, ring A is selected from the group consisting of azetidinyl and tetrahydropyrrolyl, wherein ring A is optionally substituted with one or more groups selected from the group consisting of -OH and -C₁₋₄ alkyl.

In some embodiments, ring A is selected from the group consisting of azetidinyl, tetrahydropyrrolyl, piperidinyl, diazetidinyl, imidazolidinyl, piperazinyl, oxazolidinyl, isoxazolidinyl, and morpholinyl.

In some embodiments, ring A is selected from the group consisting of 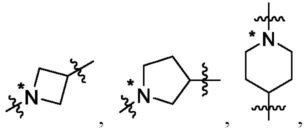 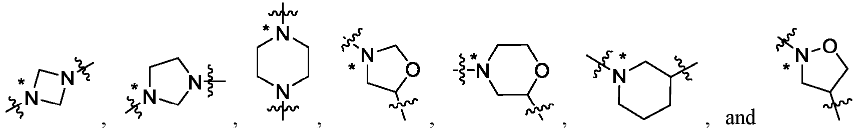 wherein the nitrogen atom marked with * is linked to L on one side and to the structural fragment 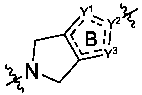 on the other side. In some embodiments, ring A is selected from the group consisting of 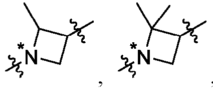 and 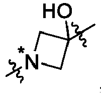 wherein the nitrogen atom marked with * is linked to L on one side and to the structural fragment 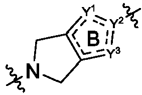 on the other side.

In some embodiments, ring A is selected from the group consisting of 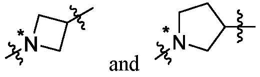 wherein ring A is optionally substituted with one or more groups selected from the group consisting of -OH and -C₁₋₄ alkyl; or ring A is optionally substituted with one or more groups selected from the group consisting of -OH, -C₁₋₄ alkyl, and -C₁₋₃ alkylene-OC₁₋₄ alkyl.

In some embodiments, ring A is selected from the group consisting of 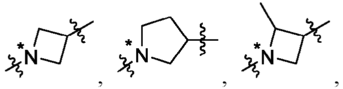 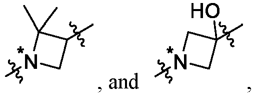 wherein * indicates the same meaning as described above.

In some embodiments, ring A is selected from the group consisting of 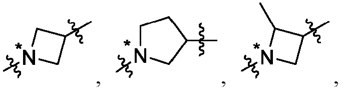 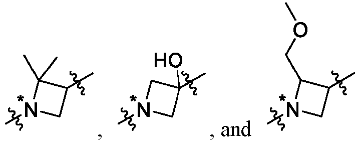 wherein * indicates the same meaning as described above.

In some embodiments, ring A is selected from the group consisting of 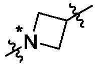 wherein * indicates the same meaning as described above.

In some embodiments, ring B is selected from the group consisting of an aromatic ring.

In some embodiments, ring B is selected from the group consisting of an aromatic ring, wherein the aromatic ring contains 1, 2, or 3 heteroatoms selected from the group consisting of N, O, and S.

In some embodiments, ring B is selected from the group consisting of a 5-membered heteroaromatic ring, wherein the 5-membered heteroaromatic ring contains 1, 2, or 3 heteroatoms selected from the group consisting of N, O, and S.

In some embodiments, ring B is selected from the group consisting of a 5-membered heteroaromatic ring, wherein the heteroaromatic ring contains 1 or 2 heteroatoms selected from the group consisting of N, O, and S.

In some embodiments, ring B is selected from the group consisting of a 5-membered heteroaromatic ring, wherein Y¹, Y², and Y³ are each independently selected from the group consisting of C, CH, N, and S.

In some embodiments, ring B is selected from the group consisting of a pyrazole ring, a pyrrole ring, a thiazole ring, an oxazole ring, an isoxazole ring, a furan ring, an imidazole ring, and a thiophene ring. In some embodiments, ring B is selected from the group consisting of a pyrazole ring, a thiazole ring, an imidazole ring, and a thiophene ring. In some embodiments, ring B is selected from the group consisting of a pyrazole ring, a thiazole ring, and a thiophene ring.

In some embodiments, ring B is selected from the group consisting of 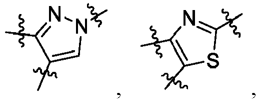 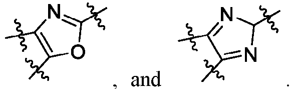 In some embodiments, ring B is selected from the group consisting of

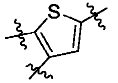

In some embodiments, ring B is selected from the group consisting of 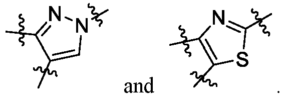 In some embodiments, ring B is selected from the group consisting of

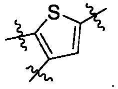

In some embodiments, ring B is selected from the group consisting of

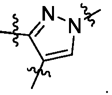

In some embodiments, the structural fragment 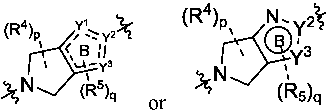 is selected from the group consisting of

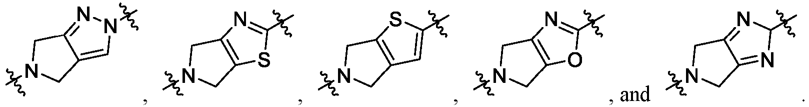

In some embodiments, the structural fragment 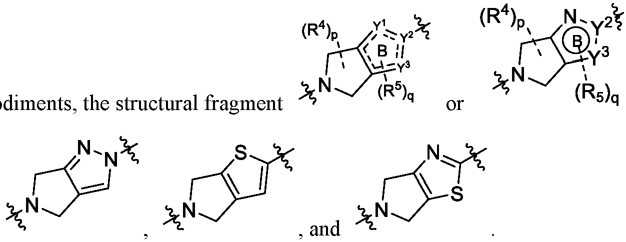 is selected from the group consisting of

In some embodiments, the structural fragment 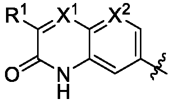 is selected from the group consisting of 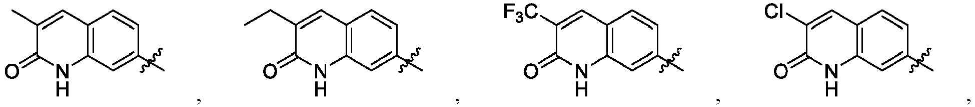 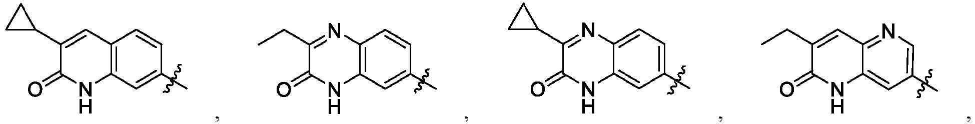 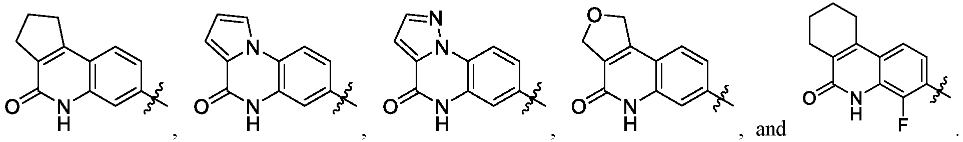 In some embodiments, the structural fragment 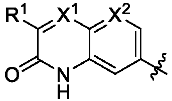 is selected from the group consisting of 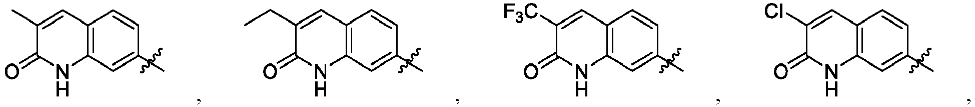 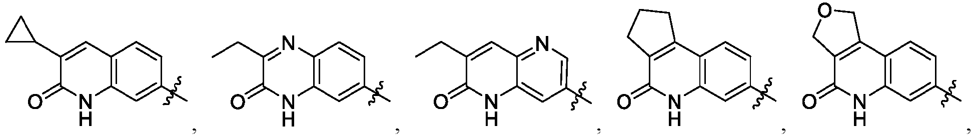 and

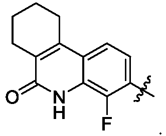

In some embodiments, Y¹ is selected from the group consisting of N and S.

In some embodiments, Y¹ is selected from the group consisting of N.

In some embodiments, Y² is selected from the group consisting of C and N.

In some embodiments, Y³ is selected from the group consisting of C, CH, N, O, and S.

In some embodiments, Y³ is selected from the group consisting of C, N, O, and S.

In some embodiments, Y³ is selected from the group consisting of CH and S.

In some embodiments, 1 or 2 of Y¹, Y², and Y³ are selected from the group consisting of N, O, and S.

In some embodiments, at least 2 of Y¹, Y², and Y³ are selected from the group consisting of N, O, and S.

In some embodiments, Y¹ and Y² are selected from the group consisting of N, and Y³ is selected from the group consisting of CH.

In some embodiments, Y¹ is selected from the group consisting of N, Y² is selected from the group consisting of C, and Y³ is selected from the group consisting of S.

In some embodiments, Y¹ is selected from the group consisting of S, Y² is selected from the group consisting of C, and Y³ is selected from the group consisting of CH.

In some embodiments, the compound of formula (I) or formula (II), the stereoisomer thereof, or the pharmaceutically acceptable salt thereof disclosed herein is selected from the group consisting of a compound of formula (IB), a stereoisomer thereof, or a pharmaceutically acceptable salt thereof, 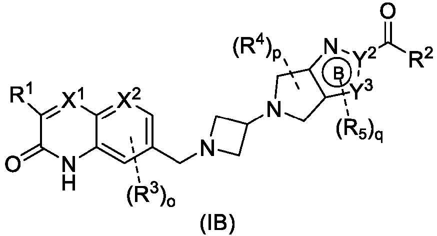
wherein R¹, R², R³, R⁴, R⁵, X¹, X², Y², Y³, o, p, and q are as defined herein;
ring B is selected from the group consisting of an aromatic ring containing 1-3 heteroatoms independently selected from the group consisting of N, O, and S.

In some embodiments, the compound of formula (I) or formula (II), the stereoisomer thereof, or the pharmaceutically acceptable salt thereof disclosed herein is selected from the group consisting of a compound of formula (IC), a stereoisomer thereof, or a pharmaceutically acceptable salt thereof: 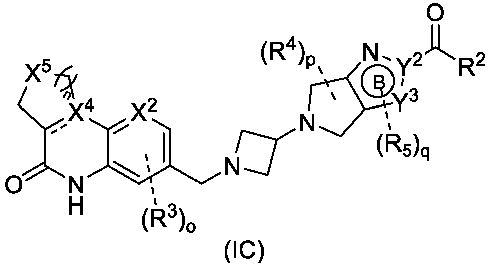
wherein R², R³, R⁴, R⁵, Y², Y³, o, p, and q are as defined herein;
ring B is selected from the group consisting of an aromatic ring containing 1-3 heteroatoms independently selected from the group consisting of N, O, and S;
X⁴ is selected from the group consisting of C, and 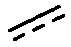 is a double bond; or X⁴ is selected from the group consisting of N, and 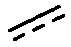 is a single bond;
X² is selected from the group consisting of CH and N;
X⁵ is selected from the group consisting of CH₂ and O;
n is selected from the group consisting of 1 and 2.

In some embodiments, the compound of formula (I) or formula (II), the stereoisomer thereof, or the pharmaceutically acceptable salt thereof disclosed herein is selected from the group consisting of a compound of formula (ID), a stereoisomer thereof, or a pharmaceutically acceptable salt thereof, 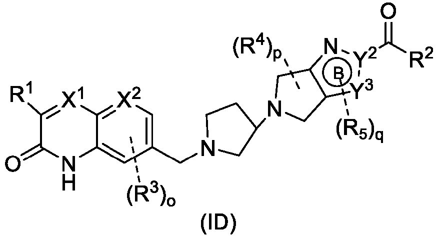
wherein R¹, R², R³, R⁴, R⁵, X¹, X², Y², Y³, o, p, and q are as defined herein;
ring B is selected from the group consisting of an aromatic ring containing 1-3 heteroatoms independently selected from the group consisting of N, O, and S.

In some embodiments, the compound of formula (I) or formula (II), the stereoisomer thereof, or the pharmaceutically acceptable salt thereof disclosed herein is selected from the group consisting of a compound of formula (IIA), a stereoisomer thereof, or a pharmaceutically acceptable salt thereof, 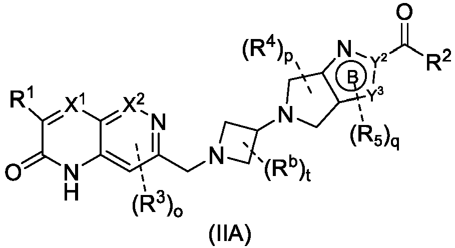
wherein R¹, R², R³, R⁴, R⁵, X¹, X², Y², Y³, o, p, and q are as defined herein;
R^{b} is selected from the group consisting of -OH, -C₁₋₆ alkyl, and -C₁₋₄ alkylene-OC₁₋₆ alkyl;
t is selected from the group consisting of 0, 1, and 2;
ring B is selected from the group consisting of an aromatic ring containing 1-3 heteroatoms independently selected from the group consisting of N, O, and S.

In some embodiments, the present application encompasses the variables defined above and embodiments thereof, as well as any combination thereof.

The heteroatom in the heterocycloalkyl or heterocycloalkenyl described above is selected from the group consisting of nitrogen (NH or N), oxygen, and sulfur (S), and the remaining ring atoms are selected from the group consisting of carbon. In some embodiments, the number of the heteroatom is selected from the group consisting of 1, 2, and 3. In some embodiments, the number of the heteroatom is selected from the group consisting of 1 and 2. In some embodiments, the number of the heteroatom is selected from the group consisting of 1.

In some embodiments, the compound, the stereoisomer thereof, or the pharmaceutically acceptable salt thereof disclosed herein is selected from the group consisting of the following compounds, stereoisomers thereof, or pharmaceutically acceptable salts thereof:

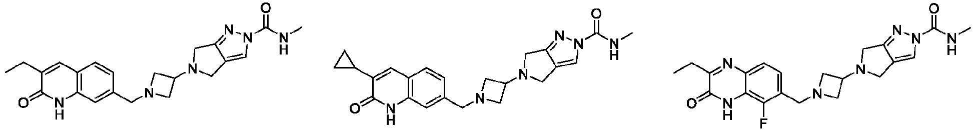

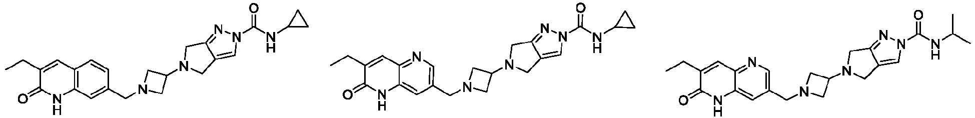

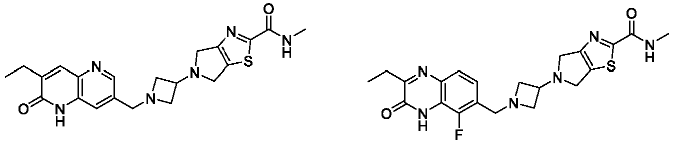

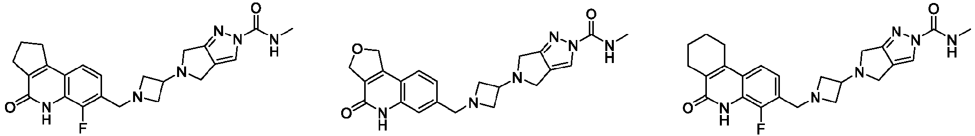

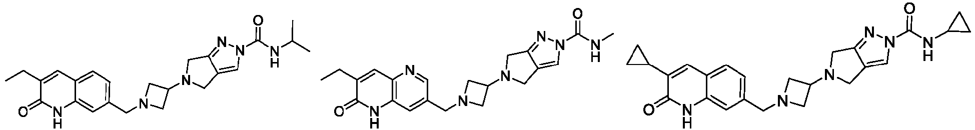

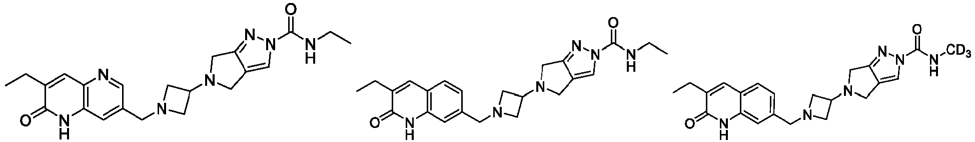

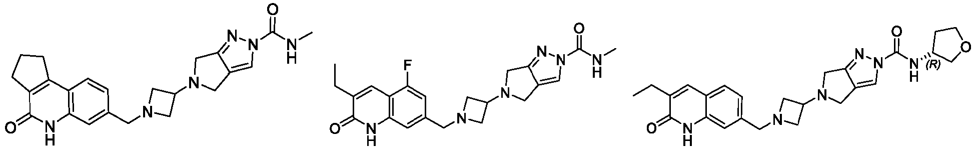

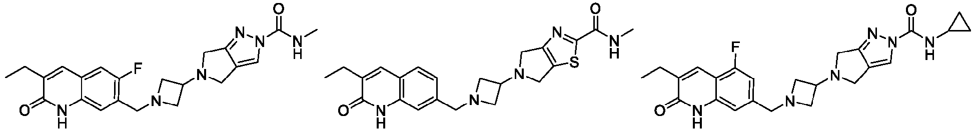

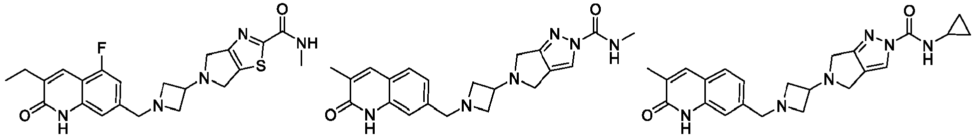

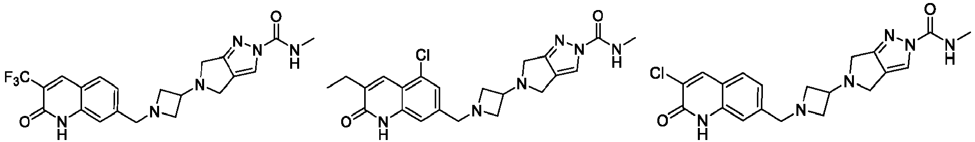

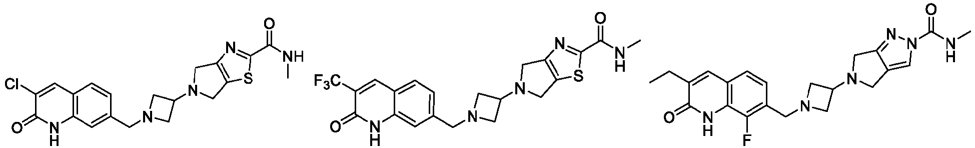

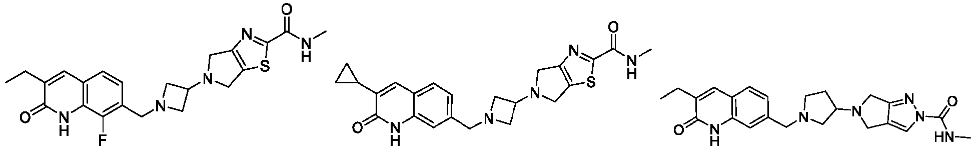

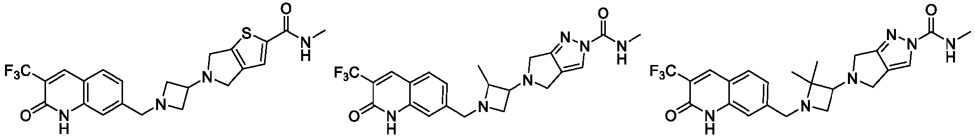

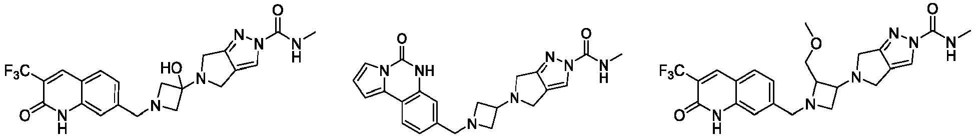

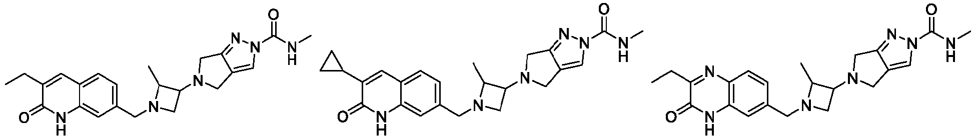

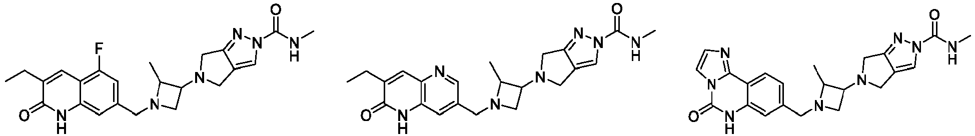

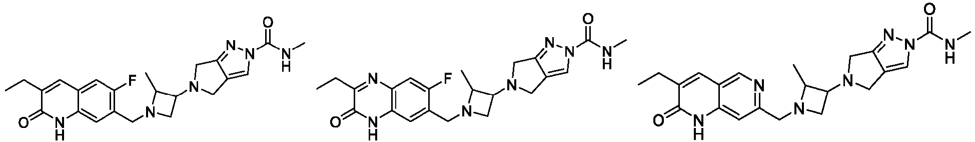

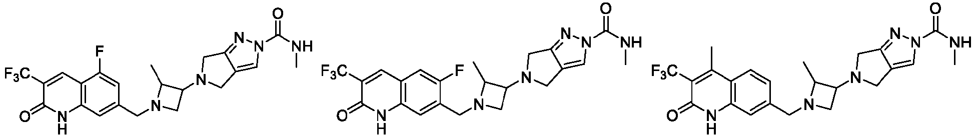

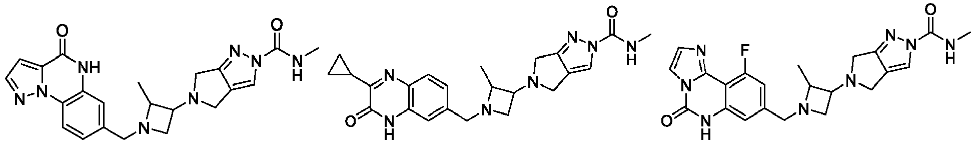

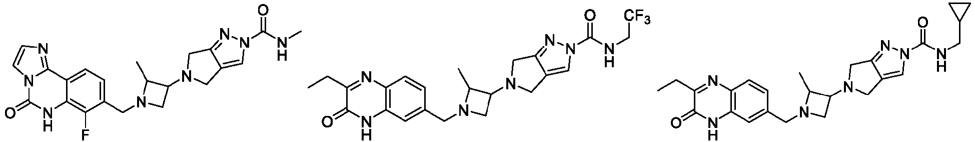

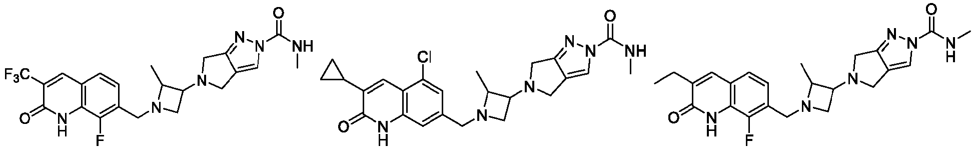

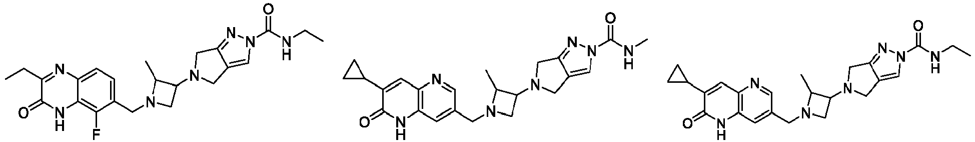

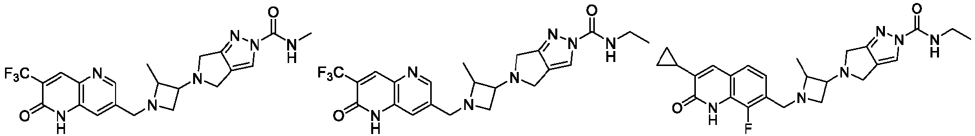

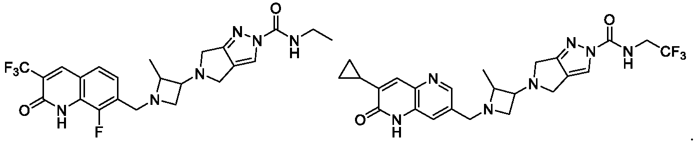

In some embodiments, the compound, the stereoisomer thereof, or the pharmaceutically acceptable salt thereof disclosed herein is selected from the group consisting of the following compounds, stereoisomers thereof, or pharmaceutically acceptable salts thereof:

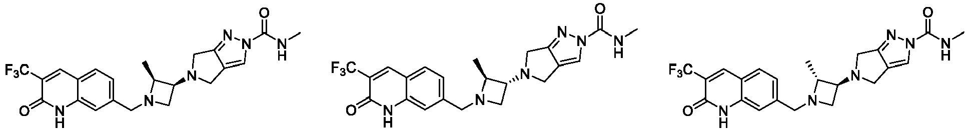

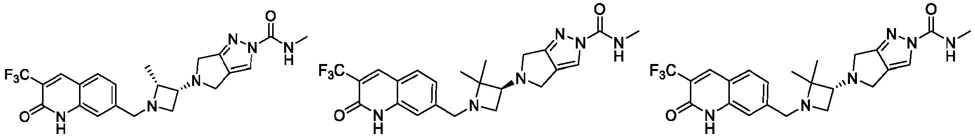

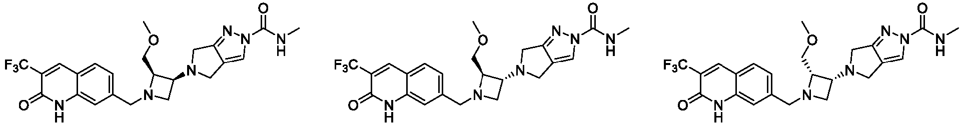

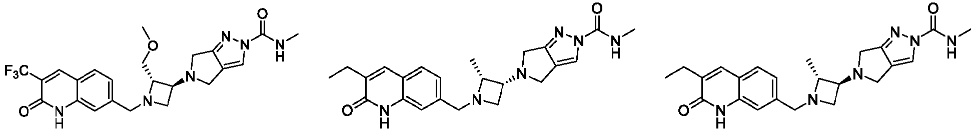

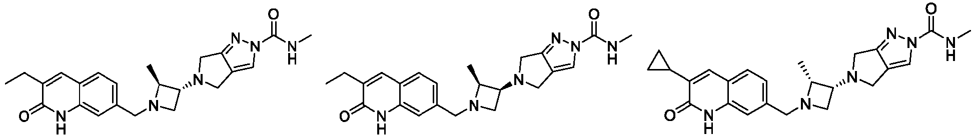

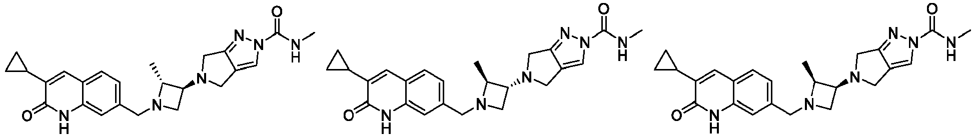

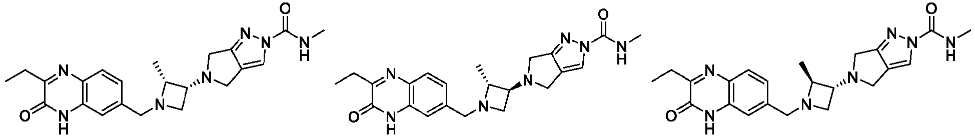

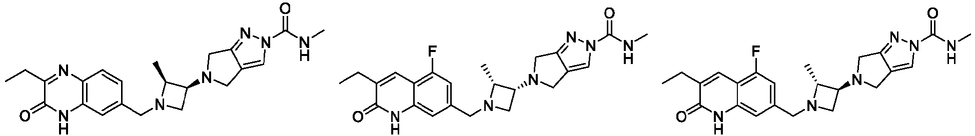

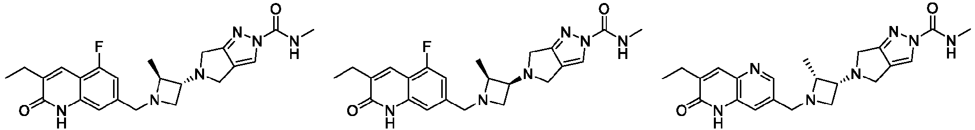

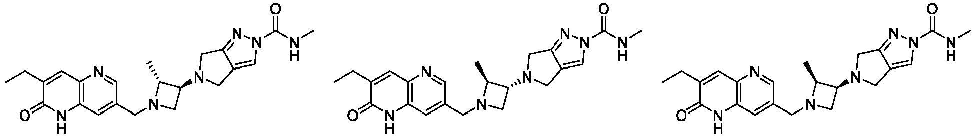

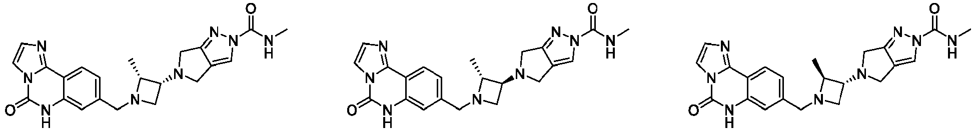

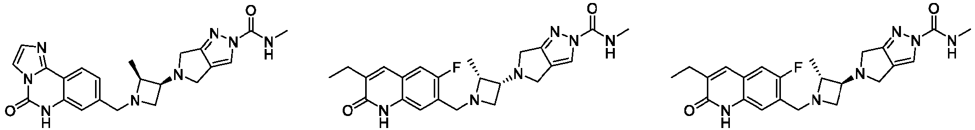

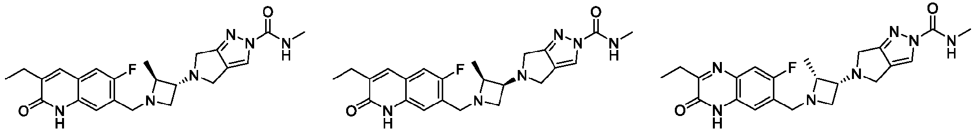

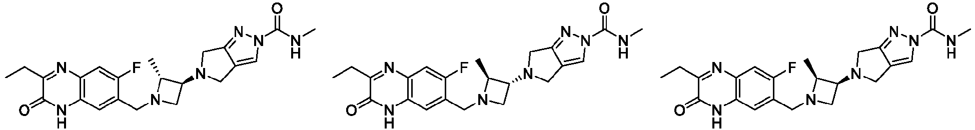

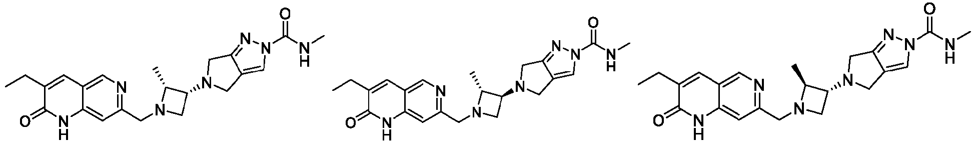

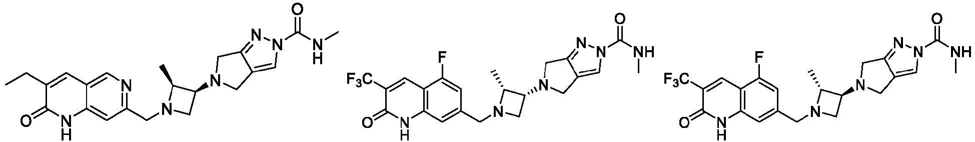

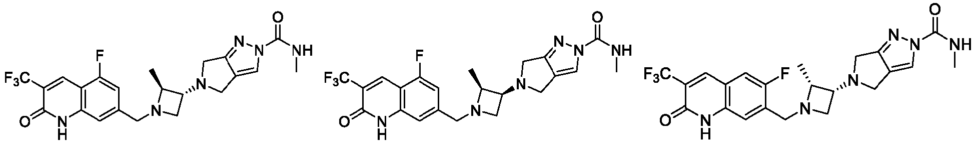

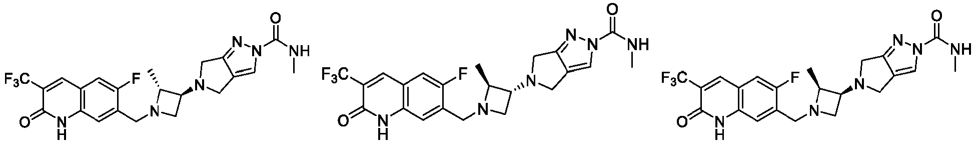

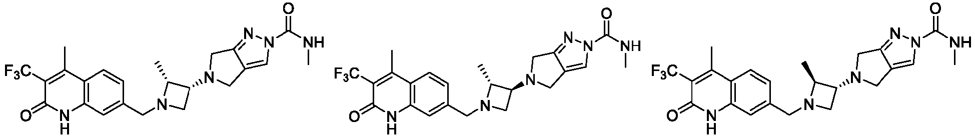

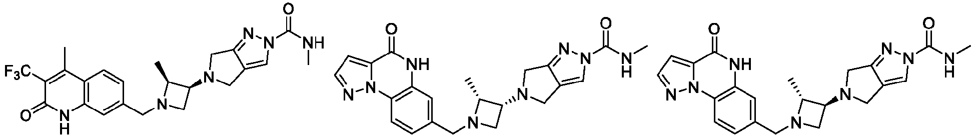

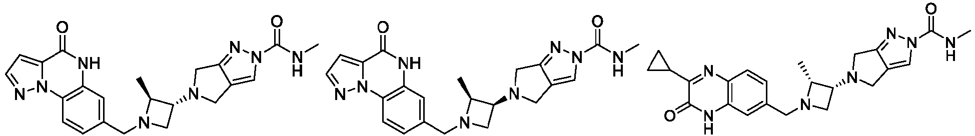

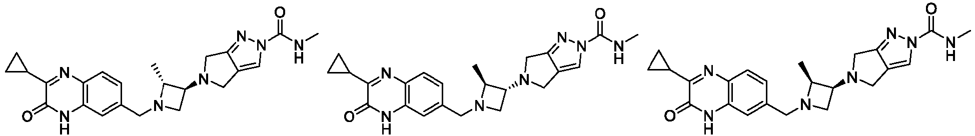

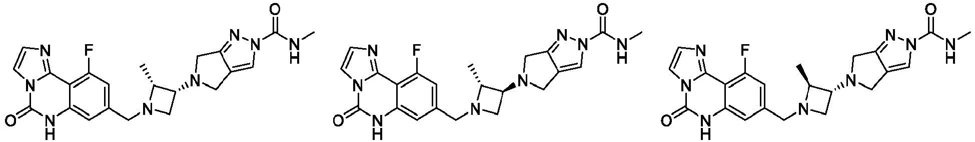

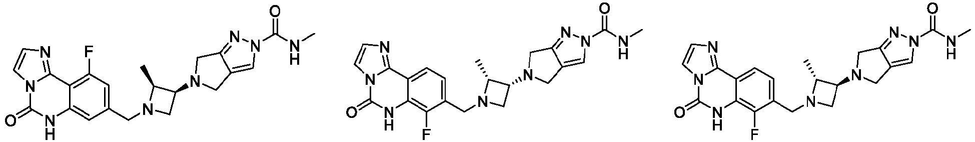

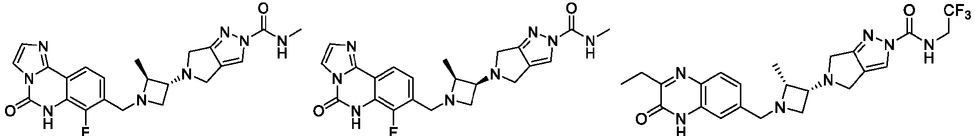

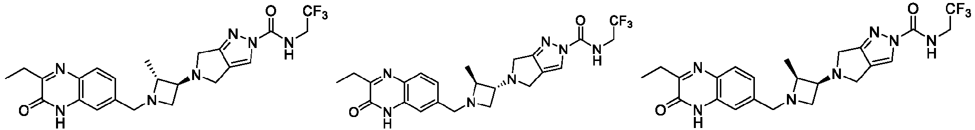

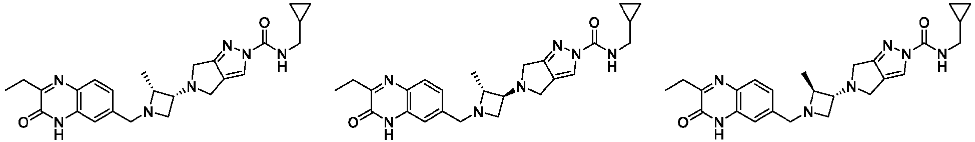

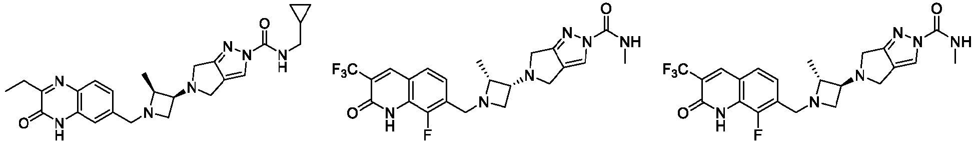

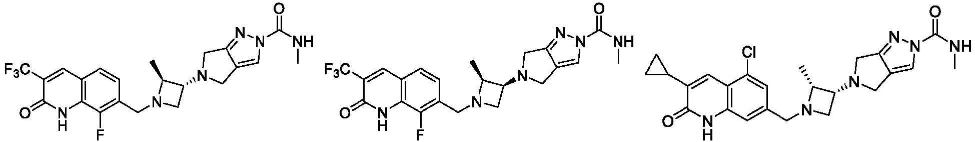

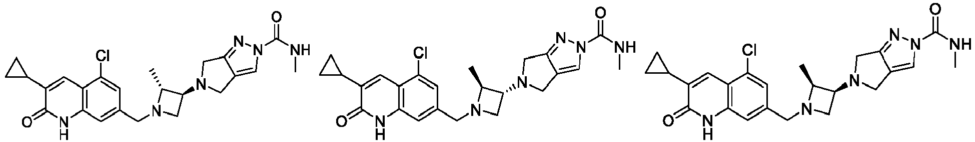

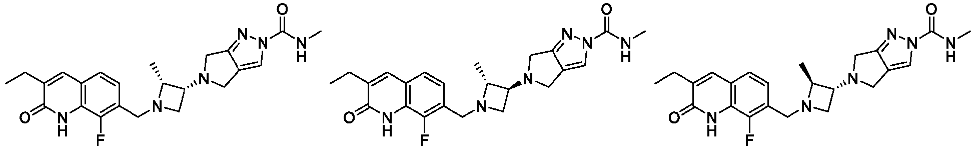

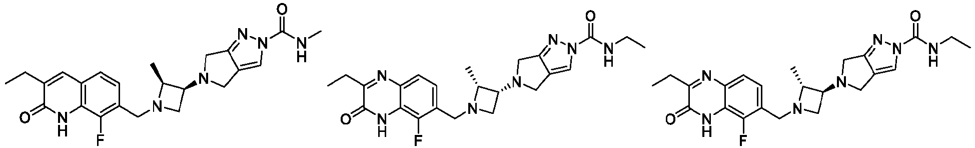

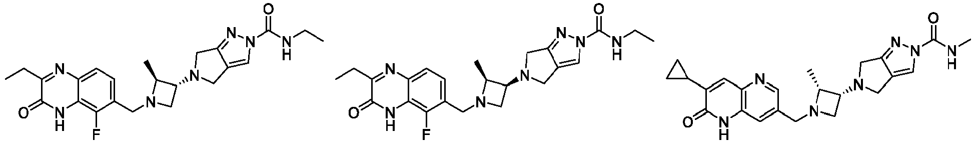

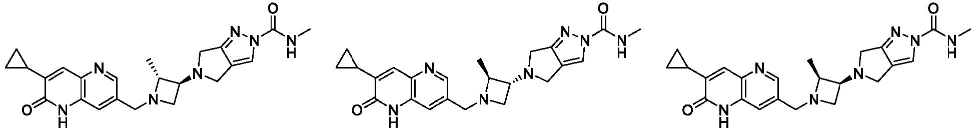

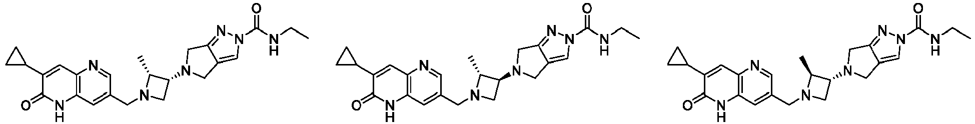

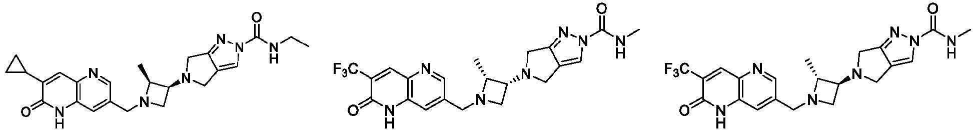

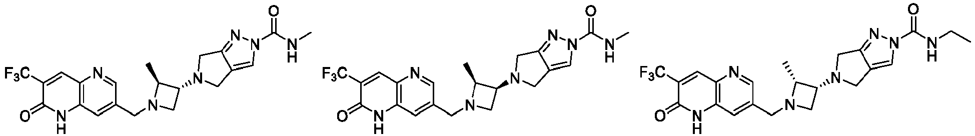

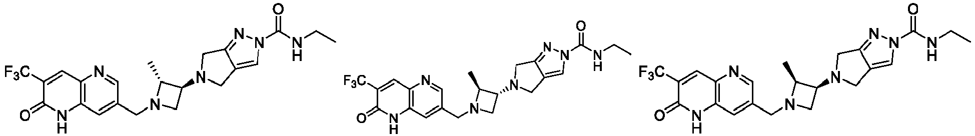

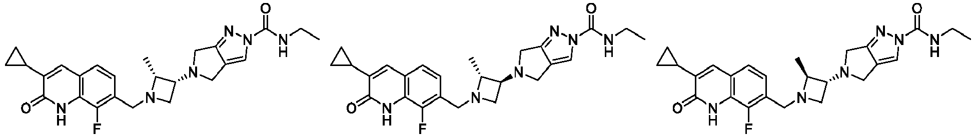

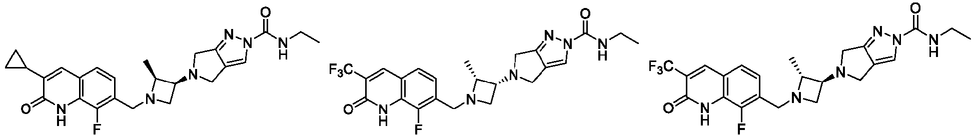

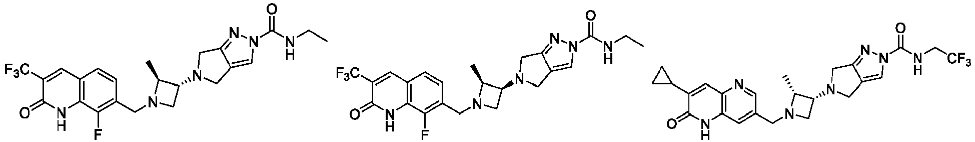

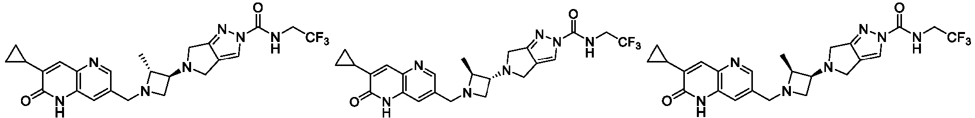

In another aspect, the present application provides a pharmaceutical composition comprising the compound, the stereoisomer thereof, or the pharmaceutically acceptable salt thereof of the present application. In some embodiments, the pharmaceutical composition of the present application further comprises a pharmaceutically acceptable excipient.

In another aspect, the present application provides a method for treating a PARP1-related disease in a mammal, which comprises administering to a mammal, preferably a human, in need of such treatment a therapeutically effective amount of the compound, the stereoisomer thereof, or the pharmaceutically acceptable salt thereof, or the pharmaceutical composition thereof described above.

In another aspect, the present application provides use of the compound, the stereoisomer thereof, or the pharmaceutically acceptable salt thereof, or the pharmaceutical composition thereof described above for preparing a medicament for treating a PARP1-related disease.

In another aspect, the present application provides use of the compound, the stereoisomer thereof, or the pharmaceutically acceptable salt thereof, or the pharmaceutical composition thereof described above for treating a PARP1-related disease.

In another aspect, the present application provides the compound, the stereoisomer thereof, or the pharmaceutically acceptable salt thereof, or the pharmaceutical composition thereof described above for treating a PARP1-related disease.

In some embodiments, the PARP1-related disease is selected from the group consisting of a tumor or a cancer. In some embodiments, the cancer is selected from the group consisting of breast cancer, ovarian cancer, colon cancer, pancreatic cancer, and prostate cancer.

The compounds of the present application exhibit strong inhibitory activity against PARP1 kinase and MDA-MB-436 cells, demonstrate high selectivity for the PARP1 protein, and possess favorable liver microsomal stability and *in vivo* pharmacokinetic properties.

### Definitions

Unless otherwise stated, the following terms used in the present application shall have the following meanings. A certain term, unless otherwise specifically defined, should not be considered uncertain or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted, and oxo is not possible in an aromatic group.

The term "optional" or "optionally" means that the subsequently described event or circumstance may or may not occur. The description includes instances where the event or circumstance occurs and instances where it does not. For example, ethyl "optionally" substituted with halogen means that the ethyl may be unsubstituted (CH₂CH₃), monosubstituted (e.g., CH₂CH₂F), polysubstituted (e.g., CHFCH₂F and CH₂CHF₂), or fully substituted (CF₂CF₃). It can be understood by those skilled in the art that for any groups comprising one or more substituents, no substitutions or substitution modes that are impossible to spatially exist and/or synthesize will be introduced.

"One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three. It can be understood by those skilled in the art that for any groups comprising one or more substituents, no substitutions or substitution modes that are impossible to spatially exist and/or synthesize will be introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs once or more times in the constitution or structure of a compound, the definition of the variable in each case is independent. Therefore, for example, if a group is substituted with 2 R, the definition of each R is independent.

When a bond of a substituent is crosslinked to two atoms on a ring, the substituent can be bonded to any atoms on the ring. For example, a structural unit 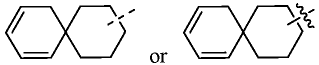 represents that substitution may occur in any one position of cyclohexyl or cyclohexadienyl. For example, in the present application, the tricyclic structure 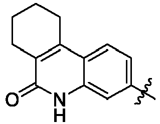 is substituted with the substituent R³, indicating that R³ can be located on any of the rings within the tricyclic system.

It may be understood that, in the present application, in 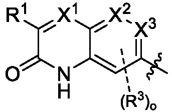 X¹ is selected from the group consisting of NR^{a}, and when R^{a} and R¹ are linked to each other to form a ring, X¹ and C(R¹) form a single bond according to the chemical bonding rule.

The term "halo-" or "halogen" refers to fluorine, chlorine, bromine, and iodine.

The term "hydroxy" refers to -OH group.

The term "amino" refers to -NH₂ group.

The term "cyano" refers to -CN group.

The term "alkyl" refers to hydrocarbyl with a general formula of CₙH₂ₙ₊₁. The alkyl may be linear or branched. For example, the term "C₁-₆ alkyl" refers to alkyl containing 1 to 6 carbon atoms (for example, methyl, ethyl, *n*-propyl, isopropyl, *n*-butyl, isobutyl, *sec*-butyl, *tert*-butyl, *n*-pentyl, 1-methylbutyl, 2-methylbutyl, 3-methylbutyl, neopentyl, hexyl, 2-methylpentyl, and the like). Similarly, the alkyl moiety (i.e., alkyl) of alkoxy, alkylamino, dialkylamino, alkylsulfonyl, and alkylthio has the same definition as those described above.

The term "alkylene" refers to divalent hydrocarbyl with a general formula of CₙH₂ₙ. For example, the term "C₁₋₆ alkylene" refers to alkylene containing 1 to 6 carbon atoms. Non-limiting examples of alkylene include, but are not limited to, methylene (-CH₂-), ethylene (-CH₂CH₂-), propylene (-CH₂CH₂CH₂- or -CH₂CH(CH₃)-), butylene (-CH₂CH₂CH₂CH₂-, -CH₂CH(CH₃)CH₂-, or -CH₂CH₂CH(CH₃)-), pentylene, hexylene, and the like.

The term "alkoxy" refers to -O-alkyl.

The term "alkylamino" refers to -NH-alkyl.

The term "cycloalkyl" refers to a carbocycle that is fully saturated and may exist as a monocyclic ring, a bridged ring, or a spiro ring. Unless otherwise indicated, the carbocycle is typically a 3- to 10-membered ring, preferably a 3- to 6-membered ring. Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocycloalkyl" refers to a cyclic group that is fully saturated and may exist as a monocyclic ring, a bridged ring, or a spiro ring. Unless otherwise specified, the heterocyclyl is usually a 3-8 membered ring containing 1-3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monoheterocycloalkyl having 4 or 6 ring atoms is preferred.

The term "heterocycloalkenyl" includes cycloalkenyl in which one or more carbon atoms are substituted with a heteroatom, such as, specifically, cycloalkenyl in which up to 3 carbon atoms, in one embodiment up to 2 carbon atoms, and in another embodiment 1 carbon atom, are each independently replaced by O, S, or N, provided that at least one cycloalkenyl carbon-carbon double bond is preserved. A cyclic group that may exist in the form of a monocyclic, bridged cyclic, or spiro cyclic structure may be a 3- to 12-membered ring (e.g., a 5-membered, 6-membered, or 7-membered ring). Examples of heterocycloalkenyl include, but are not limited to, dihydropyrrolyl, dihydrofuranyl, tetrahydropyridinyl, tetrahydroazepinyl, or azaspirooctenyl.

The term "heterocyclyl" refers to a fully saturated, partially saturated, or aromatic ring which may exist in the form of a monocyclic, bridged cyclic, or spiro cyclic structure. Unless otherwise specified, the heterocyclyl is usually a 3- to 10-membered, or 5- to 8-membered, or 5- or 6-membered ring containing 1 to 3 heteroatoms (preferably 1 or 2 heteroatoms) independently selected from the group consisting of N, O, and S. Non-limiting examples of heterocyclyl include, but are not limited to, oxiranyl, tetrahydrofuranyl, dihydrofuranyl, 3,4-dihydropyranyl, 3,6-dihydropyranyl, furanyl, pyrrolidinyl, *N*-methylpyrrolidinyl, dihydropyrrolyl, pyrrolyl, piperidinyl, piperazinyl, pyrazolidinyl, pyrazolyl, 4*H*-pyranyl, morpholinyl, thiomorpholinyl, tetrahydrothienyl, thienyl, pyrazolyl, imidazolyl, thiazolyl, oxazolyl, isoxazolyl, pyridinyl, pyrimidinyl, pyrazinyl, quinolyl, isoquinolyl, 2-oxa-7-azaspiro[3.5]nonanyl, 2-oxa-6-azaspiro[3.3]heptanyl, 3-azabicyclo[3.1.0]hexanyl, and the like.

The term "aryl" or "aromatic ring" refers to an all-carbon aromatic monocyclic or fused polycyclic group having a conjugated π-electron system. Unless otherwise specified, aryl may have 6-20 carbon atoms, 6-14 carbon atoms, or 6-12 carbon atoms. Non-limiting examples of the aryl include, but are not limited to, phenyl, naphthyl, anthryl, 1,2,3,4-tetrahydronaphthalene, and the like.

The term "heteroaryl" or "heteroaromatic ring" refers to a monocyclic or fused polycyclic system that comprises at least one ring atom selected from the group consisting of N, O and S, with the remaining ring atoms being C, and that has at least one aromatic ring. Preferably, heteroaryl has a single 5-8 membered ring, in particular a single 5-6 membered ring, or has a plurality of fused rings comprising 6 to 14 ring atoms, in particular 6 to 10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The term "fused" refers to polycyclic compounds formed when two or more carbocyclic or heterocyclic rings are parallelized through two atoms shared thereby, including fully saturated, partially saturated, and aromatic rings. Unless otherwise specified, the fused rings are 5- to 20-membered, preferably 8- to 12-membered, and more preferably 9- to 10-membered. Non-limiting examples of the fused heteroaryl include, but are not limited to, naphthalene, anthracene, phenanthrene, 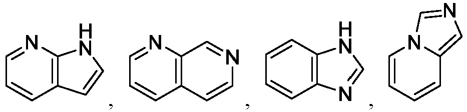 or the like.

The "cycloalkyl", "heterocycloalkyl", "cycloalkenyl", "heterocycloalkenyl", "aryl", and "heteroaryl" described herein are optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

The term "treat", "treating", or "treatment" refers to administering the compound or formulation described herein to ameliorate or eliminate a disease or one or more symptoms related to the disease, including:
(i) inhibiting a disease or disease state, i.e., arresting its progression; and
(ii) alleviating a disease or disease state, i.e., causing its regression.

The term "therapeutically effective amount" refers to an amount of the compound described herein for (i) treating the specific disease, condition or disorder described herein; (ii) alleviating, ameliorating, or eliminating one or more symptoms of the specific disease, condition or disorder described herein, or (iii) preventing or delaying onset of one or more symptoms of the specific disease, condition or disorder described herein. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, and a salt formed with a basic or acidic amino acid.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organic entity.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, and water.

The word "comprise" and variations thereof such as "comprises" or "comprising" will be understood in an open, non-exclusive sense, i.e., "including but not limited to".

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

Unless otherwise specified, terms in the singular shall be deemed to include the plural and vice versa. Unless otherwise specified, the word "a" or "an" refers to "at least one".

The compounds disclosed herein can be in the form of a geometric isomer or stereoisomer. All such compounds are contemplated herein, including *cis-* and *trans*-isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present application. The substituents such as alkyl may have an additional asymmetric carbon atom. All these isomers and mixtures thereof are encompassed within the scope of the present application.

Unless otherwise stated, the absolute configuration of a stereogenic center is represented by a wedged solid bond ( 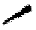 ) and a wedged dashed bond ( 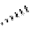 ), and the relative configuration of a stereogenic center is represented by a straight solid bond ( 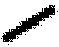 ) and a straight dashed bond ( 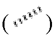 ).

Optically active (*R*)- and (*S*)-isomers and *D* and *L* isomers can be prepared by chiral synthesis or chiral reagents or other conventional techniques. An enantiomer of a certain compound disclosed herein can be prepared by asymmetric synthesis or derivatization using a chiral additive, wherein the resulting diastereoisomeric mixture is separated and the auxiliary group is cleaved so as to provide the desired pure enantiomer. Alternatively, when the molecule contains a basic functional group (for example, amino) or an acidic functional group (for example, carboxyl), the compound reacts with an appropriate optically active acid or base to form a salt of the diastereoisomer, which is then subjected to diastereoisomeric resolution through conventional methods well known in the art to give the pure enantiomer. Furthermore, the enantiomer and the diastereoisomer are generally isolated through chromatography using a chiral stationary phase, optionally in combination with chemical derivatization (e.g., carbamate generated from amines).

The present application also includes isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present application (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium. Exemplary deuterated compounds are shown below, but are not limited thereto.

The pharmaceutical composition of the present application may be prepared by combining the compound of the present application with a suitable pharmaceutically acceptable excipient, and may be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present application may be manufactured using methods well known in the art, such as conventional mixing, dissolving, granulating, dragee-making, levigating, emulsifying, and lyophilizing.

In all of the administration methods of the compound of general formula I described herein, the daily dose administered is from 0.01 mg/kg to 200 mg/kg of body weight, given in individual or separated doses.

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions in the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino in the present application). For example, reference may be made to Greene 's Protective Groups in Organic Synthesis (4th Ed.) Hoboken, New Jersey: John Wiley & Sons, Inc. All references cited herein are incorporated by reference in their entirety.

### DETAILED DESCRIPTION

For clarity, the present application is further described with the following examples, which are, however, not intended to limit the scope of the present application. It will be apparent to those skilled in the art that various changes and modifications can be made to the specific embodiments without departing from the spirit and scope of the present application. All reagents used in the present application are commercially available and can be used without further purification.

The compounds of the present application can be prepared by those skilled in the art of organic synthesis with reference to the routes or methods described in the following examples. The obtained compounds can be characterized using known instruments or methods, including but not limited to mass spectrometry and nuclear magnetic resonance.

The following abbreviations are used in the present application:
Boc represents tert-butoxycarbonyl; THF represents tetrahydrofuran; IBX represents 2-iodoxybenzoic acid; TFA represents trifluoroacetic acid; mCPBA represents m-chloroperoxybenzoic acid; RuPhosPdG3 represents methanesulfonato(2-dicyclohexylphosphino-2',6'-diisopropoxy-1,1'-biphenyl)(2-amino-1,1'-biphenyl-2-yl)pallad ium(II); DIBAL-H represents diisobutylaluminum hydride; DIPEA represents N,N-diisopropylethylamine; PdCl₂(dppf) represents [1,1'-bis(diphenylphosphino)ferrocene]palladium dichloride.

### Example 1: Preparation of Compound 1

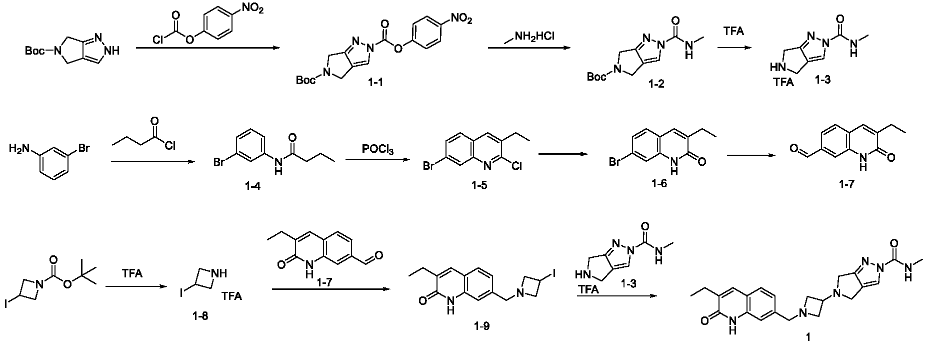

### Step A: Preparation of compound 1-1

To a 100 mL single-necked flask were added compound *tert*-butyl pyrrolo[3,4-*C*]pyrazole-5(2*H*,4*H*,6*H*)-carboxylate (1 g), dichloromethane (40 mL), sodium carbonate (1.25 g), p-nitrophenyl chloroformate (1 g) in sequence, and the mixture was reacted at room temperature for 4 h. The reaction solution was diluted with 500 mL of water and 200 mL of dichloromethane and shaken well, and the phases were separated, retaining the organic phase and the aqueous phase. The aqueous phase was extracted twice with dichloromethane (100 mL × 2). The organic phases were combined and washed with water (10 mL × 2). The organic phase was retained, concentrated to dryness by rotary evaporation, and dried to obtain 1.75 g of compound 1-1.

### Step B: Preparation of compound 1-2

To a 100 mL single-necked flask were added compound 1-1 (1.3 g), dichloromethane (40 mL), triethylamine (0.9 g), and methylamine hydrochloride (0.27 g) in sequence, and the mixture was reacted at room temperature for 4 h. The reaction solution was concentrated until no fraction was distilled off, and slurried with 30 mL of purified water. The filter cake was retained. After the reaction solution was slurried three times, the filter cakes were collected and dried to obtain 800 mg of compound 1-2.

MS (ESI, [M+H]⁺) m/z: 267.12.

### Step C: Preparation of compound 1-3

To a 100 mL single-necked flask were added compound 1-2 (250 mg), dichloromethane (10 mL), and trifluoroacetic acid (3 mL) in sequence. After the addition was completed, the system was purged with nitrogen, and the mixture was placed at room temperature under nitrogen atmosphere and reacted. After the reaction was completed, the mixture was concentrated under reduced pressure to remove excess acid and solvent to obtain compound 1-3.

MS (ESI, [M+H]⁺) *m*/*z*: 167.12.

### Step D: Preparation of compound 1-4

To a 100 mL three-necked flask were added m-bromoaniline (4 g), dichloromethane (50 mL), and triethylamine (4.7 g) in sequence, and n-butyryl chloride (2.6 g) was slowly added dropwise to the mixture under an ice salt bath under nitrogen atmosphere. After the addition was completed, the mixture was stirred at room temperature for 1 h. After the reaction was completed, 30 mL of water was added to the reaction system, and the mixture was extracted with dichloromethane (60 mL) three times. The organic phases were combined, dried, concentrated, and purified by column chromatography to obtain 2.6 g of compound 1-4.

MS (ESI, [M-H]⁻) *m*/*z:* 240.0.

### Step E: Preparation of compound 1-5

To a 100 mL single-necked flask was added phosphorus oxychloride (30 g), and the reaction system was placed under an ice salt bath under nitrogen atmosphere. N,N-Dimethylformamide (2.5 g) was weighed out and slowly added dropwise to the flask. After the dropwise addition was completed, the mixture was stirred for 2 h with the temperature controlled. Compound 1-4 (5.5 g) was slowly added to the flask in portions. After the addition was completed, the mixture was slowly warmed to room temperature and warmed to 65 °C, and reacted for 12 h with the temperature controlled. After the reaction was completed, the reaction solution was poured into 100 mL of ice water, and ammonium hydroxide was added to adjust the pH to neutral. The mixture was extracted with ethyl acetate (100 mL) three times, and the organic phases were collected. The organic phases were concentrated and purified by column chromatography to obtain 1.8 g of compound 1-5.

MS (ESI, [M+H]⁺) *m*/*z*: 269.93.

### Step F: Preparation of compound 1-6

To a 250 mL single-necked flask were added compound 1-5 (4.75 g), 1,4-dioxane (40 mL), and a 3 M aqueous hydrochloric acid solution (80 mL) in sequence, and the mixture was warmed to 100 °C and reacted for 6 h with the temperature controlled. After the reaction was completed, the reaction solution was concentrated under reduced pressure and dried to obtain 4 g of compound 1-6.

MS (ESI, [M+H]⁺) *m*/*z*: 252.10.

### Step G: Preparation of compound 1-7

Compound 1-6 (650 mg) was dissolved in tetrahydrofuran (20 mL) in a 100 mL three-necked flask. The reaction system was cooled to -78 °C under nitrogen atmosphere, and 2.5 M n-butyllithium (2.3 mL) was weighed out and slowly added dropwise to the reaction system with the temperature controlled at not more than -70 °C. The mixture was reacted at -78 °C for 1 h. *N,N*-Dimethylformamide (0.94 g) was weighed out and added dropwise to the flask, and the temperature was controlled at not more than -70 °C during the dropwise addition. The mixture was reacted at -78 °C for 2 h. After the reaction was completed, a saturated aqueous ammonium chloride solution was added to the reaction system to quench the reaction. The mixture was extracted three times with ethyl acetate (100 mL), and the organic phases were collected. The organic phases were concentrated and purified by column chromatography to obtain 130 mg of compound 1-7.

MS (ESI, [M+H]⁺) *m*/*z*: 202.10.

### Step H: Preparation of compound 1-8

Referring to the method of step C in Example 1, compound 1-8 was prepared by reacting 1-Boc-3-iodoazetidine with trifluoroacetic acid.

MS (ESI, [M+H]⁺) *m*/*z*: 184.00.

### Step I: Preparation of compound 1-9

To a 50 mL single-necked flask were added compound 1-7 (800 mg), toluene (20 mL), compound 1-8 (500 mg), tetraisopropyl titanate (706 mg), and triethylamine (503 mg) in sequence. The mixture was warmed to 60 °C. Sodium triacetoxyborohydride (1.5 g) was then weighed out and slowly added to the flask in portions. The mixture was reacted for 10 h with the temperature controlled at 60 °C. The mixture was then extracted, concentrated, and purified by column chromatography to obtain 300 mg of compound 1-9.

MS (ESI, [M+H]⁺) *m*/*z*: 369.07.

### Step J: Preparation of compound 1

To a 100 mL single-necked flask were added compound 1-3 (100 mg), acetonitrile (20 mL), triethylamine (150 mg), and compound 1-9 (150 mg) in sequence, and the mixture was reacted at 60 °C for 18 h. The mixture was purified by column chromatography to obtain 10 mg of compound 1.

¹H NMR (500 MHz, DMSO-*d₆*) δ 11.65 (s, 1H), 8.29 (q, *J =* 4.4 Hz, 1H), 7.94 (s, 1H), 7.67 (s, 1H), 7.53 (d, *J =* 8.0 Hz, 1H), 7.22 (s, 1H), 7.09 - 7.01 (m, 1H), 3.69 (s, 2H), 3.65 (d, *J =* 6.7 Hz, 4H), 3.57 (p, *J =* 6.2 Hz, 1H), 3.39 (t, *J =* 7.0 Hz, 2H), 3.06 (t, *J =* 6.8 Hz, 2H), 2.77 (d, *J =* 4.7 Hz, 3H), 2.47 (d, *J =* 7.4 Hz, 2H), 1.15 (t, *J* = 7.4 Hz, 3H).

HRMS (ESI, [M+H]⁺) *m*/*z:* 407.2195.

### Example 2: Preparation of Compound 2

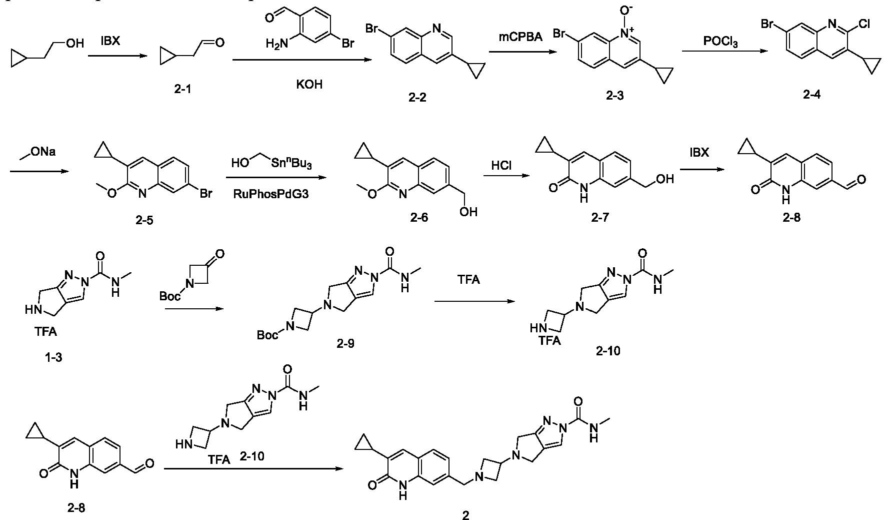

### Step A: Preparation of compound 2-1

To a 250 mL single-necked flask were added cyclopropylethanol (4 g) and 2-iodoxybenzoic acid (39.0 g). After the addition was completed, the system was purged with nitrogen, and the mixture was placed under an oil bath under nitrogen atmosphere, heated to 80 °C, and reacted under reflux. After the reaction was completed, the mixture was filtered and concentrated to obtain compound 2-1.

MS (EI, [M]⁺) *m*/*z*: 84.10.

### Step B: Preparation of compound 2-2

To a 250 mL three-necked flask were added compound 2-1 (3.91 g), 2-amino-4-bromobenzaldehyde (9.30 g), potassium hydroxide (7.82 g), and ethanol (90 mL) in sequence. After the addition was completed, the system was purged with nitrogen, and the mixture was placed under an oil bath under nitrogen atmosphere, heated to 95 °C, and reacted. After the reaction was completed, 300 mL of a saturated aqueous ammonium chloride solution was poured into the reaction solution, and the mixture was extracted with EA. The organic phases were collected, dried, concentrated, and purified by column chromatography to obtain compound 2-2.

MS (ESI, [M+H]⁺) *m*/*z*: 248.08.

### Step C: Preparation of compound 2-3

To a 500 mL single-necked flask were added compound 2-2 (8.1 g), m-chloroperoxybenzoic acid (16.9 g), and 150 mL ethyl acetate in sequence. After the addition was completed, the system was purged with nitrogen, and the mixture was placed under an oil bath under nitrogen atmosphere, heated to 70 °C, and reacted. After the reaction was completed, the mixture was extracted, concentrated, and purified by column chromatography to obtain compound 2-3.

MS (ESI, [M+H]⁺) *m*/*z*: 264.02.

### Step D: Preparation of compound 2-4

To a 250 mL single-necked flask were added compound 2-3 (6.1 g), 1,2-dichloroethane (150 mL), and phosphorus oxychloride (6.46 mL) in sequence. After the addition was completed, the system was purged with nitrogen, and the mixture was placed under an oil bath under nitrogen atmosphere, heated to 65 °C, and reacted. After the reaction was completed, the reaction solution was poured into 300 mL of ice water. Sodium carbonate was added to adjust the pH to be neutral, and the mixture was extracted, concentrated, and purified by column chromatography to obtain compound 2-4.

MS (ESI, [M+H]⁺) *m*/*z*: 282.03.

### Step E: Preparation of compound 2-5

To a 250 mL single-necked flask were added compound 2-4 (2.56 g), methanol (150 mL), and sodium methoxide (16.31 g) in sequence. After the addition was completed, the system was purged with nitrogen, and the mixture was placed under an oil bath under nitrogen atmosphere, heated to 65 °C, and reacted. After the reaction was completed, the mixture was extracted, concentrated, and purified by column chromatography to obtain compound 2-5.

MS (ESI, [M+H]⁺) *m*/*z*: 278.03.

### Step F: Preparation of compound 2-6

To a 250 mL three-necked flask were added compound 2-5 (2.3 g), methanesulfonato(2-dicyclohexylphosphino-2',6'-diisopropoxy-1,1'-biphenyl)(2-amino-1,1'-biphenyl-2-yl)palladium(II) (0.692 g), 1,4-dioxane (60 mL), and (tributyltin)methanol (5.31 g) in sequence. After the addition was completed, the system was purged with nitrogen, and the mixture was placed under an oil bath under nitrogen atmosphere, heated to 80 °C, and reacted. After the reaction was completed, the mixture was concentrated and purified by column chromatography to obtain compound 2-6.

MS (ESI, [M+H]⁺) *m*/*z*: 230.03.

### Step G: Preparation of compound 2-7

To a 100 mL single-necked flask were added compound 2-6 (1.3 g), 1,4-dioxane (30 mL), and 2 M hydrochloric acid (27.4 mL) in sequence. After the addition was completed, the mixture was placed under an oil bath, heated to 80 °C, and reacted. After the reaction was completed, the pH was adjusted to 10 with a saturated aqueous sodium carbonate solution, and the mixture was extracted, concentrated, and purified by column chromatography to obtain compound 2-7.

MS (ESI, [M+H]⁺) *m*/*z*: 216.17.

### Step H: Preparation of compound 2-8

To a 250 mL single-necked flask were added compound 2-7 (1.15 g), acetonitrile (150 mL), and 2-iodoxybenzoic acid (3 g) in sequence. After the addition was completed, the system was purged with nitrogen, and the mixture was placed under an oil bath under nitrogen atmosphere, heated to 80 °C, and reacted. After the reaction was completed, the mixture was filtered, concentrated, and purified by column chromatography to obtain compound 2-8.

MS (ESI, [M+H]⁺) *m*/*z*:214.22.

### Step I: Preparation of compound 2-9

To a 100 mL single-necked flask were added compound 1-3 (2.3 g), dichloromethane (50 mL), triethylamine (6 mL), 1-Boc-3-azetidinone (2.98 g), and sodium triacetoxyborohydride (5.53 g) in sequence. After the addition was completed, the system was purged with nitrogen, and the mixture was placed at room temperature under nitrogen atmosphere and reacted. After the reaction was completed, the mixture was extracted, concentrated, and purified by column chromatography to obtain compound 2-9.

MS (ESI, [M+H]⁺) *m*/*z*:322.26.

### Step J: Preparation of compound 2-10

Referring to the method of step C in Example 1, compound 2-10 was prepared by reacting compound 2-9 with trifluoroacetic acid.

MS (ESI, [M+H]⁺) *m*/*z*: 222.21.

### Step K: Preparation of compound 2

Referring to the method of step I in Example 1, compound 2 was prepared by reacting compound 2-8 with compound 2-10.

¹H NMR (500 MHz, DMSO-*d*₆) δ11.67 (s, 1H), 8.34 - 8.24 (m, 1H), 7.94 (s, 1H), 7.47 (d, *J =* 8.0 Hz, 1H), 7.39 (s, 1H), 7.21 (s, 1H), 7.03 (d, *J =* 8.0 Hz, 1H), 3.69 (s, 2H), 3.66 (s, 2H), 3.63 (s, 2H), 3.57 (p, *J =* 6.2 Hz, 1H), 3.38 (t, *J =* 6.9 Hz, 2H), 3.06 (t, *J =* 6.7 Hz, 2H), 2.78 (d, *J =* 4.7 Hz, 3H), 2.12 - 2.05 (m, 1H), 0.93 - 0.88 (m, 2H), 0.74 - 0.70 (m, 2H).

HRMS (ESI, [M+H]⁺) m/z:419.2197.

### Example 3: Preparation of Compound 3

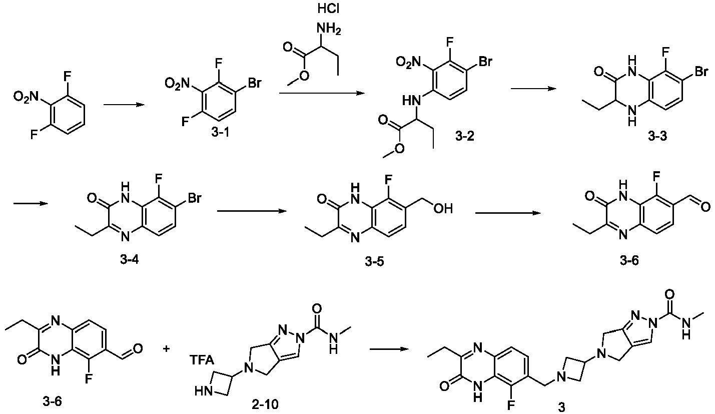

### Step A: Preparation of compound 3-1

To a 250 mL single-necked flask were added compound 2,6-difluoronitrobenzene (15 g), concentrated sulfuric acid (100 mL), and N-bromosuccinimide (17.62 g) in sequence. The mixture was stirred at 80 °C overnight under nitrogen atmosphere. After the reaction was completed, the reaction solution was poured into ice water, and the mixture was extracted three times with ethyl acetate (200 mL). The organic phase was dried and purified by column chromatography to obtain 17.63 g of compound 3-1.

### Step B: Preparation of compound 3-2

To a 250 mL single-necked flask were added compound 3-1 (17.63 g), N,N-dimethylformamide (150 mL), and methyl DL-2-amino-n-butyrate hydrochloride (12.52 g) in sequence. The reaction system was transferred to an ice salt bath, and *N,N*-diisopropylethylamine (28.7 g) was slowly added dropwise. After the dropwise addition was completed, the mixture was stirred at room temperature overnight. After the reaction was completed, the mixture was concentrated to remove the solvent, and the crude product was reconstituted in ethyl acetate, washed with saturated brine, dried, and purified by column chromatography to obtain 13 g of compound 3-2.

MS (ESI, [M-H]⁻) *m*/*z:* 333.07.

### Step C: Preparation of compound 3-3

To a 500 mL single-necked flask were added compound 3-2 (12 g), methanol (150 mL), iron powder (10 g), and acetic acid (10.75 g) in sequence. The reaction system was transferred to 70 °C and stirred for 1 h. After the reaction was completed, the reaction solution was filtered, concentrated, washed with a saturated aqueous sodium bicarbonate solution, extracted with ethyl acetate, dried, and purified by column chromatography to obtain 9 g of compound 3-3.

MS (ESI, [M-H]⁻) *m*/*z*: 271.06.

### Step D: Preparation of compound 3-4

To a 500 mL single-necked flask were added compound 3-3 (8.73 g), dichloromethane (250 mL), and 2,3-dichloro-5,6-dicyanobenzoquinone (9.43 g) in sequence. The reaction system was stirred at room temperature for 2 h. After the reaction was completed, the mixture was concentrated to remove the solvent, and 300 mL of a saturated aqueous sodium bicarbonate solution was added to the crude product, followed by stirring at room temperature overnight. The mixture was filtered, and the filter cake was collected and dried to obtain 8 g of compound 3-4.

MS (ESI, [M-H]⁻) *m*/*z*: 269.06.

### Step E: Preparation of compound 3-5

Referring to the method of step F in Example 2, compound 3-5 was prepared by reacting compound 3-4 with (tributyltin)methanol.

MS (ESI, [M+H]⁺) *m*/*z*: 223.18.

### Step F: Preparation of compound 3-6

Referring to the method of step H in Example 2, compound 3-6 was prepared by reacting compound 3-5 with 2-iodoxybenzoic acid.

MS (ESI, [M-H]⁻) *m*/*z:* 219.15.

### Step G: Preparation of compound 3

Referring to the method of step I in Example 1, compound 3 was prepared by reacting compound 3-6 with compound 2-10.

¹H NMR (500 MHz, DMSO-*d*₆) δ 12.40 (s, 1H), 8.37 - 8.22 (m, 1H), 8.06 - 7.38 (m, 2H), 7.24 (t, *J =* 7.1 Hz, 1H), 3.97 (s, 1H), 3.72 (s, 2H), 3.68 (d, *J =* 6.8 Hz, 2H), 3.65 (s, 1H), 3.61 - 3.54 (m, 1H), 3.43 - 3.39 (m, 2H), 3.14 - 3.06 (m, 2H), 2.86 - 2.80 (m, 2H), 2.78 (d, *J =* 4.6 Hz, 3H), 1.21 (t, *J =* 7.4 Hz, 3H).

HRMS (ESI, [M+H]⁺) *m*/*z:* 426.2056.

### Example 4: Preparation of Compound 4

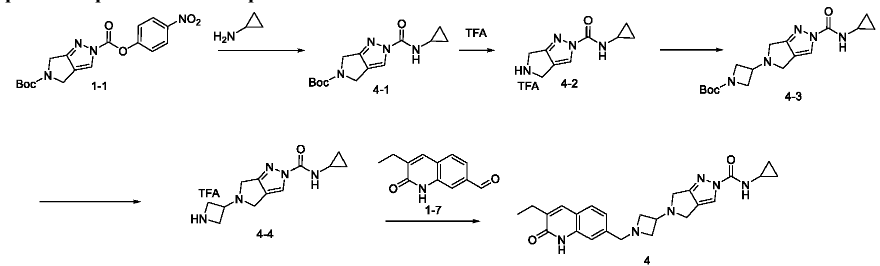

### Step A: Preparation of compound 4-1

Referring to the method of step B in Example 1, compound 4-1 was prepared by reacting compound 1-1 with cyclopropylamine.

MS (ESI, [M+H-Boc]⁺) *m*/*z:* = 193.12.

### Step B: Preparation of compound 4-2

Referring to the method of step C in Example 1, compound 4-2 was prepared by reacting compound 4-1 with trifluoroacetic acid.

MS (ESI, [M+H]⁺) *m*/*z*: 193.20.

### Step C: Preparation of compound 4-3

Referring to the method of step I in Example 2, compound 4-3 was prepared by reacting compound 4-2 with *tert*-butyl 3-oxoazetidine-1-carboxylate.

MS (ESI, [M+H]⁺) *m*/*z*: 348.28*.*

### Step D: Preparation of compound 4-4

Referring to the method of step C in Example 1, compound 4-4 was prepared by reacting compound 4-3 with trifluoroacetic acid.

MS (ESI, [M+H]⁺) *m*/*z*: 248.27.

### Step E: Preparation of compound 4

Referring to the method of step I in Example 1, compound 4 was prepared by reacting compound 1-7 with compound 4-4.

¹H NMR (500 MHz, DMSO-*d*₆) δ11.65 (s, 1H), 8.39 (d, *J =* 3.6 Hz, 1H), 7.94 (s, 1H), 7.67 (s, 1H), 7.53 (d, *J =* 8.0 Hz, 1H), 7.22 (s, 1H), 7.05 (d, *J =* 7.9 Hz, 1H), 3.75 - 3.60 (m, 6H), 3.57 (p, *J =* 6.0 Hz, 1H), 3.40 (t, *J =* 6.2 Hz, 2H), 3.17 - 2.98 (m, 2H), 2.78 - 2.70 (m, 1H), 2.50 - 2.45 (m, 2H), 1.36 - 1.22 (m, 2H), 1.16 (t, *J =* 7.4 Hz, 3H), 0.66 - 0.64 (m, 2H).

HRMS (ESI, [M+H]⁺) *m*/*z:* 433.2348.

### Example 5: Preparation of Compound 5

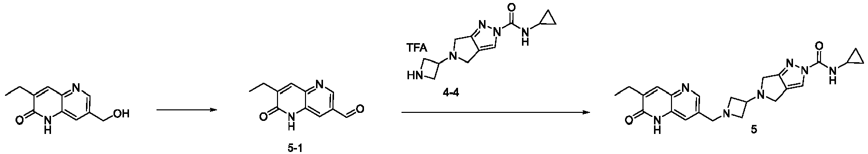

### Step A: Preparation of compound 5-1

Referring to the method of step A in Example 2, compound 5-1 was prepared by reacting 3-ethyl-7-(hydroxymethyl)-1,5-naphthyridin-2(1H)-one with 2-iodoxybenzoic acid.

MS (ESI, [M+H]⁺) *m*/*z*: 203.17.

### Step B: Preparation of compound 5

Referring to the method of step I in Example 1, compound 5 was prepared by reacting compound 5-1 with compound 4-4.

¹H NMR (500 MHz, DMSO-*d*₆) δ11.83 (s, 1H), 8.40 (d, *J =* 3.8 Hz, 1H), 8.35 (d, *J =* 1.6 Hz, 1H), 7.95 (s, 1H), 7.73 (s, 1H), 7.56 (s, 1H), 3.72 - 3.65 (m, 6H), 3.58 (p, *J =* 6.3 Hz, 1H), 3.40 (t, *J =* 6.9 Hz, 2H), 3.09 (t, *J =* 6.7 Hz, 2H), 2.74 (tq, *J =* 8.1, 4.2 Hz, 1H), 2.56 - 2.52 (m, 2H), 1.18 (t, *J =* 7.4 Hz, 3H), 0.65 (t, *J =* 4.8 Hz, 4H). HRMS (ESI, [M+H]⁺) *m*/*z:* 434.2292.

### Example 6: Preparation of Compound 6

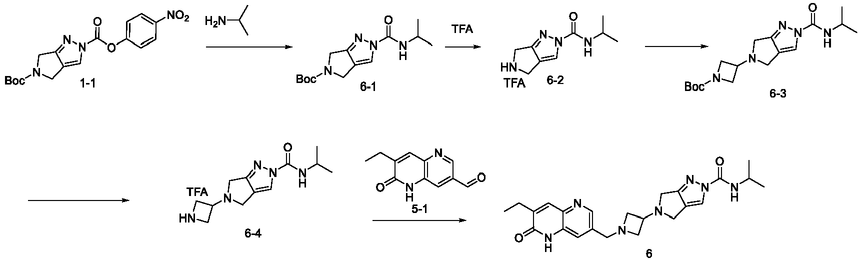

### Step A: Preparation of compound 6-1

Referring to the method of step B in Example 1, compound 6-1 was prepared by reacting compound 1-1 with isopropylamine.

MS (ESI, [M+H-Boc]⁺) *m*/*z:* 195.22.

### Step B: Preparation of compound 6-2

Referring to the method of step C in Example 1, compound 6-2 was prepared by reacting compound 6-1 with trifluoroacetic acid.

MS (ESI, [M+H]⁺) *m*/*z*: 195.21*.*

### Step C: Preparation of compound 6-3

Referring to the method of step I in Example 2, compound 6-3 was prepared by reacting compound 6-2 with *tert*-butyl 3-oxoazetidine-1-carboxylate.

MS (ESI, [M+H]⁺) *m*/*z*: 350.23.

### Step D: Preparation of compound 6-4

Referring to the method of step C in Example 1, compound 6-4 was prepared by reacting compound 6-3 with trifluoroacetic acid.

MS (ESI, [M+H]⁺) *m*/*z*: 250.25.

### Step E: Preparation of compound 6

Referring to the method of step I in Example 1, compound 6 was prepared by reacting compound 5-1 with compound 6-4.

¹H NMR (500 MHz, DMSO-*d*₆) δ 11.84 (s, 1H), 8.35 (d, *J =* 1.5 Hz, 1H), 8.08 (d, *J =* 8.4 Hz, 1H), 7.94 (s, 1H), 7.73 (s, 1H), 7.56 (s, 1H), 3.96 (dq, *J =* 13.3, 6.6 Hz, 1H), 3.76 - 3.64 (m, 6H), 3.58 (p, *J =* 6.3 Hz, 1H), 3.41 (t, *J =* 6.8 Hz, 2H), 3.10 (t, *J =* 6.5 Hz, 2H), 2.58 - 2.52 (m, 2H), 1.21 - 1.14 (m, 9H).

HRMS (ESI, [M+H]⁺) *m*/*z*:436.2460.

### Example 7: Preparation of Compound 7

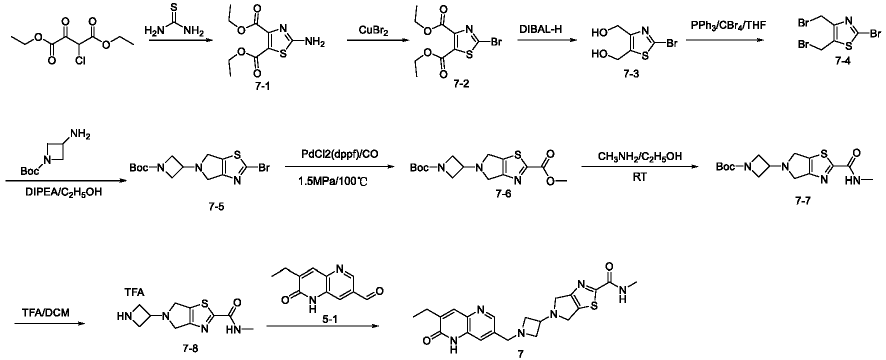

### Step A: Preparation of compound 7-1

To a 1 L single-necked flask were added diethyl 2-chloro-3-oxosuccinate (50.31 g), ethanol (500 mL), and thiourea (17.20 g) in sequence, and the mixture was heated to 90 °C and stirred overnight under reflux under nitrogen atmosphere. After the reaction was completed, the reaction solution was cooled to room temperature and concentrated to remove excess ethanol, and 200 mL of water was added to the residue, followed by slurrying for 1 h. The mixture was filtered, and the filter cake was collected and dried under vacuum at 50 °C to obtain 46.7 g of compound 7-1.

MS (ESI, [M+H]⁺) *m*/*z*: 245.11.

### Step B: Preparation of compound 7-2

To a 500 mL three-necked flask were added copper bromide (74.4 g), acetonitrile (200 mL), and *tert-butyl* nitrite (25.8 g) in sequence. The mixture was placed under an ice salt bath and cooled to -5 °C. A solution (60 mL) of compound 7-1 in acetonitrile was slowly added dropwise to the reaction system with the temperature controlled at 0 °C or lower. After the addition was completed, the mixture was stirred at 0 °C for 1 h. After the reaction was completed, the reaction solution was poured into 200 mL of water, and the mixture was extracted with ethyl acetate (300 mL × 3). The organic phases were collected, dried, concentrated, and purified by column chromatography to obtain 51 g of compound 7-2.

MS (ESI, [M+H]⁺) *m*/*z*: 307.80.

### Step C: Preparation of compound 7-3

To a 500 mL three-necked flask were added compound 7-2 (31 g) and toluene (100 mL) in sequence. The mixture was transferred to -78 °C and stirred under nitrogen atmosphere. A 1.5 M solution (201 mL) of diisobutylaluminum hydride in toluene was slowly added dropwise with the internal temperature controlled at -70 °C or lower. After the addition was completed, the reaction system was stirred at -78 °C for 3 h. After the reaction was completed, 40 mL of an aqueous ammonium chloride solution was added to the reaction system to quench the reaction. The mixture was filtered through diatomite to remove insoluble substances, and the filtrate was concentrated, mixed with silica gel, and purified by column chromatography to obtain 16 g of compound 7-3.

### Step D: Preparation of compound 7-4

To a 50 mL single-necked flask were added compound 7-3 (0.5 g), anhydrous tetrahydrofuran (20 mL), triphenylphosphine (1.288 g), and carbon tetrabromide (1.628 g) in sequence. The mixture was stirred at room temperature for 1 h under nitrogen atmosphere. After the reaction was completed, 10 mL of water was added to the reaction solution. The mixture was extracted three times with dichloromethane (50 mL). The organic phases were collected, filtered, dried, concentrated, mixed with silica gel, and purified by column chromatography to obtain 0.4 g of compound 7-4.

### Step E: Preparation of compound 7-5

To a 50 mL single-necked flask were added compound 7-4 (0.1 g), ethanol (10 mL), *tert-butyl* 3-aminoazetidine-1-carboxylate (68.9 mg), and N,N-diisopropylethylamine (0.11 g) in sequence. The mixture was stirred at 60 °C for 3 h under nitrogen atmosphere. After the reaction was completed, 10 mL of water was added to the reaction solution. The mixture was extracted three times with ethyl acetate (50 mL). The organic phases were collected, filtered, dried, concentrated, mixed with silica gel, and purified by column chromatography to obtain 30 mg of compound 7-5.

MS (ESI, [M+H-C(CH₃)₃]⁺) *m*/*z:* 304.01.

### Step F: Preparation of compound 7-6

To a 250 mL autoclave were added compound 7-5 (0.24 g), methanol (50 mL), triethylamine (0.3 g), [1,1'-bis(diphenylphosphino)ferrocene]palladium dichloride (60 mg), and 1,3-bis(diphenylphosphino)propane (60 mg) in sequence. After the addition was completed, the autoclave was sealed and purged three times with carbon monoxide. The pressure was adjusted to 1.5 MPa, and the internal temperature was set at 100 °C. The mixture was stirred for 4 h. After the reaction was completed, the reaction solution was filtered through diatomite, and 1 g of silica gel was added to the filtrate. The mixture was directly purified by column chromatography to obtain 0.1 g of compound 7-6.

MS (ESI, [M+H]⁺) *m*/*z*: *340.14.*

### Step G: Preparation of compound 7-7

To a 15 mL microwave tube were added compound 7-6 (0.11 g) and a solution of 30% methylamine in ethanol (3 mL) in sequence. After the addition was completed, the reaction system was sealed and stirred at room temperature for 3 h. After the reaction was completed, the reaction solution was directly concentrated, and 20 mL of ethyl acetate and 10 mL of water were added to the crude product for extraction. The organic phases were collected, filtered, dried, concentrated, mixed with silica gel, and purified by column chromatography to obtain 65 mg of compound 7-7.

MS (ESI, [M+H-C(CH₃)₃]⁺) *m*/*z: 283.15.*

### Step H: Preparation of compound 7-8

Referring to the method of step C in Example 1, compound 7-8 was prepared by reacting compound 7-7 with trifluoroacetic acid.

MS (ESI, [M+H]⁺) *m*/*z*: 239.20.

### Step I: Preparation of compound 7

Referring to the method of step I in Example 1, compound 7 was prepared by reacting compound 5-1 with compound 7-8.

¹H NMR (500 MHz, DMSO-*d*₆) δ 11.83 (s, 1H), 8.75 (q, *J =* 4.5 Hz, 1H), 8.35 (d, *J =* 1.7 Hz, 1H), 7.73 (s, 1H), 7.56 (s, 1H), 4.02 (t, *J* = 3.0 Hz, 2H), 3.88 (t, *J =* 3.0 Hz, 2H), 3.71 (s, 2H), 3.67 - 3.60 (m, 1H), 3.41 (t, *J =* 7.0 Hz, 2H), 3.10 (t, *J=* 6.7 Hz, 2H), 2.77 (d, *J =* 4.8 Hz, 2H), 2.54 (s, 3H), 1.17 (t, *J=* 7.4 Hz, 3H).

HRMS (ESI, [M+H]⁺) *m*/*z:* 425.1757

### Example 8: Preparation of Compound 8

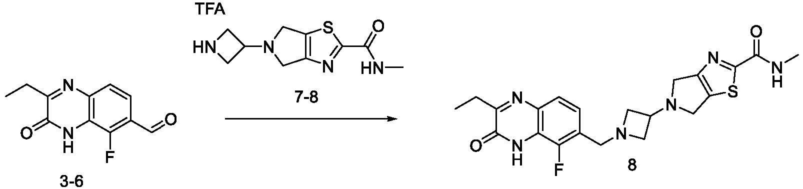

### Step A: Preparation of compound 8

Referring to the method of step I in Example 1, compound 8 was prepared by reacting compound 3-6 with compound 7-8.

¹H NMR (500 MHz, DMSO-*d*₆) δ 12.40 (s, 1H), 8.74 (q, *J =* 4.5 Hz, 1H), 7.52 (d, *J =* 8.3 Hz, 1H), 7.28 - 7.21 (m, 1H), 4.01 (t, *J =* 3.0 Hz, 2H), 3.87 (t, *J =* 3.0 Hz, 2H), 3.73 (s, 2H), 3.61 (dd, *J =* 12.5, 6.2 Hz, 1H), 3.42 (t, *J* = 6.9 Hz, 2H), 3.12 (t, *J =* 6.7 Hz, 2H), 2.81 (q, *J =* 7.4 Hz, 2H), 2.77 (d, *J =* 4.8 Hz, 3H), 1.21 (t, *J =* 7.4 Hz, 3H).

HRMS (ESI, [M+H]⁺) *m*/*z:* 443.1660.

### Example 9: Preparation of Compound 9

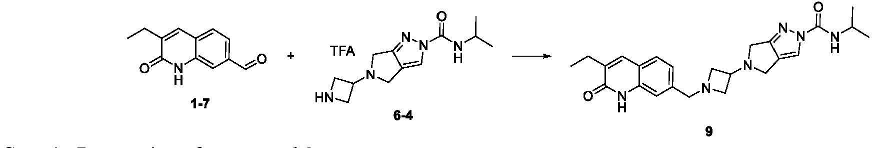

### Step A: Preparation of compound 9

Referring to the method of step I in Example 1, compound 9 was prepared by reacting compound 1-7 with compound 6-4.

¹H NMR (500 MHz, DMSO-*d*₆) δ 11.64 (s, 1H), 8.06 (d, *J =* 8.4 Hz, 1H), 7.94 (s, 1H), 7.67 (s, 1H), 7.53 (d, *J =* 8.0 Hz, 1H), 7.22 (s, 1H), 7.05 (d, *J =* 8.0 Hz, 1H), 4.00 - 3.92 (m, 1H), 3.69 (s, 2H), 3.66 (d, *J =* 4.6 Hz, 4H), 3.57 (p, *J =* 6.2 Hz, 1H), 3.40 (t, *J =* 6.8 Hz, 2H), 3.07 (t, *J =* 6.5 Hz, 2H), 2.50 - 2.46 (m, 2H), 1.19 - 1.14 (m, 9H).

HRMS (ESI, [M+H]⁺) *m*/*z:* 435.2504.

### Example 10: Preparation of Compound 10

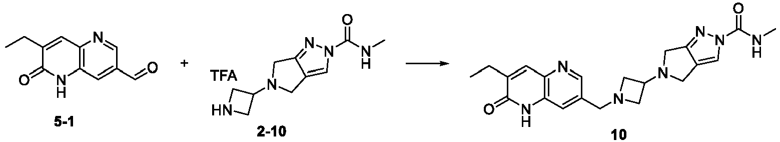

### Step A: Preparation of compound 10

Referring to the method of step I in Example 1, compound 10 was prepared by reacting compound 5-1 with compound 2-10.

¹H NMR (500 MHz, DMSO-*d*₆) δ 11.83 (s, 1H), 8.35 (d, *J =* 1.8 Hz, 1H), 8.28 (q, *J =* 4.6 Hz, 1H), 7.94 (s, 1H), 7.73 (d, *J =* 1.4 Hz, 1H), 7.56 (d, *J =* 1.8 Hz, 1H), 3.70 (d, *J =* 1.6 Hz, 4H), 3.66 (s, 2H), 3.58 (p, *J =* 6.3 Hz, 1H), 3.44 - 3.37 (m, 2H), 3.09 (dd, *J =* 7.4, 5.9 Hz, 2H), 2.77 (d, *J =* 4.6 Hz, 3H), 2.54(m, 2H), 1.17 (t, *J =* 7.4 Hz, 3H).

HRMS (ESI, [M+H]⁺) *m*/*z:* 408.2143.

### Example 11: Preparation of Compound 11

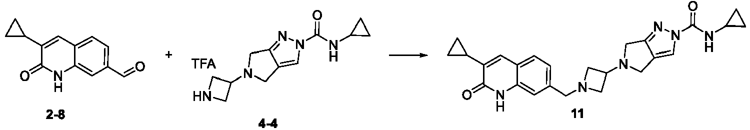

### Step A: Preparation of compound 11

Referring to the method of step I in Example 1, compound 11 was prepared by reacting compound 2-8 with compound 4-4.

¹H NMR (500 MHz, DMSO-*d*₆) δ 11.66 (s, 1H), 8.39 (d, *J =* 3.9 Hz, 1H), 7.94 (s, 1H), 7.47 (d, *J =* 8.0 Hz, 1H), 7.39 (s, 1H), 7.20 (s, 1H), 7.03 (d, *J =* 8.0 Hz, 1H), 3.68 - 3.62 (m, 6H), 3.56 (p, *J =* 6.2 Hz, 1H), 3.38 (t, *J =* 6.8 Hz, 2H), 3.05 (t, *J =* 6.6 Hz, 2H), 2.78 - 2.71 (m, 1H), 2.11 - 2.05 (m, 1H), 0.92 - 0.88 (m, 2H), 0.73 - 0.70 (m, 2H), 0.66 (d, *J =* 6.9 Hz, 4H).

HRMS (ESI, [M+H]⁺) *m*/*z:* 445.2348.

### Example 12: Preparation of Compound 12

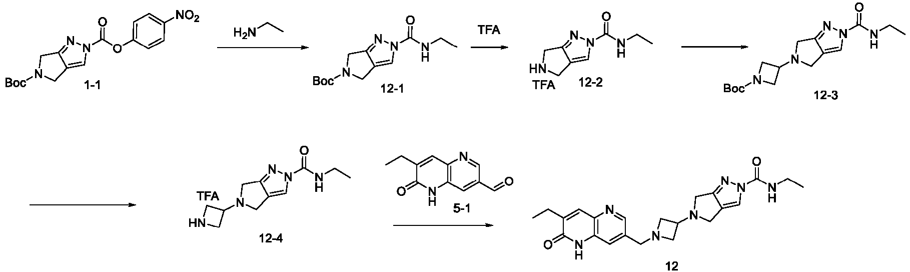

### Step A: Preparation of compound 12-1

Referring to the method of step B in Example 1, compound 12-1 was prepared by reacting compound 1-1 with ethylamine.

MS (ESI, [M+H]⁺) *m*/*z*: 281.12.

### Step B: Preparation of compound 12-2

Referring to the method of step C in Example 1, compound 12-2 was prepared by reacting compound 12-1 with trifluoroacetic acid.

MS (ESI, [M+H]⁺) *m*/*z*: 181.12.

### Step C: Preparation of compound 12-3

Referring to the method of step I in Example 2, compound 12-3 was prepared by reacting compound 12-2 with *tert*-butyl 3-oxoazetidine-1-carboxylate.

MS (ESI, [M+H]⁺) *m*/*z*: 336.25*.*

### Step D: Preparation of compound 12-4

Referring to the method of step C in Example 1, compound 12-4 was prepared by reacting compound 12-3 with trifluoroacetic acid.

MS (ESI, [M+H]⁺) *m*/*z*: 236.24.

### Step E: Preparation of compound 12

Referring to the method of step I in Example 1, compound 12 was prepared by reacting compound 5-1 with compound 12-4.

¹H NMR (500 MHz, DMSO-*d*₆) δ 11.83 (s, 1H), 8.35 (d, *J =* 7.9 Hz, 2H), 7.94 (s, 1H), 7.74 (s, 1H), 7.58 (s, 1H), 3.71 (d, *J =* 5.1 Hz, 4H), 3.67 (m, 3H), 3.27 - 3.23 (m, 2H), 3.10 (t, *J =* 6.6 Hz, 2H), 2.55 (t, *J =* 7.4 Hz, 2H), 1.88 (s, 2H), 1.18 *(t, J=* 7.5 Hz, 3H), 1.11 *(t, J=* 7.1 Hz, 3H).

HRMS (ESI, [M+H]⁺) *m*/*z:* 422.2300.

### Example 13: Preparation of Compound 13

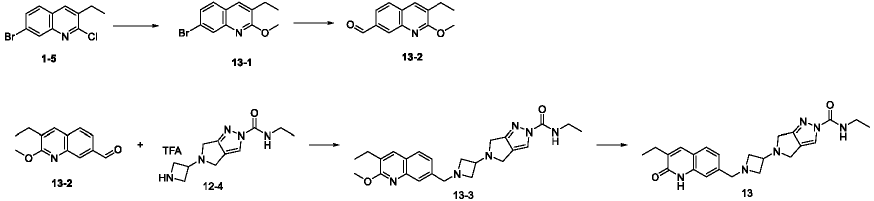

### Step A: Preparation of compound 13-1

Referring to the method of step E in Example 2, compound 13-1 was prepared by reacting compound 1-5 with sodium methoxide.

### Step B: Preparation of compound 13-2

Compound 13-1 (650 mg) was dissolved in anhydrous tetrahydrofuran (20 mL) in a 100 mL three-necked flask. The reaction system was cooled to -78 °C under nitrogen atmosphere, and 2.5 M n-butyllithium (2.3 mL) was slowly added dropwise to the reaction system with the temperature controlled at not more than -70 °C. The mixture was reacted at -78 °C for 1 h. N,N-Dimethylformamide (0.94 g) was slowly added dropwise to the reaction system, and the temperature was controlled at not more than -70 °C during the dropwise addition. The mixture was reacted at -78 °C for 2 h. After the reaction was completed, a saturated aqueous ammonium chloride solution was added to the reaction system to quench the reaction. The mixture was extracted three times with ethyl acetate (100 mL), and the organic phases were collected. The organic phases were concentrated and purified by column chromatography to obtain compound 13-2.

MS (ESI, [M+H]⁺) *m*/*z*: 216.09.

### Step C: Preparation of compound 13-1

Referring to the method of step I in Example 1, compound 13-1 was prepared by reacting compound 13-2 with compound 12-4.

MS (ESI, [M+H]⁺) *m*/*z*: 435.37.

### Step D: Preparation of compound 13

To a 25 mL single-necked flask were added compound 13-1 (80 mg) and a 4 M solution of hydrogen chloride in dioxane (4.60 mL) were added in sequence, and the mixture was placed under an oil bath at 60 °C under nitrogen atmosphere and reacted overnight. After the reaction was completed, the pH was adjusted to 8 with a saturated aqueous sodium bicarbonate solution, and the mixture was extracted three times with ethyl acetate (20 mL). The organic phases were collected, filtered, dried, concentrated, mixed with silica gel, and purified by column chromatography to obtain 45 mg of compound 13.

¹H NMR (500 MHz, DMSO-*d*₆) δ 11.72 (s, 1H), 8.37 (t, *J =* 5.5 Hz, 1H), 7.95 (s, 1H), 7.69 (s, 1H), 7.57 (d, *J =* 7.9 Hz, 1H), 7.26 (s, 1H), 7.12 (d, *J =* 7.1 Hz, 1H), 3.85 (s, 2H), 3.73 (s, 2H), 3.69 (s, 2H), 3.62 (d, *J =* 40.6 Hz, 3H), 3.25 (dd, *J* = 13.3, 6.7 Hz, 4H), 2.48 *(d, J=* 7.3 Hz, 2H), 1.16 (t, *J =* 7.4 Hz, 3H), 1.11 *(t, J=* 7.1 Hz, 3H). HRMS (ESI, [M+H]⁺) *m*/*z:* 421.2346.

### Example 14: Preparation of Compound 14

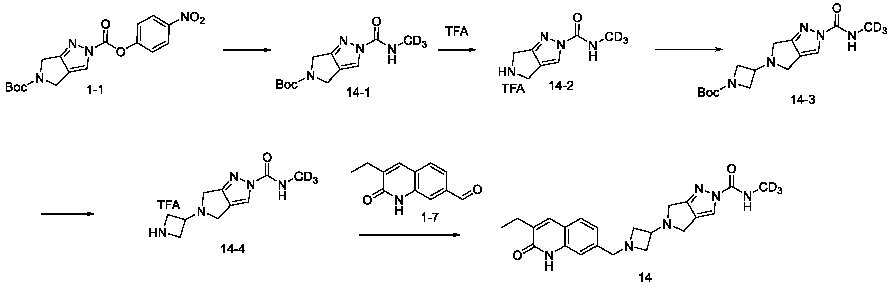

### Step A: Preparation of compound 14-1

Referring to the method of step B in Example 1, compound 14-1 was prepared by reacting compound 1-1 with deuterated methylamine hydrochloride.

MS (ESI, [M+H-Boc]⁺) *m*/*z:* 170.16.

### Step B: Preparation of compound 14-2

Referring to the method of step C in Example 1, compound 14-2 was prepared by reacting compound 14-1 with trifluoroacetic acid.

MS (ESI, [M+H]⁺) *m*/*z*: 170.15.

### Step C: Preparation of compound 14-3

Referring to the method of step I in Example 2, compound 14-3 was prepared by reacting compound 14-2 with *tert*-butyl 3-oxoazetidine-1-carboxylate.

MS (ESI, [M+H]⁺) *m*/*z*: 325.21.

### Step D: Preparation of compound 14-4

Referring to the method of step C in Example 1, compound 14-4 was prepared by reacting compound 14-3 with trifluoroacetic acid.

MS (ESI, [M+H]⁺) *m*/*z*: 225.22.

### Step E: Preparation of compound 14

Referring to the method of step I in Example 1, compound 14 was prepared by reacting compound 1-7 with compound 14-4.

¹H NMR (500 MHz, DMSO-*d*₆) δ 11.64 (s, 1H), 8.25 (s, 1H), 7.94 (s, 1H), 7.67 (s, 1H), 7.53 (d, *J =* 8.0 Hz, 1H), 7.22 (s, 1H), 7.05 (d, *J =* 8.0 Hz, 1H), 3.70 (s, 2H), 3.68 - 3.62 (m, 4H), 3.61 - 3.53 (m, 1H), 3.39 *(t, J=* 6.8 Hz, 2H), 3.06 (t, *J =* 6.6 Hz, 2H), 2.49 - 2.45 (m, 2H), 1.16 (t, *J =* 7.4 Hz, 3H).

HRMS (ESI, [M+H]⁺) *m*/*z:* 410.2387.

### Example 15: Preparation of Compound 15

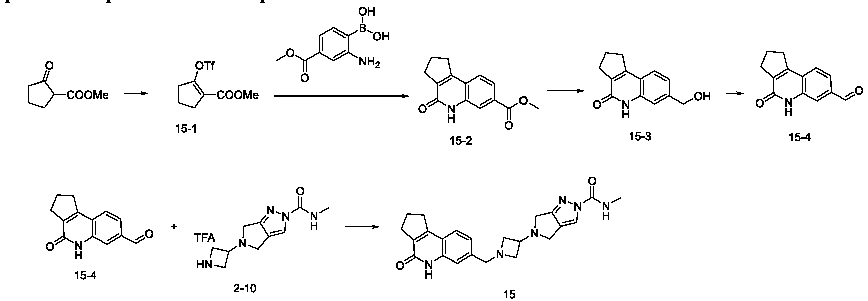

### Step A: Preparation of compound 15-1

To a 250 mL three-necked flask were added methyl 2-oxocyclopentane-1-carboxylate (7 g) and anhydrous tetrahydrofuran (100 mL) in sequence. The reaction system was transferred to an ice salt bath under nitrogen atmosphere. Sodium hydride (2.95 g) was added in portions, and the mixture was stirred under an ice salt bath for 10 min. Trifluoromethanesulfonic anhydride (16.67 g) was slowly added dropwise, and after the addition was completed, the reaction system was transferred to room temperature and stirred for 4 h. After the reaction was completed, ice water was slowly added dropwise to quench the reaction, and the mixture was extracted three times with dichloromethane (100 mL), washed with saturated brine, dried over anhydrous sodium sulfate, concentrated, and subjected to column chromatography to obtain compound 15-1.

### Step B: Preparation of compound 15-2

To a 100 mL single-necked flask were added compound 15-1 (520 mg), 1,4-dioxane (20 mL), [2-amino-4-(methoxycarbonyl)phenyl]boronic acid (370 mg), potassium carbonate (656 mg), [1,1'-bis(diphenylphosphino)ferrocene]palladium dichloride (140 mg), and water (1 mL). The system was purged with nitrogen three times, and the reaction system was placed under an oil bath, warmed to 100 °C, and reacted overnight with the temperature controlled. After the reaction was completed, the reaction solution was directly mixed with silica gel and purified by column chromatography to obtain compound 15-2.

MS (ESI, [M+H]⁺) *m*/*z*: 244.18.

### Step C: Preparation of compound 15-3

To a 50 mL single-necked flask were added compound 15-2 (280 mg) and anhydrous tetrahydrofuran (10 mL) in sequence. The reaction system was transferred to an ice salt bath under nitrogen atmosphere. A 2.5 M solution (0.9 mL) of lithium aluminum hydride in tetrahydrofuran was slowly added dropwise, and the mixture was reacted for 0.5 h with the temperature maintained. After the reaction was completed, water was added to quench the reaction, and the mixture was mixed with silica gel and purified by column chromatography to obtain compound 15-3.

MS (ESI, [M+H]⁺) *m*/*z*: 216.17.

### Step D: Preparation of compound 15-4

Referring to the method of step H in Example 2, compound 15-4 was prepared by reacting compound 15-3 with 2-iodoxybenzoic acid.

MS (ESI, [M+H]⁺) *m*/*z*: 214.21.

### Step E: Preparation of compound 15

Referring to the method of step I in Example 1, compound 15 was prepared by reacting compound 15-4 with compound 2-10.

¹H NMR (500 MHz, DMSO-*d*₆) δ 11.50 (s, 1H), 8.27 (d, *J =* 4.8 Hz, 1H), 7.93 (s, 1H), 7.67 (s, 1H), 7.32 (d, *J =* 8.1 Hz, 1H), 7.17 (dd, *J =* 8.1, 6.3 Hz, 1H), 3.79 - 3.61 (m, 6H), 3.56 (q, *J =* 6.3 Hz, 1H), 3.40 (t, *J =* 6.9 Hz, 4H), 3.09 (q, *J =* 7.0, 6.4 Hz, 3H), 2.77 (d, *J =* 4.8 Hz, 4H), 2.11 (p, *J =* 8.5, 8.0 Hz, 2H).

HRMS (ESI, [M+H]⁺) *m*/*z:* 419.2197.

### Example 16: Preparation of Compound 16

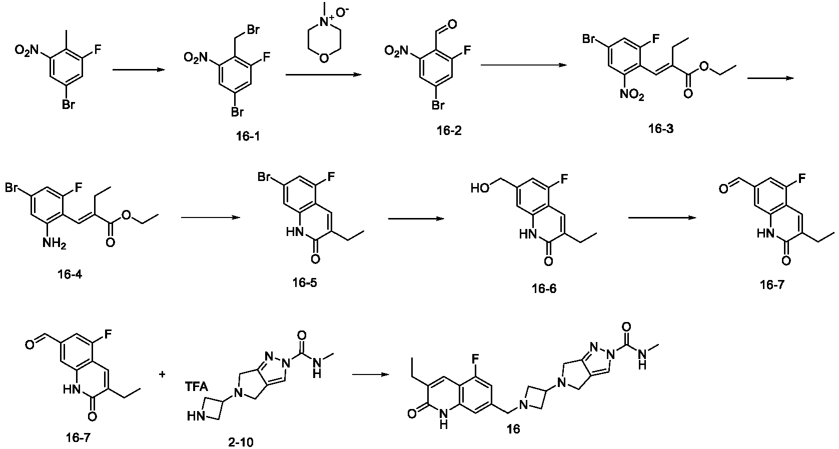

### Step A: Preparation of compound 16-1

To a 250 mL single-necked flask were added 4-bromo-2-fluoro-6-nitrotoluene (10 g), carbon tetrachloride (100 mL), *N*-bromosuccinimide (9.13 g), and dibenzoyl peroxide (1.04 g) in sequence. The mixture was stirred at 90 °C overnight under an oil bath under reflux under nitrogen atmosphere. After the reaction was completed, the mixture was extracted, concentrated, and purified by column chromatography to obtain 10 g of compound 16-1. ¹H NMR (500 MHz, DMSO-*d*₆) δ 8.22 - 8.09 (m, 2H), 4.73 (d, *J =* 1.6 Hz, 2H).

### Step B: Preparation of compound 16-2

To a 25 mL single-necked flask were added compound 16-1 (1 g), acetonitrile (15 mL), 4A molecular sieve (2.5 g), and *N*-methylmorpholine oxide (0.75 g) in sequence. The mixture was stirred at room temperature for 2 h under nitrogen atmosphere. After the reaction was completed, the mixture was extracted, concentrated, and purified by column chromatography to obtain 0.64 g of compound 16-2.

¹H NMR (500 MHz, DMSO-*d*₆) δ 10.13 (s, 1H), 8.29 - 8.27 (m, 1H), 8.25 (dd, *J* = 9.5, 1.7 Hz, 1H).

### Step C: Preparation of compound 16-3

To a 100 mL three-necked flask were added anhydrous tetrahydrofuran (10 mL) and sodium hydride (0.25 g, 60% mass content) in sequence, and the system was kept under nitrogen atmosphere until no gas was generated. The reaction system was transferred to an ice salt bath and cooled to 0-5 °C. Ethyl 2-(diethoxyphosphoryl)butyrate (0.98 g) was slowly added dropwise through a disposable syringe with the temperature controlled at 0-5 °C. After the dropwise addition was completed, the reaction system was stirred at 0-5 °C for 30 min. The reaction system was slightly turbid, and the reaction system was transferred to 40 °C and stirred for 5 min to become brown clear liquid. The reaction system was transferred to -78 °C, and a solution (5 mL) of compound 16-2 (0.64 g) in tetrahydrofuran was slowly added dropwise. After the dropwise addition was completed, the reaction system was stirred at -78 °C for 1 h. After the reaction was completed, 20 mL of a saturated aqueous ammonium chloride solution was added to the reaction system to quench the reaction. The reaction system was then extracted, concentrated, and purified by column chromatography to obtain 0.6 g of compound 16-3.

¹H NMR (500 MHz, DMSO-*d*₆) δ 8.29 - 8.21 (m, 1H), 8.18 (dd, *J =* 8.8, 1.8 Hz, 1H), 7.34 (s, 1H), 4.24 (q, *J =* 7.1 Hz, 2H), 2.09 (q, *J =* 7.4 Hz, 2H), 1.28 (t, *J=* 7.1 Hz, 3H), 0.88 (t, *J* = 7.4 Hz, 3H).

MS (ESI, [M+H]⁺) *m*/*z*: 345.96.

### Step D: Preparation of compound 16-4

To a 50 mL single-necked flask were added compound 16-3 (0.6 g), ethanol (6 mL), acetic acid (5 mL), and iron powder (0.3 g) in sequence. The mixture was stirred at 80 °C for 1 h under nitrogen atmosphere. After the reaction was completed, the mixture was filtered, concentrated, washed with a saturated aqueous sodium bicarbonate solution, extracted with ethyl acetate, dried, and purified by column chromatography to obtain 0.16 g of compound 16-4.

¹H NMR (500 MHz, DMSO-*d*₆) δ 7.00 (s, 1H), 6.71 (d, *J =* 0.7 Hz, 1H), 6.61 (dd, *J =* 9.3, 1.7 Hz, 1H), 5.63 (s, 2H), 4.21 (q, *J =* 7.1 Hz, 2H), 2.14 (q, *J =* 7.3 Hz, 2H), 1.28 *(t, J=* 7.1 Hz, 3H), 0.93 (t, *J* = 7.4 Hz, 3H).

### Step E: Preparation of compound 16-5

To a 15 mL microwave tube were added compound 16-4 (0.2 g), ethanol (2 mL), and acetic acid (2 mL) in sequence. The reaction system was transferred to a photocatalytic synthesizer and stirred for 24 h at room temperature under the wavelength of 450 nM. After the reaction was completed, the target compound was precipitated from the reaction system. The mixture was directly filtered and dried to obtain 0.07 g of compound 16-5.

¹H NMR (500 MHz, DMSO-*d*₆) δ 12.03 (s, 1H), 7.71 (s, 1H), 7.29 (s, 2H), 2.51 - 2.20 (m, 2H), 1.16 (s, 3H).

### Step F: Preparation of compound 16-6

Referring to the method of step F in Example 2, compound 16-6 was prepared by reacting compound 16-5 with (tributyltin)methanol.

MS (ESI, [M+H]⁺) *m*/*z*: 222.33.

### Step G: Preparation of compound 16-7

Referring to the method of step A in Example 2, compound 16-7 was prepared by reacting compound 16-6 with 2-iodoxybenzoic acid.

MS (ESI, [M+H]⁺) *m*/*z*: 220.16.

### Step H: Preparation of compound 16

Referring to the method of step I in Example 1, compound 16 was prepared by reacting compound 16-7 with compound 2-10.

¹H NMR (500 MHz, DMSO-*d*₆) δ 11.90 (s, 1H), 8.28 (d, *J =* 4.6 Hz, 1H), 7.95 (s, 1H), 7.72 (s, 1H), 7.08 (s, 1H), 6.91 (d, *J =* 10.7 Hz, 1H), 3.79 - 3.64 (m, 6H), 3.64 - 3.55 (m, 1H), 3.45 (s, 2H), 3.14 (s, 2H), 2.78 (d, *J =* 4.6 Hz, 3H), 2.57 - 2.51 (m, 2H), 1.16 (t, *J =* 7.4 Hz, 3H).

HRMS (ESI, [M+H]⁺) *m*/*z:* 425.2090.

### Example 17: Preparation of Compound 17

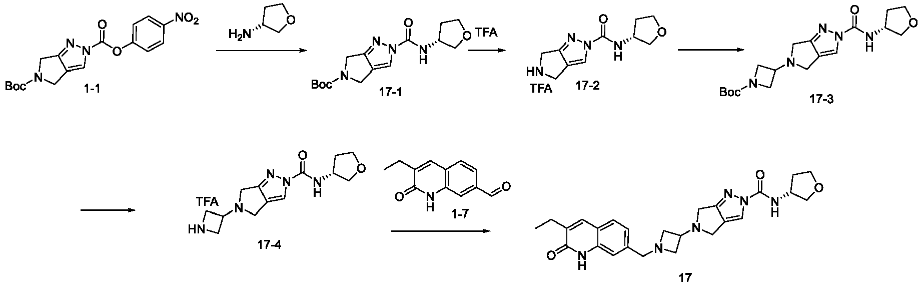

### Step A: Preparation of compound 17-1

Referring to the method of step B in Example 1, compound 17-1 was prepared by reacting compound 1-1 with (*R*)-3-aminotetrahydrofuran.

MS (ESI, [M+H]⁺) *m*/*z*: 323.15.

### Step B: Preparation of compound 17-2

Referring to the method of step C in Example 1, compound 17-2 was prepared by reacting compound 17-1 with trifluoroacetic acid.

MS (ESI, [M+H]⁺) *m*/*z*: 223.19.

### Step C: Preparation of compound 17-3

Referring to the method of step I in Example 2, compound 17-3 was prepared by reacting compound 17-2 with *tert*-butyl 3-oxoazetidine-1-carboxylate.

MS (ESI, [M+H]⁺) *m*/*z*: 378.30.

### Step D: Preparation of compound 17-4

Referring to the method of step C in Example 1, compound 17-4 was prepared by reacting compound 17-3 with trifluoroacetic acid.

MS (ESI, [M+H]⁺) *m*/*z*: 278.19.

### Step E: Preparation of compound 17

Referring to the method of step E in Example 1, compound 17 was prepared by reacting compound 1-7 with compound 17-4.

¹H NMR (500 MHz, DMSO-*d*₆) δ 11.63 (s, 1H), 8.34 (d, *J =* 6.2 Hz, 1H), 7.95 (s, 1H), 7.67 (s, 1H), 7.53 (d, *J =* 7.6 Hz, 1H), 7.23 (s, 1H), 7.06 (d, *J =* 7.5 Hz, 1H), 4.35 (s, 1H), 3.89 - 3.79 (m, 2H), 3.78 - 3.63 (m, 7H), 3.63 - 3.55 (m, 2H), 3.41 (s, 2H), 3.09 (s, 2H), 2.48 (s, 2H), 2.18 - 1.95 (m, 2H), 1.16 (t, *J =* 6.9 Hz, 3H).

HRMS (ESI, [M+H]⁺) *m*/*z:* 463.2462.

### Example 18: Preparation of Compound 18

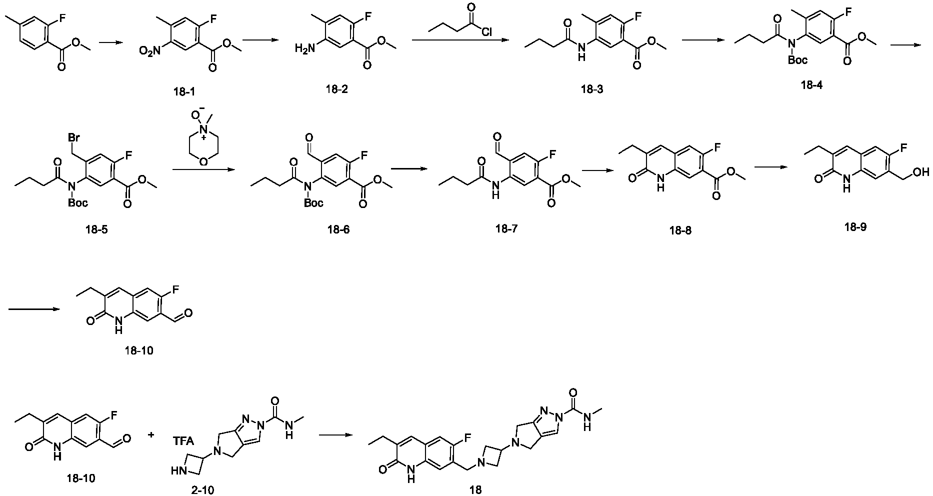

### Step A: Preparation of compound 18-1

To a 500 mL three-necked flask were added methyl 2-fluoro-4-methylbenzoate (18 g) and concentrated sulfuric acid (200 mL). After the addition was completed, the mixture was placed under an ice bath, cooled to 0 °C, and stirred, followed by slowly adding potassium nitrate (16.24 g) in portions while keeping the internal temperature of the reaction at not higher than 10 °C. After the reaction was completed, the reaction solution was poured into 1 L of ice water, and the mixture was extracted with a proper amount of EA. The organic phase was washed with a saturated aqueous sodium chloride solution, concentrated, and purified by column chromatography to obtain compound 18-1.

### Step B: Preparation of compound 18-2

Referring to the method of step D in Example 16, compound 18-2 was prepared by reacting compound 18-1 with iron powder.

MS (ESI, [M-H]⁻) *m*/*z*: 182.12.

### Step C: Preparation of compound 18-3

Referring to the method of step D in Example 1, compound 18-3 was prepared by reacting compound 18-2 with *n*-butyryl chloride.

MS (ESI, [M-H]⁻) *m*/*z:* 252.21.

### Step D: Preparation of compound 18-4

To a 500 mL three-necked flask were added compound 18-3 (23 g), dichloromethane (250 mL), triethylamine (32 mL), di-tert-butyl dicarbonate (25.8 g), and 4-dimethylaminopyridine (555 mg). After the addition was completed, the mixture was reacted at room temperature. After the reaction was completed, the reaction solution was washed with a saturated aqueous ammonium chloride solution and a saturated aqueous sodium chloride solution, concentrated, and purified by column chromatography to obtain compound 18-4.

MS (ESI, [M-Boc+H]⁺) *m*/*z:* 254.25.

### Step E: Preparation of compound 18-5

To a 1 L three-necked flask were added compound 18-4 (29.3 g), dichloroethane (500 mL), N-bromosuccinimide (14.6 g), and azobisisobutyronitrile (1.17 g). After the addition was completed, the system was purged with nitrogen, and the mixture was heated to 80 °C under nitrogen atmosphere and reacted. After the reaction was completed, the reaction solution was washed with a saturated aqueous sodium thiosulfate solution and a saturated aqueous sodium chloride solution, dried over anhydrous sodium sulfate, and concentrated to obtain compound 18-5.

MS (ESI, [M-Boc+H]⁺) *m*/*z:* 332.04.

### Step F: Preparation of compound 18-6

Referring to the method of step B in Example 16, compound 18-6 was prepared by reacting compound 18-5 with *N*-methylmorpholine oxide.

MS (ESI, [M-Boc+H]⁺) *m*/*z:* 268.15.

### Step G: Preparation of compound 18-7

Referring to the method of step C in Example 1, compound 18-7 was prepared by reacting compound 18-6 with trifluoroacetic acid.

MS (ESI, [M-H]⁻) *m*/*z*: 266.12.

### Step H: Preparation of compound 18-8

To a 250 mL three-necked flask were added compound 18-7 (4.4 g), N,N-dimethylformamide (120 mL), and potassium carbonate (11.4 g). After the addition was completed, the system was purged with nitrogen, and the mixture was heated to 60 °C under nitrogen atmosphere and reacted. After the reaction was completed, the mixture was concentrated by evaporation under reduced pressure to remove the solvent, and a proper amount of a saturated aqueous sodium chloride solution and ethyl acetate were added to the residue. The mixture was stirred for a while, and the phases were separated. The organic phases were concentrated and purified by column chromatography to obtain compound 18-8.

MS (ESI, [M+H]⁺) *m*/*z*: 250.18.

### Step I: Preparation of compound 18-9

Referring to the method of step C in Example 15, compound 18-9 was prepared by reacting compound 18-8 with a solution of lithium aluminum hydride in tetrahydrofuran.

MS (ESI, [M-H]⁻) *m*/*z*: 220.19.

### Step J: Preparation of compound 18-10

Referring to the method of step A in Example 2, compound 18-10 was prepared by reacting compound 18-9 with 2-iodoxybenzoic acid.

MS (ESI, [M-H]⁻) *m*/*z*: 218.18.

### Step K: Preparation of compound 18

Referring to the method of step I in Example 1, compound 18 was prepared by reacting compound 18-10 with compound 2-10.

¹H NMR (500 MHz, DMSO-*d*₆) δ 11.69 (s, 1H), 8.28 (q, *J =* 4.7 Hz, 1H), 7.95 (s, 1H), 7.67 (s, 1H), 7.43 (d, *J =* 10.2 Hz, 1H), 7.33 (d, *J =* 6.3 Hz, 1H), 3.73 - 3.71 (m, 2H), 3.70 - 3.66 (m, 4H), 3.63 - 3.54 (m, 2H), 3.47 - 3.41 (m, 2H), 3.17 - 3.09 (m, 2H), 2.78 (d, *J =* 4.7 Hz, 3H), 2.49 - 2.45 (m, 1H), 1.15 (t, *J =* 7.4 Hz, 3H). HRMS (ESI, [M+H]⁺) m/z:425.2095.

### Example 19: Preparation of Compound 19

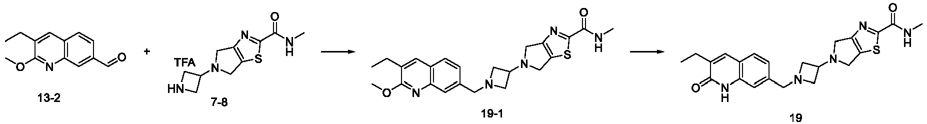

### Step A: Preparation of compound 19-1

Referring to the method of step I in Example 1, compound 19-1 was prepared by reacting compound 13-2 with compound 7-8.

MS (ESI, [M+H]⁺) *m*/*z*: 438.48*.*

### Step B: Preparation of compound 19

Referring to the method of step D in Example 13, compound 19 was prepared by reacting compound 19-1 with a 4 M solution of hydrogen chloride in dioxane.

¹H NMR (500 MHz, DMSO-*d*₆) δ 11.67 (s, 1H), 8.75 (d, *J =* 4.6 Hz, 1H), 7.68 (s, 1H), 7.54 (d, *J =* 8.0 Hz, 1H), 7.24 (s, 1H), 7.07 (d, *J =* 7.9 Hz, 1H), 4.03 (s, 2H), 3.89 (s, 2H), 3.72 (s, 2H), 3.69 - 3.59 (m, 1H), 3.46 (s, 2H), 3.15 (s, 2H), 2.78 (d, *J =* 4.6 Hz, 3H), 2.47 (d, *J =* 7.3 Hz, 2H), 1.16 (t, *J =* 7.4 Hz, 3H).

HRMS (ESI, [M+H]⁺) *m*/*z:* 424.1802.

### Example 20: Preparation of Compound 20

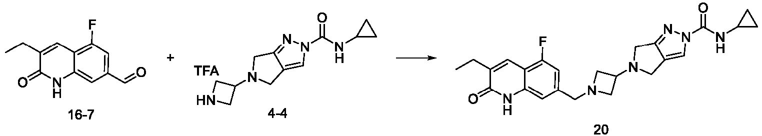

### Step A: Preparation of compound 20

Referring to the method of step I in Example 1, compound 20 was prepared by reacting compound 16-7 with compound 4-4.

¹H NMR (500 MHz, DMSO-*d*₆) δ 11.91 (s, 1H), 8.39 (d, *J =* 3.6 Hz, 1H), 7.95 (s, 1H), 7.73 (s, 1H), 7.09 (s, 1H), 6.93 (d, *J* = 10.7 Hz, 1H), 3.70 (t, *J =* 13.3 Hz, 6H), 3.61 (d, *J =* 5.7 Hz, 1H), 3.48 (s, 2H), 3.19 (s, 2H), 2.79 - 2.70 (m, 1H), 2.56 - 2.51 (m, 2H), 1.16 (t, *J =* 7.4 Hz, 3H), 0.66 (d, *J =* 7.1 Hz, 4H).

HRMS (ESI, [M+H]⁺) *m*/*z:* 451.2260.

### Example 21: Preparation of Compound 21

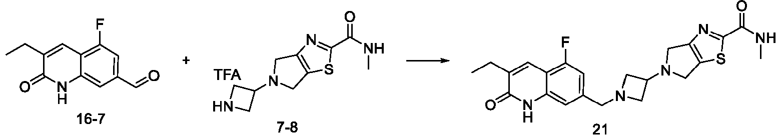

### Step A: Preparation of compound 21

Referring to the method of step I in Example 1, compound 21 was prepared by reacting compound 16-7 with compound 7-8.

¹H NMR (500 MHz, DMSO-*d*₆) δ 11.89 (s, 1H), 8.74 (d, *J =* 4.7 Hz, 1H), 7.73 (s, 1H), 7.07 (s, 1H), 6.91 (d, *J =* 10.8 Hz, 1H), 4.03 (d, *J =* 2.9 Hz, 2H), 3.89 (d, *J* = 2.9 Hz, 2H), 3.73 - 3.60 (m, 3H), 3.43 (s, 2H), 3.11 (s, 2H), 2.77 (d, *J =* 4.8 Hz, 3H), 2.53 (d, *J =* 7.3 Hz, 2H), 1.16 (t, *J =* 7.4 Hz, 3H).

HRMS (ESI, [M+H]⁺) *m*/*z:* 442.1707.

### Example 22: Preparation of Compound 22

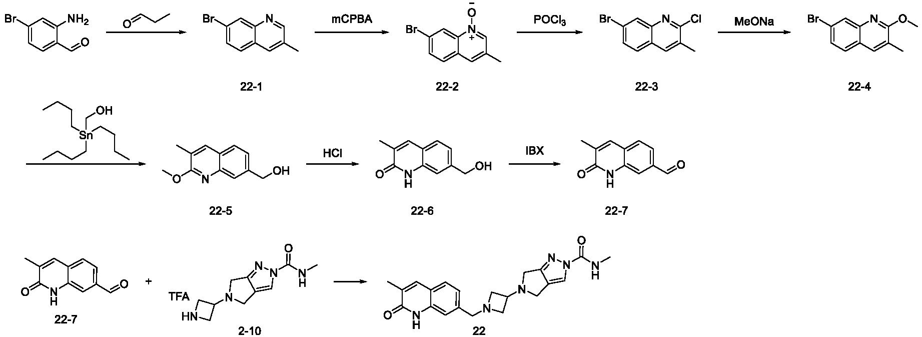

### Step A: Preparation of compound 22-1

To a 250 mL single-necked flask were added 2-amino-4-bromobenzaldehyde (5 g), ethanol (100 mL), propionaldehyde (2.9 g), and potassium hydroxide (5.61 g). After the addition was completed, the mixture was heated to 90 °C and reacted. After the reaction was completed, the mixture was concentrated by evaporation under reduced pressure to remove ethanol. The remaining solid was washed with a saturated aqueous ammonium chloride solution, extracted with ethyl acetate, concentrated, and purified by column chromatography to obtain compound 22-1.

MS (ESI, [M+H]⁺) *m*/*z*: 222.04.

### Step B: Preparation of compound 22-2

Referring to the method of step C in Example 2, compound 22-2 was prepared by reacting compound 22-1 with m-chloroperoxybenzoic acid.

MS (ESI, [M+H]⁺) *m*/*z*: 238.06.

### Step C: Preparation of compound 22-3

Referring to the method of step D in Example 2, compound 22-3 was prepared by reacting compound 22-2 with phosphorus oxychloride.

MS (ESI, [M+H]⁺) *m*/*z*: 255.97.

### Step D: Preparation of compound 22-4

Referring to the method of step E in Example 2, compound 22-4 was prepared by reacting compound 22-3 with sodium methoxide.

MS (ESI, [M+H]⁺) *m*/*z*: 252.08.

### Step E: Preparation of compound 22-5

Referring to the method of step F in Example 2, compound 22-5 was prepared by reacting compound 22-4 with (tributyltin)methanol.

MS (ESI, [M+H]⁺) *m*/*z*: 204.15*.*

### Step F: Preparation of compound 22-6

Referring to the method of step G in Example 2, compound 22-6 was prepared by reacting compound 22-4 with hydrochloric acid.

MS (ESI, [M+H]⁺) *m*/*z*: 190.15*.*

### Step G: Preparation of compound 22-7

Referring to the method of step A in Example 2, compound 22-7 was prepared by reacting compound 22-6 with 2-iodoxybenzoic acid.

MS (ESI, [M-H]⁻) *m*/*z*: 186.11.

### Step H: Preparation of compound 22

Referring to the method of step I in Example 1, compound 22 was prepared by reacting compound 22-7 with compound 2-10.

¹H NMR (500 MHz, Chloroform-d) δ 8.90 (s, 1H), 7.88 (s, 1H), 7.58 (s, 1H), 7.43 (d, *J =* 8.1 Hz, 1H), 7.11 (d, *J* = 7.7 Hz, 1H), 7.06 (s, 1H), 6.92 (d, *J* = 4.3 Hz, 1H), 3.73 (s, 6H), 3.64 - 3.61 (m, 1H), 3.56 - 3.52 (m, 2H), 3.21 - 3.08 (m, 2H), 3.00 (d, *J =* 4.9 Hz, 3H), 2.25 (s, 3H).

HRMS (ESI, [M+H]⁺) *m*/*z:* 393.2029.

### Example 23: Preparation of Compound 23

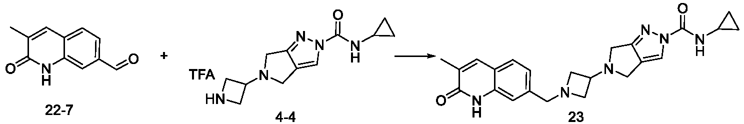

### Step A: Preparation of compound 23

Referring to the method of step I in Example 1, compound 23 was prepared by reacting compound 22-7 with compound 4-4.

¹H NMR (500 MHz, Chloroform-d) δ 10.86 (s, 1H), 7.88 (s, 1H), 7.60 (s, 1H), 7.45 (d, *J =* 8.0 Hz, 1H), 7.32 (s, 1H), 7.19 (d, *J* = 7.9 Hz, 1H), 7.07 (s, 1H), 3.90 (s, 2H), 3.72 (d, *J =* 3.4 Hz, 7H), 3.32 (s, 2H), 2.84 - 2.78 (m, 1H), 2.27 (s, 3H), 0.87 - 0.83 (m, 2H), 0.69 - 0.65 (m, 2H).

HRMS (ESI, [M+H]⁺) m/z:419.2199.

### Example 24: Preparation of Compound 24

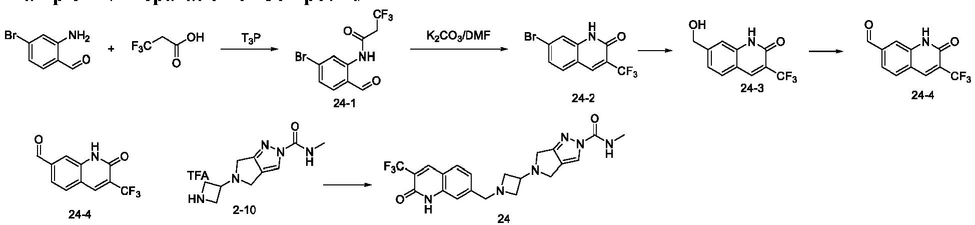

### Step A: Preparation of compound 24-1

To a 100 mL single-necked flask were added 2-amino-4-bromobenzaldehyde (1 g), dichloromethane (10 mL), 3,3,3-trifluoropropionic acid (0.83 g), *N,N*-diisopropylethylamine (1.3 g), and 1-propylphosphoric anhydride (4.1 g) in sequence. The mixture was stirred at room temperature for 30 min under nitrogen atmosphere. After the reaction was completed, 10 mL of water was added to the reaction solution to quench the reaction, and the mixture was extracted three times with dichloromethane (50 mL). The organic phases were washed with a saturated aqueous sodium bicarbonate solution, dried over anhydrous sodium sulfate, filtered, mixed with silica gel, and purified by column chromatography to obtain 1.2 g of compound 24-1.

MS (ESI, [M-H]⁻) *m*/*z:* 308.06.

### Step B: Preparation of compound 24-2

To a 100 mL single-necked flask were added compound 24-1 (0.9 g), *N,N*-dimethylformamide (10 mL), and potassium carbonate (1.2 g) in sequence. The mixture was stirred at 60 °C under an oil bath for 2 h under nitrogen atmosphere. After the reaction was completed, the mixture was filtered to remove insoluble substances, and the filtrate was directly concentrated to remove the solvent. To the crude product was added 50 mL of ethyl acetate for reconstitution. The organic phase was washed with a saturated aqueous sodium chloride solution, dried over anhydrous sodium sulfate, filtered, mixed with silica gel, and purified by column chromatography to obtain 0.6 g of compound 24-2.

MS (ESI, [M+H]⁺) *m*/*z*: 292.03.

### Step C: Preparation of compound 24-3

Referring to the method of step F in Example 2, compound 24-3 was prepared by reacting compound 24-2 with (tributyltin)methanol.

MS (ESI, [M+H]⁺) *m*/*z*: 244.29.

### Step D: Preparation of compound 24-4

Referring to the method of step A in Example 2, compound 24-4 was prepared by reacting compound 24-3 with 2-iodoxybenzoic acid.

MS (ESI, [M-H]⁻) *m*/*z:* 240.14.

### Step E: Preparation of compound 24

Referring to the method of step I in Example 1, compound 24 was prepared by reacting compound 24-4 with compound 2-10.

¹H NMR (500 MHz, DMSO-*d*₆) δ 12.25 (s, 1H), 8.51 (s, 1H), 8.28 (d, *J =* 4.6 Hz, 1H), 7.95 (s, 1H), 7.79 (d, *J* = 8.1 Hz, 1H), 7.32 (s, 1H), 7.19 (d, *J =* 8.1 Hz, 1H), 3.72 (d, *J =* 5.1 Hz, 4H), 3.68 (s, 2H), 3.64 - 3.56 (m, 1H), 3.43 (t, *J =* 6.3 Hz, 2H), 3.12 (s, 2H), 2.78 (d, *J =* 4.6 Hz, 3H).

HRMS (ESI, [M+H]⁺) *m*/*z:* 447.1757.

### Example 25: Preparation of Compound 25

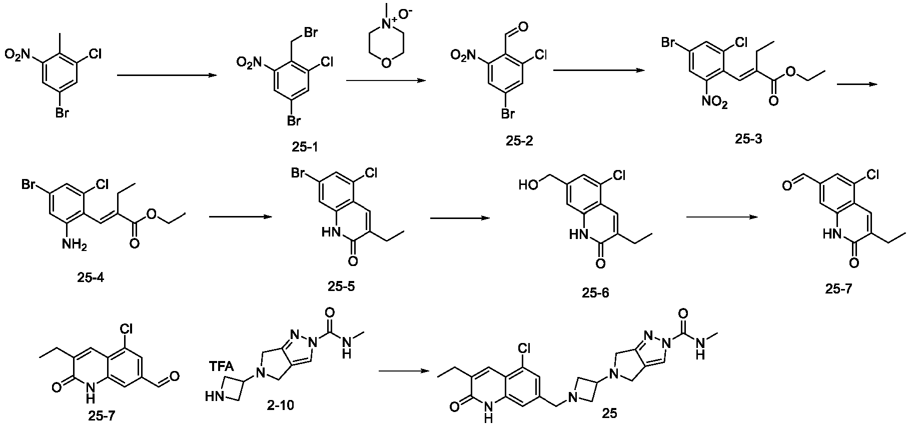

### Step A: Preparation of compound 25-1

Referring to the method of step A in Example 16, compound 25-1 was prepared by reacting 3-chloro-5-bromo-2-methylnitrobenzene with N-bromosuccinimide.

### Step B: Preparation of compound 25-2

Referring to the method of step B in Example 16, compound 25-2 was prepared by reacting compound 25-1 with *N*-methylmorpholine oxide.

### Step C: Preparation of compound 25-3

Referring to the method of step C in Example 16, compound 25-3 was prepared by reacting compound 25-2 with ethyl 2-(diethoxyphosphoryl)butyrate.

MS (ESI, [M+H]⁺) *m*/*z:* 362.14.

### Step D: Preparation of compound 25-4

Referring to the method of step D in Example 16, compound 25-4 was prepared by reacting compound 25-3 with iron powder.

### Step E: Preparation of compound 25-5

Referring to the method of step E in Example 16, compound 25-5 was prepared by reacting compound 25-4 in a photocatalytic synthesizer.

MS (ESI, [M+H]⁺) *m*/*z*: 286.01

### Step F: Preparation of compound 25-6

Referring to the method of step F in Example 2, compound 25-6 was prepared by reacting compound 25-5 with (tributyltin)methanol.

MS (ESI, [M+H]⁺) *m*/*z*: 238.17.

### Step G: Preparation of compound 25-7

Referring to the method of step A in Example 2, compound 25-7 was prepared by reacting compound 25-6 with 2-iodoxybenzoic acid.

MS (ESI, [M-H]⁻) *m*/*z:* 234.12.

### Step H: Preparation of compound 25

Referring to the method of step I in Example 1, compound 25 was prepared by reacting compound 25-7 with compound 2-10.

¹H NMR (500 MHz, DMSO-*d*₆) δ 11.93 (s, 1H), 8.28 (q, *J=* 4.6 Hz, 1H), 7.94 (s, 1H), 7.83 (s, 1H), 7.25 - 7.18 (m, 2H), 3.70 (s, 2H), 3.66 (d, *J =* 9.0 Hz, 4H), 3.59 (p, *J =* 6.3 Hz, 1H),3.43 - 3.40 (m, 2H), 3.11 - 3.06 (m, 2H), 2.78 (d, *J =* 4.6 Hz, 3H), 2.57 - 2.52 (m, 2H), 1.17 *(t, J=* 7.4 Hz, 3H).

HRMS (ESI, [M+H]⁺) m/z:441.1801

### Example 26: Preparation of Compound 26

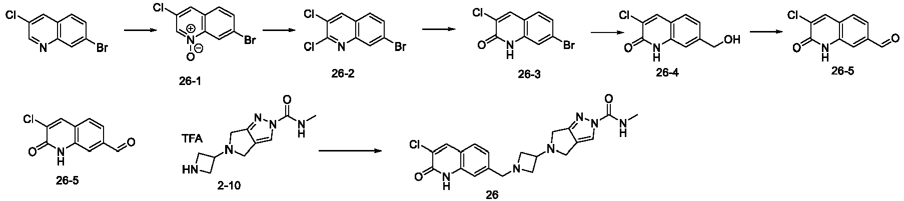

### Step A: Preparation of compound 26-1

Referring to the method of step C in Example 2, compound 26-1 was prepared by reacting 7-bromo-3-chloroquinoline with m-chloroperoxybenzoic acid.

MS (ESI, [M+H]⁺) *m*/*z:* 258.16.

### Step B: Preparation of compound 26-2

Referring to the method of step D in Example 2, compound 26-2 was prepared by reacting compound 26-1 with phosphorus oxychloride.

MS (ESI, [M+H]⁺) *m*/*z*: 276.07.

### Step C: Preparation of compound 26-3

To a 15 mL microwave tube were added compound 26-2 (0.4 g) and concentrated hydrochloric acid (5 mL) in sequence. The reaction system was transferred to a microwave reactor, heated to 100 °C under 150 W, and reacted for 2 h. After the reaction was completed, the pH was adjusted to alkalinity with a saturated aqueous sodium bicarbonate solution, and the mixture was extracted three times with ethyl acetate (50 mL). The organic phase was washed with a saturated aqueous sodium chloride solution, dried over anhydrous sodium sulfate, filtered, mixed with silica gel, and purified by column chromatography to obtain 0.35 g of compound 26-3.

MS (ESI, [M-H]⁻) *m*/*z:* 255.96.

### Step D: Preparation of compound 26-4

Referring to the method of step F in Example 2, compound 26-4 was prepared by reacting compound 26-3 with (tributyltin)methanol.

MS (ESI, [M+H]⁺) *m*/*z*: 210.15.

### Step E: Preparation of compound 26-5

Referring to the method of step A in Example 2, compound 26-5 was prepared by reacting compound 26-4 with 2-iodoxybenzoic acid.

MS (ESI, [M-H]⁻) *m*/*z*: 206.10.

### Step F: Preparation of compound 26

Referring to the method of step I in Example 1, compound 26 was prepared by reacting compound 26-5 with compound 2-10.

¹H NMR (500 MHz, DMSO-*d*₆) δ 12.22 (s, 1H), 8.27 (d, *J =* 5.7 Hz, 2H), 7.94 (s, 1H), 7.60 (d, *J =* 8.1 Hz, 1H), 7.29 (s, 1H), 7.14 (d, *J =* 8.0 Hz, 1H), 3.70 (s, 2H), 3.67 (d, *J =* 3.9 Hz, 4H), 3.58 (p, *J =* 6.1 Hz, 1H), 3.40 *(t, J=* 6.9 Hz, 2H), 3.08 (t, *J =* 6.7 Hz, 2H), 2.78 (d, *J =* 4.6 Hz, 3H).

HRMS (ESI, [M+H]⁺) *m*/*z:* 413.1490.

### Example 27: Preparation of Compound 27

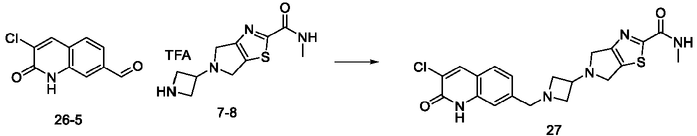

### Step A: Preparation of compound 27

Referring to the method of step I in Example 1, compound 27 was prepared by reacting compound 26-5 with compound 7-8.

¹H NMR (500 MHz, DMSO-*d*₆) δ 12.25 (s, 1H), 8.75 (d, *J =* 4.8 Hz, 1H), 8.27 (s, 1H), 7.61 (d, *J =* 8.1 Hz, 1H), 7.29 (s, 1H), 7.15 (d, *J =* 8.1 Hz, 1H), 4.07 - 3.98 (m, 2H), 3.89 (d, *J =* 2.8 Hz, 2H), 3.74 (d, *J =* 16.4 Hz, 2H), 3.68 - 3.61 (m, 1H), 3.45 (t, *J =* 6.6 Hz, 2H), 3.13 (s, 2H), 2.78 (d, *J =* 4.8 Hz, 3H).

HRMS (ESI, [M+H]⁺) *m*/*z:* 430.1103.

### Example 28: Preparation of Compound 28

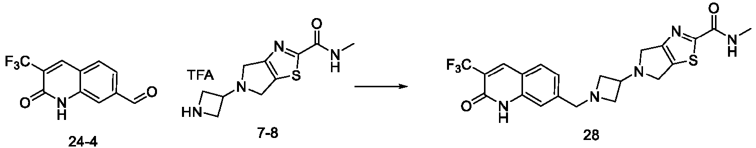

### Step A: Preparation of compound 28

Referring to the method of step I in Example 1, compound 28 was prepared by reacting compound 24-4 with compound 7-8.

¹H NMR (500 MHz, DMSO-*d*₆) δ 12.26 (s, 1H), 8.74 (d, *J =* 4.7 Hz, 1H), 8.52 (s, 1H), 7.80 (d, *J =* 8.1 Hz, 1H), 7.32 (s, 1H), 7.20 (d, *J =* 8.1 Hz, 1H), 4.04 (s, 2H), 3.90 (s, 2H), 3.75 (s, 2H), 3.69 - 3.61 (m, 1H), 3.45 (s, 2H), 3.14 (s, 2H), 2.78 (d, *J =* 4.7 Hz, 3H).

HRMS (ESI, [M+H]⁺) *m*/*z:* 464.1379.

### Example 29: Preparation of Compound 29

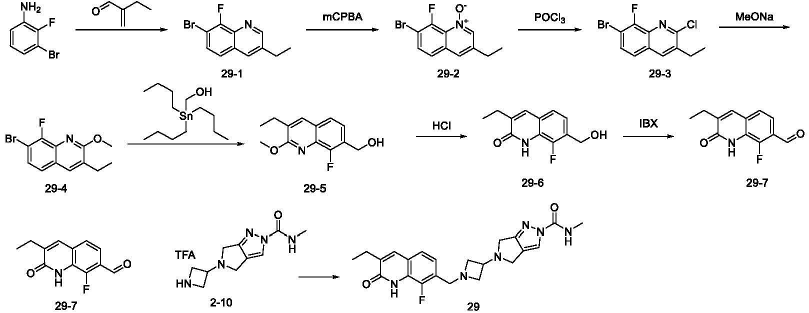

### Step A: Preparation of compound 29-1

To a 100 mL three-necked flask were added 3-bromo-2-fluoroaniline (1 g), sodium iodide (0.079 g), and 4 M sulfuric acid (25 mL). After the addition was completed, the system was purged with nitrogen, and the mixture was heated to 110 °C under nitrogen atmosphere. 2-Ethylacrolein (0.531 g) was then added dropwise to the reaction system. After the reaction was completed, the mixture was neutralized with a saturated aqueous sodium carbonate solution, extracted, concentrated, and purified by column chromatography to obtain compound 29-1.

MS (ESI, [M+H]⁺) *m*/*z:* 254.24.

### Step B: Preparation of compound 29-2

Referring to the method of step C in Example 2, compound 29-2 was prepared by reacting compound 29-1 with m-chloroperoxybenzoic acid.

MS (ESI, [M+H]⁺) *m*/*z:* 270.23.

### Step C: Preparation of compound 29-3

Referring to the method of step D in Example 2, compound 29-3 was prepared by reacting compound 29-2 with phosphorus oxychloride.

MS (ESI, [M+H]⁺) *m*/*z:* 288.16*.*

### Step D: Preparation of compound 29-4

Referring to the method of step E in Example 2, compound 29-4 was prepared by reacting compound 29-3 with sodium methoxide.

MS (ESI, [M+H]⁺) *m*/*z:* 284.22.

### Step E: Preparation of compound 29-5

Referring to the method of step F in Example 2, compound 29-5 was prepared by reacting compound 29-4 with (tributyltin)methanol.

MS (ESI, [M+H]⁺) *m*/*z:* 236.36.

### Step F: Preparation of compound 29-6

Referring to the method of step G in Example 2, compound 29-6 was prepared by reacting compound 29-5 with hydrochloric acid.

MS (ESI, [M+H]⁺) *m*/*z:* 222.18*.*

### Step G: Preparation of compound 29-7

Referring to the method of step A in Example 2, compound 29-7 was prepared by reacting compound 29-6 with 2-iodoxybenzoic acid.

MS (ESL [M-H]⁻) *m*/*z:* 218.17.

### Step H: Preparation of compound 29

Referring to the method of step I in Example 1, compound 29 was prepared by reacting compound 29-7 with compound 2-10.

¹H NMR (500 MHz, DMSO-*d*₆) δ 11.69 (s, 1H), 8.28 (d, *J =* 4.6 Hz, 1H), 7.93 (s, 1H), 7.73 (s, 1H), 7.40 (d, *J =* 8.0 Hz, 1H), 7.13 (t, *J* = 7.1 Hz, 1H), 3.70 (s, 2H), 3.68 (s, 2H), 3.65 (s, 2H), 3.55 (p, *J =* 6.2 Hz, 1H), 3.40 (t, *J* = 6.7 Hz, 2H), 3.10 (t, *J =* 6.5 Hz, 2H), 2.77 (d, *J =* 4.6 Hz, 3H), 2.48 (s, 2H), 1.17 (t, *J =* 7.4 Hz, 3H).

HRMS (ESI, [M+H]⁺) *m*/*z:* 425.2099.

### Example 30: Preparation of Compound 30

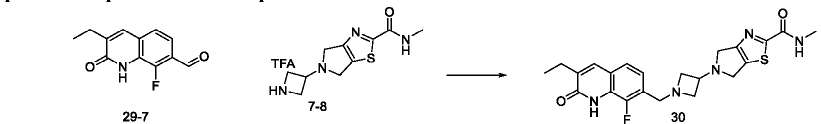

### Step A: Preparation of compound 30

Referring to the method of step I in Example 1, compound 30 was prepared by reacting compound 29-7 with compound 7-8.

¹H NMR (500 MHz, DMSO-*d*₆) δ 11.70 (s, 1H), 8.75 (d, *J =* 4.7 Hz, 1H), 7.73 (s, 1H), 7.40 (d, *J =* 8.1 Hz, 1H), 7.21 - 7.05 (m, 1H), 4.01 (s, 2H), 3.86 (s, 2H), 3.71 (s, 2H), 3.61 (p, *J =* 6.2 Hz, 1H), 3.40 (t, *J =* 6.9 Hz, 2H), 3.10 *(t, J=* 6.6 Hz, 2H), 2.77 *(d, J=* 4.7 Hz, 3H), 2.50 - 2.48 (m, 2H), 1.17 *(t, J=* 7.4 Hz, 3H).

HRMS (ESI, [M+H]⁺) *m*/*z:* 442.1709.

### Example 31: Preparation of Compound 31

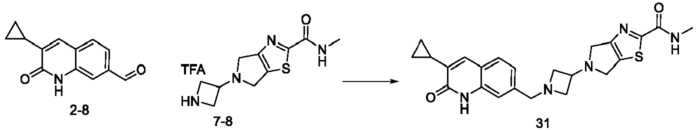

### Step A: Preparation of compound 31

Referring to the method of step I in Example 1, compound 31 was prepared by reacting compound 2-8 with compound 7-8.

¹H NMR (500 MHz, DMSO-*d*₆) δ 11.67 (s, 1H), 8.75 (d, *J =* 4.7 Hz, 1H), 7.47 (d, *J =* 8.0 Hz, 1H), 7.39 (s, 1H), 7.21 (s, 1H), 7.03 (d, *J =* 8.0 Hz, 1H), 4.02 (s, 2H), 3.88 (s, 2H), 3.63 (d, *J =* 8.2 Hz, 3H), 3.39 (t, *J =* 6.8 Hz, 2H), 3.06 (t, *J =* 6.5 Hz, 2H), 2.78 (d, *J =* 4.7 Hz, 3H), 2.15 - 2.01 (m, 1H), 0.99 - 0.84 (m, 2H), 0.72 (q, *J =* 5.5 Hz, 2H).

HRMS (ESI, [M+H]⁺) *m*/*z:* 436.1808.

### Example 32: Preparation of Compound 32

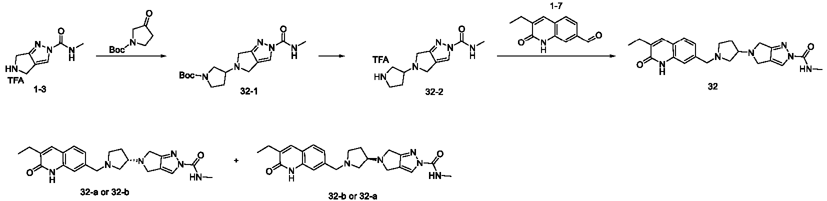

### Step A: Preparation of compound 32-1

Referring to the method of step I in Example 2, compound 32-1 was prepared by reacting compound 1-3 with *tert-butyl* 3-oxopyrrolidine-1-carboxylate.

MS (ESI, [M+H]⁺) *m*/*z:* 336.29.

### Step B: Preparation of compound 32-2

Referring to the method of step C in Example 1, compound 32-2 was prepared by reacting compound 32-1 with trifluoroacetic acid.

MS (ESI, [M+H]⁺) *m*/*z:* 236.29.

### Step C: Preparation of compound 32

Referring to the method of step I in Example 1, compound 32 was prepared by reacting compound 1-7 with compound 32-2. Compound 32 was subjected to resolution and preparation by chromatographic column ASA CHIRALPAK IG (30 × 250 mm, S-10 µm) (mobile phase A: ethanol-dichloromethane (2:1, V/V); mobile phase B: n-hexane; elution gradient: mobile phase A:mobile phase B = 75:25 (V/V); flow rate: 40 mL/min; detection wavelength: 254 nM) to obtain compounds 32-a and 32-b. The peak times were 13.28 min (compound 32-a) and 19.50 min (compound 32-b), respectively.

### Compound 32-a:

¹H NMR (500 MHz, DMSO-*d*₆) δ 11.65 (s, 1H), 8.26 (q, *J =* 4.6 Hz, 1H), 7.92 (s, 1H), 7.68 (s, 1H), 7.54 (d, *J =* 8.0 Hz, 1H), 7.25 (d, *J* = 1.6 Hz, 1H), 7.10 (dd, *J =* 8.1, 1.6 Hz, 1H), 3.74 - 3.55 (m, 6H), 2.84 - 2.69 (m, 4H), 2.65 - 2.52 (m, 4H), 2.49 - 2.41 (m, 2H), 2.04 - 1.92 (m, 1H), 1.82 - 1.72 (m, 1H), 1.16 (t, *J* = 7.4 Hz, 3H). HRMS (ESI, [M+H]⁺) *m*/*z:* 421.2353.

### Compound 32-b:

¹H NMR (500 MHz, DMSO-*d*₆) δ 11.65 (s, 1H), 8.26 (q, *J =* 4.6 Hz, 1H), 7.91 (s, 1H), 7.68 (s, 1H), 7.54 (d, *J =* 8.0 Hz, 1H), 7.25 (s, 1H), 7.10 (dd, *J =* 8.1, 1.5 Hz, 1H), 3.69 (p, *J =* 7.3 Hz, 4H), 3.60 (dd, *J =* 16.0, 12.7 Hz, 2H), 2.77 (d, *J =* 4.7 Hz, 4H), 2.65 - 2.50(m, 4H), 2.49 - 2.41 (m, 2H), 2.03 - 1.93 (m, 1H), 1.78 (tt, *J =* 13.5, 5.9 Hz, 1H), 1.15 (t, *J =* 7.4 Hz, 3H).

HRMS (ESI, [M+H]⁺) *m*/*z:* 421.2349.

### Example 33: Preparation of Compound 33

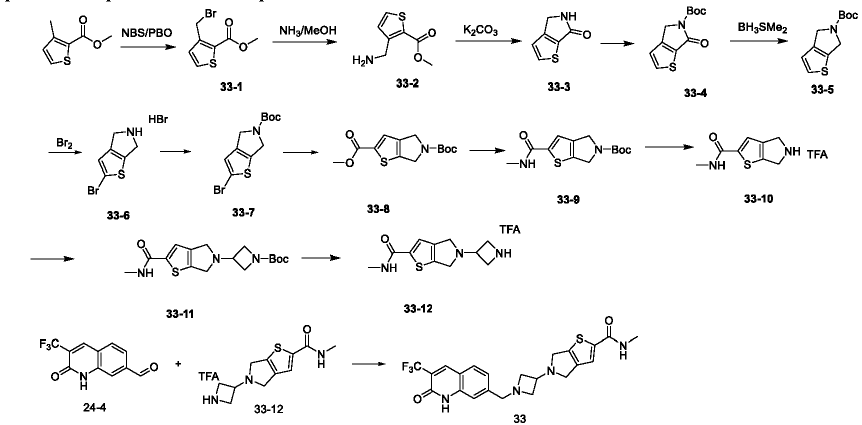

### Step A: Preparation of compound 33-1

To a 100 mL single-necked flask were added methyl 3-methylthiophene-2-carboxylate (2 g), chloroform (20 mL), N-bromosuccinimide (2.165 g), and a catalytic amount of benzoyl peroxide was added. The mixture was refluxed at 70 °C overnight. After the reaction was completed, the mixture was concentrated by rotary evaporation to remove the solvent, diluted with ethyl acetate (100 mL), washed with water, dried, and purified by column chromatography to obtain compound 33-1.

### Step B: Preparation of compound 33-2

To a 100 mL single-necked flask were added compound 33-1 (2.2 g) and a solution of ammonia in methanol, and the mixture was stirred at room temperature for 2 h. The reaction solution was directly concentrated to dryness by rotary evaporation, mixed with silica gel, and purified by column chromatography to obtain compound 33-2.

MS (ESI, [M+H]⁺) *m*/*z:* 172.08.

### Step C: Preparation of compound 33-3

To a 100 mL single-necked flask were added compound 33-2 (0.5 g), ethanol (5 mL), methanol (5.00 mL), and potassium carbonate (0.444 g), and the mixture was reacted under reflux at 90 °C overnight. After the reaction was completed, the reaction solution was concentrated to dryness by rotary evaporation, diluted with ethyl acetate (50 mL), washed with water, dried, and concentrated to dryness by rotary evaporation to obtain compound 33-3.

MS (ESI, [M+H]⁺) *m*/*z:* 140.01.

### Step D: Preparation of compound 33-4

Referring to the method of step D in Example 18, compound 33-4 was prepared by reacting compound 33-3 with di-tert-butyl dicarbonate.

MS (ESI, [M+H]⁺) *m*/*z:* 240.01.

### Step E: Preparation of compound 33-5

To a 100 mL three-necked flask were added compound 33-4 (0.3 g) and tetrahydrofuran (15 mL), and borane dimethyl sulfide (0.5 g) was added dropwise under an ice bath under nitrogen atmosphere. After the addition was completed, the mixture was placed under an oil bath at 40 °C and stirred overnight. After the reaction was completed, a small amount of methanol was added to quench the reaction, and the mixture was concentrated to remove the solvent, reconstituted in 50 mL of ethyl acetate, washed with water, dried, and purified by column chromatography to obtain compound 33-5.

MS (ESI, [M+H]⁺) *m*/*z*: 226.08.

### Step F: Preparation of compound 33-6

To a 100 mL single-necked flask were added compound 33-5 (120 mg), chloroform (5 mL), and a catalytic amount of acetic acid. Liquid bromine (85 mg) was added dropwise under an ice bath, and the mixture was stirred at room temperature overnight. To the reaction solution was directly added 5 mL of diethyl ether. The mixture was filtered, and the filter cake was washed with diethyl ether to obtain a filter cake, namely compound 33-6.

MS (ESI, [M+H]⁺) *m*/*z:* 204.03.

### Step G: Preparation of compound 33-7

Referring to the method of step D in Example 18, compound 33-7 was prepared by reacting compound 33-6 with di-tert-butyl dicarbonate.

MS (ESI, [M+H]⁺) *m*/*z:* 304.03.

### Step H: Preparation of compound 33-8

Referring to the method of step F in Example 7, compound 33-8 was prepared by reacting compound 33-7 with carbon monoxide.

MS (ESI, [M+H]⁺) *m*/*z:* 284.41.

### Step I: Preparation of compound 33-9

Referring to the method of step G in Example 7, compound 33-9 was prepared by reacting compound 33-8 with a solution of methylamine in ethanol.

MS (ESI, [M+H]⁺) *m*/*z:* 283.22.

### Step J: Preparation of compound 33-10

Referring to the method of step C in Example 1, compound 33-10 was prepared by reacting compound 33-9 with trifluoroacetic acid.

MS (ESI, [M+H]⁺) *m*/*z:* 183.22.

### Step K: Preparation of compound 33-11

Referring to the method of step I in Example 2, compound 33-11 was prepared by reacting compound 33-10 with 1-Boc-3-azetidinone.

MS (ESI, [M+H]⁺) *m*/*z:* 338.23.

### Step L: Preparation of compound 33-12

Referring to the method of step C in Example 1, compound 33-12 was prepared by reacting compound 33-11 with trifluoroacetic acid.

MS (ESI, [M+H]⁺) *m*/*z: 238.23.*

### Step M: Preparation of compound 33

Referring to the method of step I in Example 1, compound 33 was prepared by reacting compound 24-4 with compound 33-12.

¹H NMR (500 MHz, DMSO-*d*₆) δ 12.24 (s, 1H), 8.51 (s, 1H), 8.33 (d, *J =* 4.6 Hz, 1H), 7.79 (d, *J =* 8.1 Hz, 1H), 7.47 (s, 1H), 7.32 (s, 1H), 7.18 (d, *J =* 8.1 Hz, 1H), 3.93 (s, 2H), 3.78 (d, *J =* 2.7 Hz, 2H), 3.71 (s, 2H),3.60 (s, 1H), 3.41 (t, *J =* 7.0 Hz, 2H), 3.08 (t, *J =* 6.7 Hz, 2H), 2.74 (d, *J =* 4.5 Hz, 3H).

HRMS (ESI, [M+H]⁺) *m*/*z:* 463.1409.

### Example 34: Preparation of Compound 34

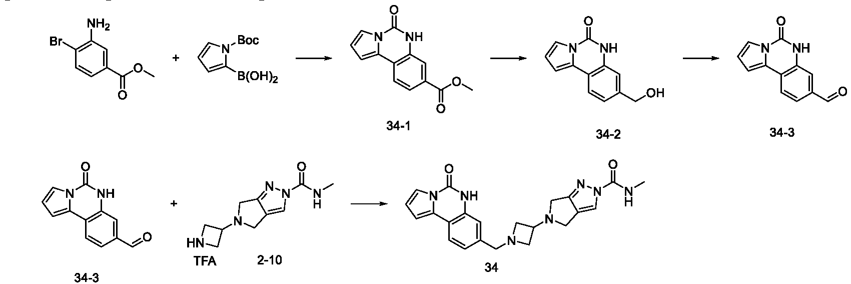

### Step A: Preparation of compound 34-1

To a 100 mL single-necked flask were added methyl 3-amino-4-bromobenzoate (1 g), acetonitrile (20 mL), cesium carbonate (2.83 g), and bis(triphenylphosphine)palladium(II) chloride (3.05) in sequence. The mixture was heated to 80 °C under nitrogen atmosphere, and (1-(tert-butoxycarbonyl)-1H-pyrrol-2-yl)boronic acid (1.1 g) was dissolved in 10 mL of acetonitrile and slowly added dropwise to the reaction system described above over about 0.5 h. After the addition was completed, the reaction system was stirred at 100 °C overnight. After the reaction was completed, the reaction solution was directly mixed with silica gel, and purified by column chromatography to obtain 0.6 g of compound 34-1.

MS (ESI, [M+H]⁺) *m*/*z:* 243.22.

### Step B: Preparation of compound 34-2

Referring to the method of step C in Example 15, compound 34-2 was prepared by reacting compound 34-1 with a 2.5 M solution of lithium aluminum hydride in tetrahydrofuran.

MS (ESI, [M+H]⁺) *m*/*z:* 215.24*.*

### Step C: Preparation of compound 34-3

Referring to the method of step H in Example 2, compound 34-3 was prepared by reacting compound 34-2 with 2-iodoxybenzoic acid.

MS (ESI, [M-H]⁻) *m*/*z*: 211.19.

### Step D: Preparation of compound 34

Referring to the method of step I in Example 1, compound 34 was prepared by reacting compound 34-3 with compound 2-10.

¹H NMR (500 MHz, DMSO-*d*₆) δ11.43 (s, 1H), 8.28 (d, *J =* 4.7 Hz, 1H), 7.94 (s, 1H), 7.84 (d, *J =* 8.0 Hz, 1H), 7.57 (dd, *J =* 3.0, 1.4 Hz, 1H), 7.20 (s, 1H), 7.11 (d, *J =* 8.1 Hz, 1H), 6.96 (dd, *J =* 3.5, 1.3 Hz, 1H), 6.66 (t, *J =* 3.3 Hz, 1H), 3.70 (s, 2H), 3.67 (s, 2H), 3.64 (s, 2H), 3.59 (dd, *J =* 12.7, 6.4 Hz, 1H), 3.40 (t, *J =* 6.9 Hz, 2H), 3.08 (t, *J =* 6.3 Hz, 2H), 2.78 (d, *J =* 4.7 Hz, 3H).

HRMS (ESI, [M+H]⁺) *m*/*z:* 418.1990.

### Example 35: Preparation of Compound 35

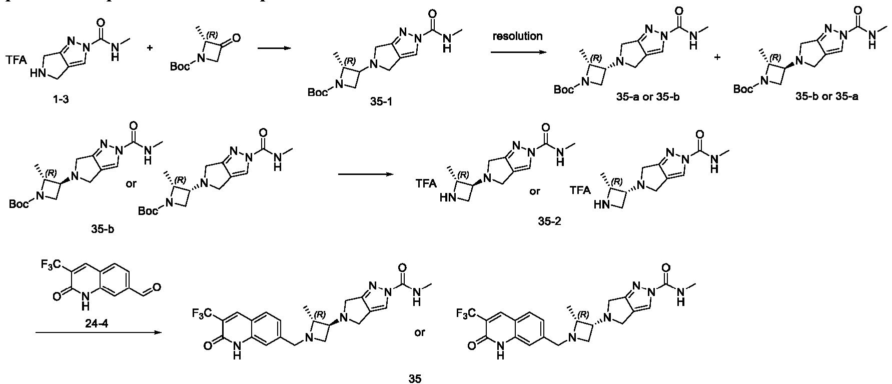

### Step A: Preparation of compounds 35-a and 35-b

Referring to the method of step I in Example 2, compound 35-1 was prepared by reacting compound 1-3 with *tert-butyl* (*R*)-2-methyl-3-oxoazetidine-1-carboxylate. Compound 35-1 was subjected to resolution and preparation by chromatographic column ASA Pre-packed Regis IA (30 × 250 mm, 10 µm) (mobile phase A: ethanol; mobile phase B: n-hexane; elution gradient: mobile phase A:mobile phase B = 35:65 (V/V); flow rate: 40 mL/min; detection wavelength: 254 nm) to obtain compounds 35-a and 35-b. The peak times were 7.58 min (compound 35-a) and 12.08 min (compound 35-b), respectively.

### Compound 35-a:

MS (ESI, [M+H]⁺) *m*/*z:* 336.40.

### Compound 35-b:

MS (ESI, [M+H]⁺) *m*/*z:* 336.01.

### Step B: Preparation of compound 35-2

Referring to the method of step C in Example 1, compound 35-2 was prepared by reacting compound 35-b with trifluoroacetic acid.

MS (ESI, [M+H]⁺) *m*/*z:* 236.33.

### Step C: Preparation of compound 35

Referring to the method of step I in Example 1, compound 35 was prepared by reacting compound 24-4 with compound 35-2.

¹H NMR (500 MHz, DMSO-*d*₆) δ 12.23 (s, 1H), 8.51 (s, 1H), 8.26 (d, *J =* 4.7 Hz, 1H), 7.95 (s, 1H), 7.79 (d, *J =* 8.1 Hz, 1H), 7.35 (s, 1H), 7.19 (dd, *J =* 8.1, 1.0 Hz, 1H), 3.81 (dd, *J =* 14.1, 11.5 Hz, 5H), 3.60-3.53 (m, 2H), 3.47 (s, 1H), 3.36 (dd, *J =* 7.9, 2.9 Hz, 1H), 3.03 (s, 1H), 2.78 (d, *J =* 4.7 Hz, 3H), 1.12 (d, *J =* 6.5 Hz, 3H). HRMS (ESI, [M+H]⁺) *m*/*z:* 461.1908.

### Example 36: Preparation of Compound 36

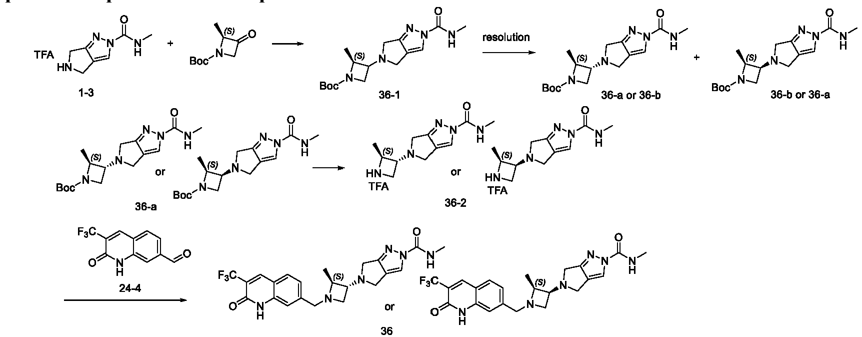

### Step A: Preparation of compounds 36-a and 36-b

Referring to the method of step I in Example 2, compound 36-1 was prepared by reacting compound 1-3 with *tert-butyl* (*S*)-2-methyl-3-oxoazetidine-1-carboxylate. Compound 36-1 was subjected to resolution and preparation by chromatographic column (R,R)-Whelk-O1 (20 × 250 mm, S-5 µm) (mobile phase A: ethanol-dichloromethane (1:1, V/V); mobile phase B: n-hexane; elution gradient: mobile phase A:mobile phase B = 30:70 (V/V); flow rate: 25 mL/min; detection wavelength: 254 nm) to obtain compounds 36-a and 36-b. The peak times were 10.3 min (compound 36-a) and 11.7 min (compound 36-b), respectively.

### Compound 36-a:

MS (ESI, [M+H]⁺) *m*/*z:* 336.01*.*

### Compound 36-b:

MS (ESI, [M+H]⁺) *m*/*z:* 336.01.

### Step B: Preparation of compound 36-2

Referring to the method of step C in Example 1, compound 36-2 was prepared by reacting compound 36-a with trifluoroacetic acid.

MS (ESI, [M+H]⁺) *m*/*z:* 236.34.

### Step C: Preparation of compound 36

Referring to the method of step I in Example 1, compound 36 was prepared by reacting compound 24-4 with compound 36-2.

¹H NMR (500 MHz, DMSO-*d*₆) δ 12.08 (s, 1H), 8.51 (s, 1H), 8.30 - 8.18 (m, *J =* 4.3 Hz, 1H), 7.95 (s, 1H), 7.79 (d, *J =* 8.1 Hz, 1H), 7.36 (s, 1H), 7.19 (d, *J =* 8.1 Hz, 1H), 3.84 (s, 1H), 3.81 (s, 2H), 3.79 (s, 2H), 3.60 - 3.52 (m, 2H), 3.51 - 3.42 (m, *J =* 12.8, 6.4 Hz, 1H), 3.36 (d, *J =* 2.9 Hz, 1H), 3.07 - 2.99 (m, *J =* 7.4 Hz, 1H), 2.79 (d, *J =* 4.6 Hz, 3H), 1.12 (d, *J =* 6.4 Hz, 3H).

HRMS (ESI, [M+H]⁺) *m*/*z:* 461.1915.

### Example 37: Preparation of Compound 37

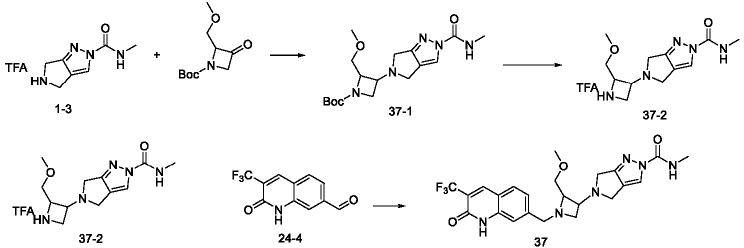

### Step A: Preparation of compound 37-1

Referring to the method of step I in Example 2, compound 37-1 was prepared by reacting compound 1-3 with *tert-butyl* 2-(methoxymethyl)-3-oxoazetidine-1-carboxylate.

MS (ESI, [M+H]⁺) *m*/*z:* 366.37.

### Step B: Preparation of compound 37-2

Referring to the method of step C in Example 1, compound 37-2 was prepared by reacting compound 37-1 with trifluoroacetic acid.

MS (ESI, [M+H]⁺) *m*/*z:* 266.35.

### Step C: Preparation of compound 37

Referring to the method of step I in Example 1, compound 37 was prepared by reacting compound 24-4 with compound 37-2.

¹H NMR (500 MHz, DMSO-*d*₆) δ 12.24 (s, 1H), 8.51 (s, 1H), 8.33 - 8.20 (m, *J =* 4.5 Hz, 1H), 7.95 (s, 1H), 7.79 (d, *J =* 8.0 Hz, 1H), 7.33 (s, 1H), 7.18 (d, *J =* 8.0 Hz, 1H), 3.93 (d, *J =* 14.4 Hz, 1H), 3.84 - 3.75 (m, *J =* 17.7, 8.0 Hz, 4H), 3.68 - 3.55 (m, 3H), 3.50 (d, *J =* 5.6 Hz, 1H), 3.40 - 3.35 (m, 1H), 3.21 (s, 3H), 3.13 - 3.05 (m, 1H), 3.05 - 2.97 (m, *J =* 7.4 Hz, 1H), 2.78 *(d, J=* 4.7 Hz, 3H).

HRMS (ESI, [M+H]⁺) *m*/*z:* 491.32017.

### Example 38: Preparation of Compound 38

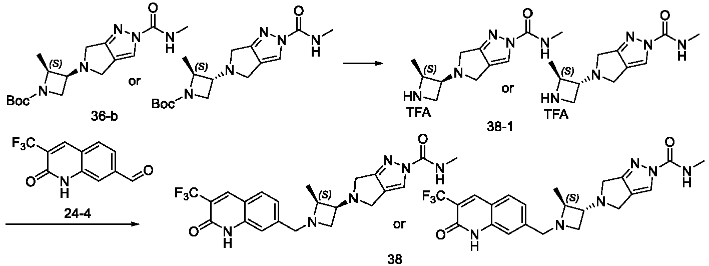

### Step A: Preparation of compound 38-1

Referring to the method of step C in Example 1, compound 38-1 was prepared by reacting compound 36-b with trifluoroacetic acid.

MS (ESI, [M+H]⁺) *m*/*z:* 236.34.

### Step B: Preparation of compound 38

Referring to the method of step I in Example 1, compound 38 was prepared by reacting compound 24-4 with compound 38-1.

¹H NMR (500 MHz, DMSO-*d*₆) δ 12.23 (s, 1H), 8.52 (s, 1H), 8.30 - 8.21 (m, *J =* 4.5 Hz, 1H), 7.94 (s, 1H), 7.79 (d, *J =* 8.1 Hz, 1H), 7.34 (s, 1H), 7.27 - 7.13 (m, *J =* 8.1, 1.0 Hz, 1H), 3.87 (d, *J =* 14.0 Hz, 1H), 3.69 - 3.62 (m, *J =* 5.7 Hz, 3H), 3.62 - 3.53 (m, 2H), 3.48 - 3.41 (m, *J =* 6.4 Hz, 1H), 3.22 - 3.14 (m, *J =* 6.0 Hz, 1H), 3.13 - 3.05 (m, *J =* 6.7 Hz, 1H), 2.78 (d, *J =* 4.7 Hz, 3H), 2.76 - 2.71 (m, *J =* 6.9 Hz, 1H), 1.13 (d, *J =* 6.0 Hz, 3H).

HRMS (ESI, [M+H]⁺) *m*/*z:* 461.1913.

### Example 39: Preparation of Compound 39

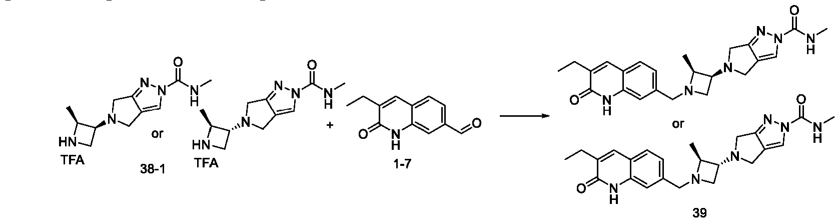

### Step A: Preparation of compound 39

Referring to the method of step I in Example 1, compound 39 was prepared by reacting compound 1-7 with compound 38-1.

¹H NMR (500 MHz, DMSO-*d₆*) δ 11.65 (s, 1H), 8.27 (q, *J =* 4.6 Hz, 1H), 7.95 (s, 1H), 7.69 (s, 1H), 7.55 (d, *J* = 8.0 Hz, 1H), 7.25 (s, 1H), 7.10 (d, *J =* 7.9 Hz, 1H), 3.83 (s, 1H), 3.74 - 3.40 (m, 6H), 3.11 (s, 2H), 2.78 (d, *J =* 4.7 Hz, 4H), 1.25 (dd, *J =* 12.0, 3.9 Hz, 2H), 1.16 (h, *J =* 6.4, 5.9 Hz, 6H).

HRMS (ESI, [M+H]⁺) *m*/*z: 421.2350*

### Example 40: Preparation of Compound 40

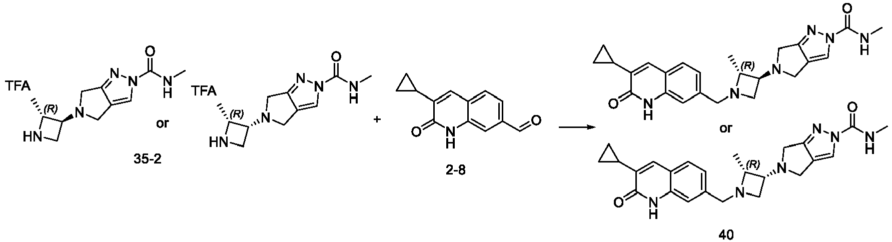

### Step A: Preparation of compound 40

Referring to the method of step I in Example 1, compound 40 was prepared by reacting compound 2-8 with compound 35-2.

¹H NMR (500 MHz, DMSO-*d*₆) δ 11.66 (s, 1H), 8.26 (q, *J =* 4.3 Hz, 1H), 7.93 (d, *J =* 10.7 Hz, 1H), 7.47 (d, *J =* 8.0 Hz, 1H), 7.39 (s, 1H), 7.24 (s, 1H), 7.04 (d, *J =* 8.0 Hz, 1H), 3.77 (s, 5H), 3.54 (td, *J =* 6.7, 3.0 Hz, 1H), 3.48 (d, *J =* 13.8 Hz, 1H), 3.45 - 3.41 (m, 1H), 3.31 (dd, *J =* 7.7, 2.8 Hz, 1H), 3.00 (t, *J =* 7.4 Hz, 1H), 2.79 (d, *J* = 4.7 Hz, 3H), 2.08 (dq, *J* = 8.5, 5.4 Hz, 1H), 1.09 (d, *J =* 6.5 Hz, 3H), 0.95 - 0.85 (m, 2H), 0.77 - 0.67 (m, 2H).

HRMS (ESI, [M+H]⁺) *m*/*z:* 433.2356.

### Example 41: Preparation of Compound 41

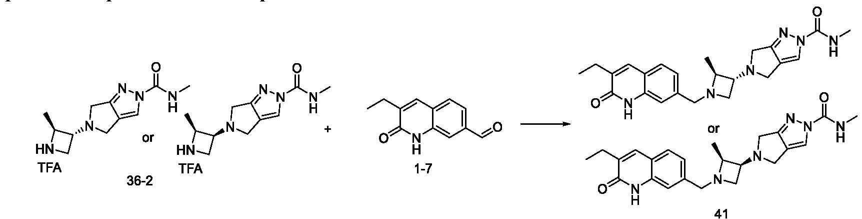

### Step A: Preparation of compound 41

Referring to the method of step I in Example 1, compound 41 was prepared by reacting compound 1-7 with compound 36-2.

¹H NMR (500 MHz, DMSO-*d₆*) δ 11.65 (s, 1H), 8.27 (q, *J =* 4.6 Hz, 1H), 7.94 (s, 1H), 7.68 (s, 1H), 7.53 (d, *J =* 8.0 Hz, 1H), 7.26 (s, 1H), 7.07 (d, *J =* 8.0 Hz, 1H), 3.88 - 3.68 (m, 5H), 3.63 - 3.35 (m, 5H), 3.02 (s, 1H), 2.79 (d, *J =* 4.7 Hz, 3H), 1.25 (dd, *J =* 12.5, 3.9 Hz, 1H), 1.21 - 1.04 (m, 6H).

HRMS (ESI, [M+H]⁺) *m*/*z:* 421.2342.

### Example 42: Preparation of Compound 42

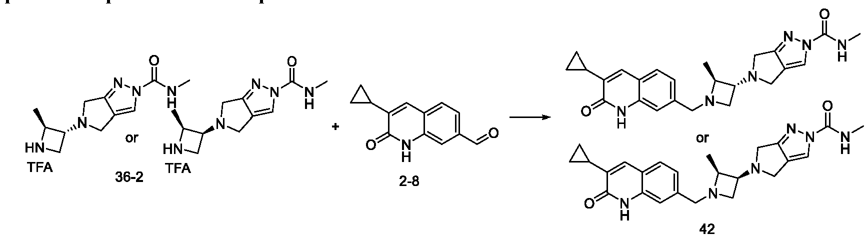

### Step A: Preparation of compound 42

Referring to the method of step I in Example 1, compound 42 was prepared by reacting compound 2-8 with compound 36-2.

¹H NMR (500 MHz, DMSO-*d*₆) δ 11.66 (s, 1H), 8.30 - 8.21 (m, *J =* 4.4 Hz, 1H), 7.94 (s, 1H), 7.47 (d, *J =* 8.0 Hz, 1H), 7.38 (s, 1H), 7.23 (s, 1H), 7.08 - 7.00 (m, *J =* 8.0, 1.1 Hz, 1H), 3.85 - 3.71 (m, *J =* 19.0, 13.9 Hz, 5H), 3.58 - 3.52 (m, *J =* 6.8, 3.0 Hz, 1H), 3.48 (d, *J =* 13.8 Hz, 1H), 3.45 - 3.38 (m, *J =* 13.0, 6.5 Hz, 1H), 3.31 - 3.28 (m, 1H), 3.03 - 2.97 (m, *J* = 7.4 Hz, 1H), 2.78 (d, *J =* 4.7 Hz, 3H), 2.12 - 2.03 (m, 1H), 1.10 *(d, J=* 6.5 Hz, 3H), 0.94 - 0.87 (m, 2H), 0.75 - 0.66 (m, 2H).

HRMS (ESI, [M+H]⁺) *m*/*z:* 433.2361.

### Example 43: Preparation of Compound 43

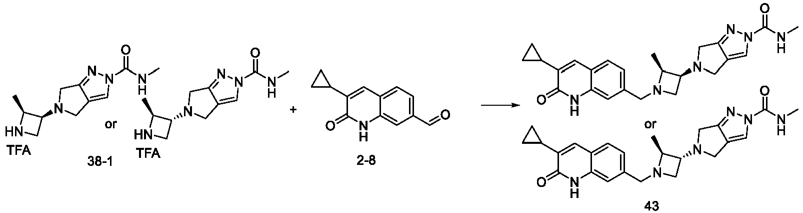

### Step A: Preparation of compound 43

Referring to the method of step I in Example 1, compound 43 was prepared by reacting compound 2-8 with compound 38-1.

¹H NMR (500 MHz, DMSO-*d*₆) δ 11.64 (s, 1H), 8.31 - 8.22 (m, *J =* 4.4 Hz, 1H), 7.93 (s, 1H), 7.47 (d, *J =* 8.0 Hz, 1H), 7.39 (s, 1H), 7.22 (s, 1H), 7.10 - 6.99 (m, *J =* 8.0, 0.9 Hz, 1H), 3.79 (d, *J =* 13.4 Hz, 1H), 3.67 - 3.55 (m, 4H), 3.49 (d, *J =* 13.4 Hz, 1H), 3.43 - 3.36 (m, *J =* 6.3 Hz, 1H), 3.17 - 3.10 (m, *J =* 6.0 Hz, 1H), 3.10 - 3.03 (m, *J =* 13.4, 6.7 Hz, 1H), 2.77 (d, *J =* 4.7 Hz, 3H), 2.74 - 2.68 (m, *J =* 6.9 Hz, 1H), 2.12 - 2.02 (m, 1H), 1.10 (d, *J =* 6.0 Hz, 3H), 0.94 - 0.86 (m, 2H), 0.76 - 0.68 (m, 2H).

HRMS (ESI, [M+H]⁺) *m*/*z:* 433.2355.

### Example 44: Preparation of Compound 44

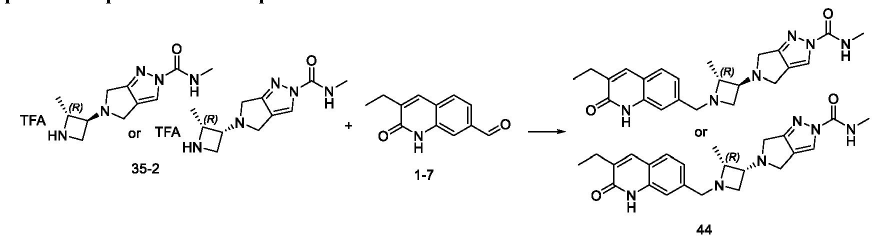

### Step A: Preparation of compound 44

Referring to the method of step I in Example 1, compound 44 was prepared by reacting compound 1-7 with compound 35-2.

¹H NMR (500 MHz, DMSO-*d*₆) δ 11.67 (s, 1H), 8.26 (q, *J =* 4.7 Hz, 1H), 7.94 (s, 1H), 7.68 (s, 1H), 7.54 (d, *J =* 8.0 Hz, 1H), 7.27 (s, 1H), 7.09 (s, 1H), 3.96 - 3.71 (m, 5H), 3.57 (s, 3H), 3.03 (s, 2H), 2.78 (d, *J =* 4.7 Hz, 3H), 2.47 *(d, J=* 7.4 Hz, 2H), 1.15 (*q, J =* 8.6, 8.0 Hz, 6H).

HRMS (ESI, [M+H]⁺) *m*/*z:* 421.2354.

### Example 45: Preparation of Compound 45

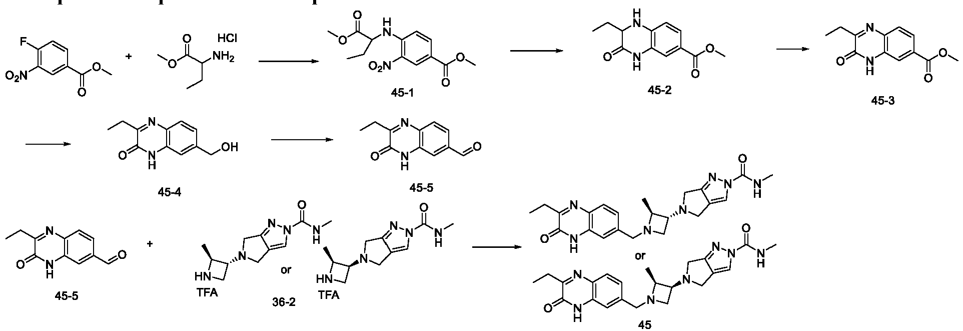

### Step A: Preparation of compound 45-1

Referring to the method of step B in Example 3, compound 45-1 was prepared by reacting methyl 4-fluoro-3-nitrobenzoate with methyl DL-2-amino-n-butyrate hydrochloride.

MS (ESI, [M-H]⁻) *m*/*z:* 295.20.

### Step B: Preparation of compound 45-2

Referring to the method of step C in Example 3, compound 45-2 was prepared by reacting compound 45-1 with iron powder.

MS (ESI, [M-H]⁻) *m*/*z: 233.18.*

### Step C: Preparation of compound 45-3

Referring to the method of step D in Example 3, compound 45-3 was prepared by reacting compound 45-2 with 2,3-dichloro-5,6-dicyanobenzoquinone.

MS (ESI, [M-H]⁻) *m*/*z:* 231.18.

### Step D: Preparation of compound 45-4

Referring to the method of step C in Example 15, compound 45-4 was prepared by reacting compound 45-3 with a 2.5 M solution of lithium aluminum hydride in tetrahydrofuran.

MS (ESI, [M-H]⁻) *m*/*z:* 203.15.

### Step E: Preparation of compound 45-5

Referring to the method of step H in Example 2, compound 45-5 was prepared by reacting compound 45-4 with 2-iodoxybenzoic acid.

MS (ESI, [M-H]⁻) *m*/*z*: 201.17.

### Step F: Preparation of compound 45

Referring to the method of step I in Example 1, compound 45 was prepared by reacting compound 45-5 with compound 36-2.

¹H NMR (500 MHz, DMSO-*d*₆) δ 12.23 (s, 1H), 8.27 (q, *J =* 4.6 Hz, 1H), 7.95 (s, 1H), 7.65 (d, *J =* 8.2 Hz, 1H), 7.26 (d, *J =* 1.7 Hz, 1H), 7.19 (dd, *J =* 8.2, 1.8 Hz, 1H), 3.88 - 3.70 (m, 5H), 3.62 - 3.52 (m, 2H), 3.46 (p, *J* = 6.5 Hz, 1H), 3.03 (t, *J =* 7.4 Hz, 1H), 2.87 - 2.72 (m, 6H), 1.22 (t, *J =* 7.4 Hz, 3H), 1.12 (d, *J =* 6.4 Hz, 3H). HRMS (ESI, [M+H]⁺) *m*/*z:* 422.2303.

### Example 46: Preparation of Compound 46

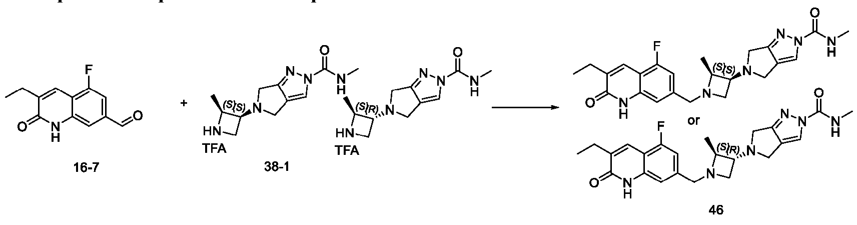

### Step A: Preparation of compound 46

Referring to the method of step I in Example 1, compound 46 was prepared by reacting compound 16-7 with compound 38-1.

¹H NMR (500 MHz, DMSO-*d*₆) δ 11.86 (s, 1H), 8.26 (d, *J =* 4.7 Hz, 1H), 7.93 (s, 1H), 7.73 (s, 1H), 7.09 (s, 1H), 6.92 (d, *J=* 10.8 Hz, 1H), 3.80 (d, *J =* 13.8 Hz, 1H), 3.61 (dd, *J =* 24.5, 9.7 Hz, 4H), 3.50 (d, *J =* 13.8 Hz, 1H), 3.43 (t, *J =* 6.4 Hz, 1H), 3.20 - 3.12 (m, 1H), 3.08 (dd, *J =* 13.4, 6.7 Hz, 1H), 2.78 (d, *J =* 4.6 Hz, 3H), 2.74 (t, *J* = 6.9 Hz, 1H), 2.53 (d, *J =* 7.6 Hz, 2H), 1.20 - 1.08 (m, 6H).

HRMS (ESI, [M+H]⁺) *m*/*z:* 439.2263.

### Example 47: Preparation of Compound 47

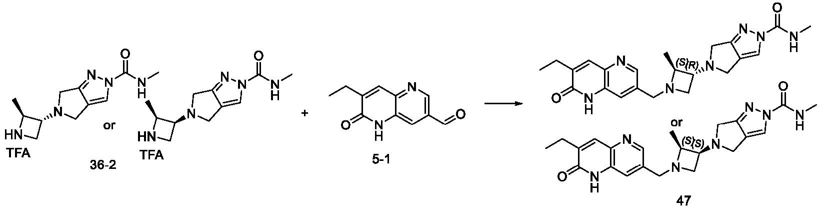

### Step A: Preparation of compound 47

Referring to the method of step I in Example 1, compound 47 was prepared by reacting compound 5-1 with compound 36-2.

¹H NMR (500 MHz, DMSO-*d*₆) δ 11.81 (s, 1H), 8.35 (s, 1H), 8.31 - 8.15 (m, 1H), 7.94 (s, 1H), 7.73 (s, 1H), 7.59 (s, 1H), 3.89 - 3.70 (m, 5H), 3.61 - 3.51 (m, 2H), 3.50 - 3.43 (m, 1H), 3.04 (t, *J =* 6.7 Hz, 1H), 2.88 - 2.69 (m, 3H), 2.60 - 2.51 (m, 3H), 1.18 (t, *J =* 7.1 Hz, 3H), 1.11 (d, *J =* 5.7 Hz, 3H).

HRMS (ESI, [M+H]⁺) *m*/*z:* 422.2301.

### Example 48: Preparation of Compound 48

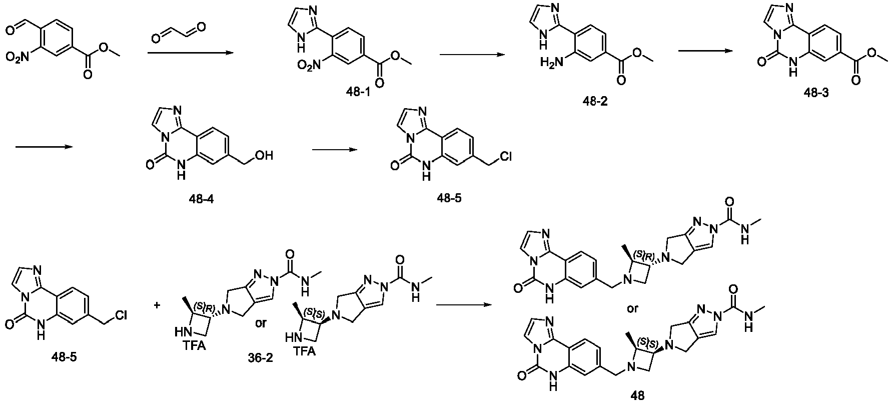

### Step A: Preparation of compound 48-1

To a 250 mL three-necked flask were added methyl 4-formyl-3-nitrobenzoate (4.5 g) and methanol (100 mL) in sequence. After the addition was completed, the system was purged with nitrogen. A solution (7 M; 45 mL) of ammonia in methanol and glyoxal (22 mL) were added to the system via syringe. After the addition was completed, the mixture was reacted at room temperature overnight. After the reaction was completed, the mixture was concentrated under reduced pressure to evaporate the excess solvent, and purified by silica gel column chromatography to obtain compound 48-1.

MS (ESI, [M+H]⁺) *m*/*z:* 248.24.

### Step B: Preparation of compound 48-2

To a 100 mL single-necked flask were added compound 48-1 (900 mg), ethyl acetate (40 mL), and stannous chloride dihydrate (4.1 g). After the addition was completed, the system was purged with nitrogen, and the mixture was placed under an oil bath under nitrogen atmosphere, heated to 80 °C, and reacted for 4 h. After the reaction was completed, the reaction solution was diluted with 100 mL of purified water, followed by adding a saturated aqueous sodium bicarbonate solution to adjust the pH to 8. The aqueous phase was extracted with ethyl acetate, and the organic phases were combined and dried over anhydrous sodium sulfate. The crude product obtained by concentrating the organic phase was further purified by silica gel column chromatography to obtain compound 48-2.

MS (ESI, [M+H]⁺) *m*/*z*: 218.21.

### Step C: Preparation of compound 48-3

To a 50 mL two-necked flask were added compound 48-2 (565 mg) and 1,4-dioxane (20 mL) in sequence. After the addition was completed, the system was purged with nitrogen. Triphosgene (810 mg) was then added to the reaction system under nitrogen atmosphere, and after the addition was completed, the mixture was heated to 80 °C under an oil bath and reacted for about 12 h. After the reaction was completed, the reaction solution was quenched with 5 mL of methanol, concentrated, and purified by column chromatography to obtain compound 48-3.

MS (ESI, [M+H]⁺) *m*/*z:* 244.26.

### Step D: Preparation of compound 48-4

Referring to the method of step C in Example 15, compound 48-4 was prepared by reacting compound 48-3 with a 2.5 M solution of lithium aluminum hydride in tetrahydrofuran.

MS (ESI, [M+H]⁺) *m*/*z*: 216.19.

### Step E: Preparation of compound 48-5

To a 50 mL single-necked flask were added compound 48-4 (250 mg), dichloromethane (20 mL), and *N,N*-dimethylformamide (0.05 mL) in sequence. After the addition was completed, the reaction system was placed under an ice bath, cooled to 0 °C, and stirred. Thionyl chloride (0.22 mL) was then slowly added dropwise to the reaction system. After the addition was completed, the reaction system was transferred to room temperature and reacted overnight. After the reaction was completed, the mixture was concentrated under reduced pressure to evaporate the solvent and thionyl chloride to obtain a crude product of compound 48-5, which was directly used in the next step.

MS (ESI, [M+H]⁺) *m*/*z:* 234.17.

### Step F: Preparation of compound 48

To a 50 mL single-necked flask were added compound 36-2 (104 mg), acetonitrile (10 mL), triethylamine (0.25 mL), anhydrous potassium carbonate (103 mg), potassium iodide (10 mg), and compound 48-5 (70 mg) in sequence. After the addition was completed, the system was purged with nitrogen, placed under an oil bath, heated to 80 °C, and reacted for 2 h. After the reaction was completed, the mixture was concentrated and purified by column chromatography to obtain compound 48.

¹H NMR (500 MHz, DMSO-*d*₆) δ 11.92 (s, 1H), 8.38 - 8.20 (m, 1H), 8.05 (d, *J =* 7.9 Hz, 1H), 7.95 (s, 1H), 7.86 (s, 1H), 7.43 (s, 1H), 7.36 (s, 1H), 7.24 (d, *J =* 7.8 Hz, 1H), 3.87 - 3.75 (m, 5H), 3.60 - 3.52 (m, 2H), 3.51 - 3.44 (m, 1H), 3.35 - 3.34 (m, 1H), 3.08 - 3.00 (m, 1H), 2.79 (d, *J =* 4.2 Hz, 3H), 1.13 (d, *J =* 6.1 Hz, 3H).

HRMS (ESI, [M+H]⁺) *m*/*z:* 433.2102.

### Example 49: Preparation of Compound 49

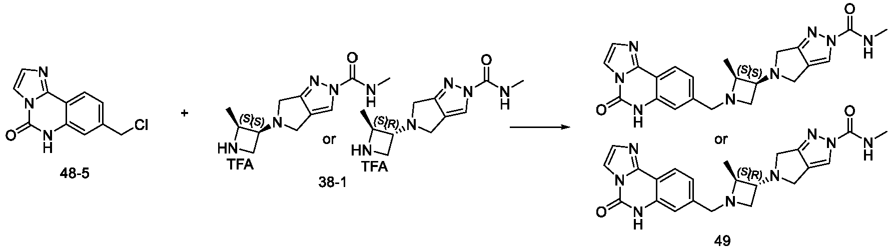

### Step A: Preparation of compound 49

Referring to the method of step F in Example 48, compound 49 was prepared by reacting compound 48-5 with compound 38-1.

¹H NMR (500 MHz, DMSO-*d*₆) δ 11.91 (s, 1H), 8.27 (q, *J =* 4.6 Hz, 1H), 8.06 (d, *J =* 8.0 Hz, 1H), 7.94 (s, 1H), 7.86 (d, *J =* 1.6 Hz, 1H), 7.43 (d, *J =* 1.6 Hz, 1H), 7.34 (s, 1H), 7.26 (dd, *J* = 8.1, 1.5 Hz, 1H), 3.85 (d, *J =* 13.6 Hz, 1H), 3.69 - 3.62 (m, 3H), 3.62 - 3.52 (m, 2H), 3.44 (t, *J =* 6.4 Hz, 1H), 3.22 - 3.14 (m, 1H), 3.13 - 3.05 (m, 1H), 2.84 - 2.70 (m, 4H), 1.14 (d, *J =* 6.0 Hz, 3H).

HRMS (ESI, [M+H]⁺) *m*/*z:* 433.2109.

### Test Example 1. Inhibitory Activity for In Vitro Cell Proliferation

### 1.1 Assay on inhibitory activity for MDA-MB-436 cell proliferation

MDA-MB-436 cells in a good growth state were collected into a centrifuge tube, adjusted to a cell density of 2 × 10⁴ cells/mL using a complete medium (DMEM high glucose + 10% FBS + 1× insulin-transferrin-selenium (ITS-G) + 16 µg/mL reduced glutathione), and seeded in a 96-well plate (100 µL/well), and the plate was incubated overnight in a cell incubator. Compounds were added using a pipettor, such that the final concentrations of the compounds were 400 nM-0.02 nM. The wells were set in duplicate, and a control well was set. After 168 h of incubation in the cell incubator, the assay reagent CCK-8 (manufacturer: Dojindo Laboratories, Beijing; 10 µL/well) was added. After 1.5 h of incubation in the cell incubator, the absorbance was measured at 450 nm on a PerkinElmer Envision microplate reader. A four-parameter analysis was performed, a dose-response curve was fitted, and IC₅₀ was calculated, wherein A represents IC₅₀ ≤ 50 nM, and the results are shown in Table 1:

**Table 1. Inhibitory activity results for DA-MB-436 cell proliferation of compounds**

| Compound No. | MDA-MB-436 cell IC₅₀ (nM) | Compound No. | MDA-MB-436 cell IC₅₀ (nM) |
|---|---|---|---|
| 1 | A | 27 | A |
| 2 | A | 28 | A |
| 4 | A | 29 | A |
| 5 | A | 30 | A |
| 7 | A | 31 | A |
| 9 | A | 33 | A |
| 10 | A | 34 | A |
| 11 | A | 35 | A |
| 13 | A | 36 | A |
| 14 | A | 38 | A |
| 15 | A | 39 | A |
| 16 | A | 40 | A |
| 17 | A | 41 | A |
| 18 | A | 42 | A |
| 19 | A | 43 | A |
| 20 | A | 44 | A |
| 21 | A | 45 | A |
| 22 | A | 46 | A |
| 23 | A | 47 | A |
| 24 | A | 48 | A |
| 25 | A | 49 | A |
| 26 | A | | |

### Test Example 2. Inhibitory Activity for PARP Protein

### 2.1 Assay on PARP1 protein activity

A chemiluminescence assay kit (BPS, Cat. No. 80551) was used. Fifty microliters of 1× histone were added to a 96-well plate and incubated overnight at 4 °C. Each well was washed three times with 200 µL of PBST buffer (containing 0.05% Tween-20), and the liquid was removed from the plate. Two hundred microliters of blocking buffer 3 were added, and the plate was blocked at room temperature for 60-90 min. The blocking solution was discarded, and the plate was washed three times with PBST buffer, followed by the removal of the liquid from the plate. Twenty-five microliters of master mix (2.5 µL of 10× PARP buffer + 2.5 µL of 10× biotinylated substrate assay mix + 5 µL of activated DNA (5×) + 15 µL of water) and 5 µL of 1× PARP buffer were added to each well. Compounds were sprayed using a nanoliter pipettor in the compound group. Twenty microliters of 1× PARP buffer were added to the blank control group, and 20 µL of PARP1 enzyme (2.4 ng/mL) was added to the other wells to initiate the reaction. The plate was incubated at room temperature for 1 h. Fifty microliters of streptavidin-HRP (diluted 1:50 in blocking buffer 3) were added to each well, and the plate was incubated at room temperature for 30 min. The plate was washed three times with PBST, and the liquid was removed from the plate. Fifty microliters of ELISA ECL substrate A and 50 µL of ELISA ECL substrate B were mixed on ice before use, and 100 µL were added to each well. The absorbance was measured on a PerkinElmer Envision microplate reader in LUMINESCENCE mode. A four-parameter analysis was performed, a dose-response curve was fitted, and IC₅₀ was calculated, wherein +++ represents IC₅₀ ≤ 1 nM, ++ represents IC₅₀ ≤ 10 nM, and the results are shown in Table 2.

**Table 2. Inhibitory activity results for PARP1 kinase of compounds**

| Compound No. | PARP1 IC₅₀ (nM) |
|---|---|
| 2 | +++ |
| 24 | +++ |
| 36 | ++ |
| 45 | +++ |
| 47 | +++ |

The compounds of the present application were found to exhibit high PARP1 protein kinase inhibitory activity.

### Test Example 3. Trap Activity for PARP Protein

### 3.1 Assay on PARP1 protein Trap activity

The PARPtrap^{™} assay kit (BPS, Cat. No. 80584-2) was used, and the experiment was divided into a blank group, control group, low FP control group, high FP control group, and compound group. First, a mixture consisting of 5× PARPtrap^{™} assay buffer, 25 nM fluorescent-labeled DNA, and water was prepared. According to the group settings, the mixture, or 5× PARPtrap^{™} assay buffer, 1× PARPtrap^{™} assay buffer, PARP1 (0.5 ng/µL) prepared with 1× PARPtrap^{™} assay buffer, and the compounds were added to a 384-well plate. After incubation at room temperature for 60 min, 10× NAD+ or water was added to each well according to the group settings, followed by incubation at room temperature for 60 min.

The polarization value (FP) was measured using a PerkinElmer Envision microplate reader FP-480/530, and mP was calculated using the formula: mP = 1000 * (S - G * P) / (S + G * P), where S represents the vertical light fluorescence intensity, P represents the parallel light fluorescence intensity, and G represents the correction factor. A four-parameter analysis was performed, a dose-response curve was fit, and EC₅₀ was calculated.

### 3.2 Assay on PARP2 protein Trap activity

The PARPtrap^{™} assay kit (BPS, Cat. No. 78296-2) was used, and the experiment was divided into a blank group, control group, low FP control group, high FP control group, and compound group. First, DTT was added to 5× PARPtrap^{™} assay buffer to prepare 5× PARPtrap^{™} assay buffer 2 containing a final concentration of 10 mM DTT, and a mixture consisting of 5× PARPtrap^{™} assay buffer 2, 12.5 nM fluorescent-labeled DNA, and water was prepared. According to the group settings, the mixture, or 5× PARPtrap^{™} assay buffer 2, 1× PARPtrap^{™} assay buffer 2, PARP2 (3.75 ng/µL) prepared with 1× PARPtrap^{™} assay buffer 2, and the compounds were added to a 384-well plate. After incubation at room temperature for 60 min, 10× NAD+ or water was added to each well, followed by incubation at room temperature for 60 min.

The polarization value (FP) was measured using a PerkinElmer Envision microplate reader FP-480/530, and mP was calculated using the formula: mP = 1000 * (S - G * P) / (S + G * P), where S represents the vertical light fluorescence intensity, P represents the parallel light fluorescence intensity, and G represents the correction factor. A four-parameter analysis was performed, a dose-response curve was fit, and EC₅₀ was calculated.

### Test Example 4. In Vitro Pharmacokinetics

### 4.1 In vitro liver microsomal stability assay

Liver microsome incubation samples were prepared by mixing a PBS buffer (pH 7.4), a liver microsome solution (0.5 mg/mL), a test compound, and an NADPH + MgCl₂ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution containing an internal standard was added to the samples, and the supernatants were prepared by protein precipitation, diluted, and then assayed by LC/MS/MS.

The compounds of the present application exhibit good stability in liver microsomes.

### Test Example 5. In Vivo Pharmacokinetics

### 5.1 Mouse pharmacokinetics

ICR mice weighing 20-25 g were randomized into groups after 3-5 days of acclimatization, with 9 mice in each group, and then intragastrically given a test compound solution at a dose of 1 mg/kg.

Plasma samples to be tested were prepared by taking blood from the orbit at time points of 0 min, 5 min, 0.25 min, 0.5 h, 2 h, 6 h, 10 h, and 24 h.

Twenty microliters of each plasma sample to be tested and a standard curve sample were taken, and an acetonitrile solution containing an internal standard was added. Supernatants were obtained by protein precipitation, diluted, and then assayed by LC/MS/MS.

### 5.2 Rat pharmacokinetics

SD rats weighing 180-220 g were randomized into groups after 3-5 days of acclimatization, with 3 mice in each group, and then intragastrically given a test compound solution at a dose of 0.5 mg/kg.

Plasma samples to be tested were prepared by taking blood from the orbit at time points of 0 min, 15 min, 30 min, 1 h, 2 h, 4 h, 6 h, 8 h, 10 h, and 24 h.

50 µL of each plasma sample to be tested and a standard curve sample were taken, and an acetonitrile solution containing an internal standard was added. Supernatants were obtained by protein precipitation, diluted, and then assayed by LC/MS/MS.

The compounds of the present application were found to exhibit favorable pharmacokinetic properties *in vivo,* such as a long half-life (T_{1/2}), high *in vivo* exposure (AUC), and high bioavailability (F%).

### Test Example 6. PARP1/2-Tracer Activity Assay

The enzyme inhibition activity of the compounds against PARP1/2-Tracer was tested using the FP method. First, 100 nL of inhibitors with a final concentration of 1% DMSO were transferred to a 384-well reaction plate (Corning 4514) using Echo655. Then, 5 µL of PARP1/2 (BPS, Cat #80501/80502) enzyme solution was added to each well, followed by centrifugation at 1000 RPM for 1 min at room temperature, and the reaction was allowed to proceed for 10 min. Subsequently, 5 µL of Tracer (ICE, Cat #001315-009) solution was added to each well, followed by centrifugation at 1000 RPM for 1 min at room temperature, and the reaction was allowed to proceed for 60 min. PARP1/2 and Tracer were prepared in a buffer containing 50 mM Tris (pH 8.0), 10 mM MgCl₂, 150 mM NaCl, and 0.001% Triox-100, with final concentrations of 5/10 nM and 2.5 nM, respectively. Finally, the FP signal was read using BMG (PHERAstar FSX), and the IC₅₀ values and nonlinear regression curve fitting were obtained using GraphPad Prism software. The results are shown in Table 3.

**Table 3. Selective activity of compounds against PARP1/2 proteins**

| Compound No. | PARP1 IC₅₀ (nM) | PARP2 IC₅₀ (nM) | PARP2/PARP1 selectivity (fold) |
|---|---|---|---|
| 24 | 2.48 | >500 | >200 |
| 45 | 2.52 | >500 | >200 |

The compounds of the present application were found to exhibit high selectivity for the PARP1 protein.

## Claims

1. A compound of formula (II), a stereoisomer thereof, or a pharmaceutically acceptable salt thereof, 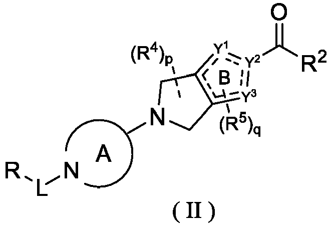 wherein,
R is selected from the group consisting of 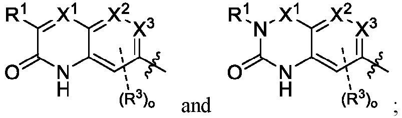
X¹ is selected from the group consisting of CR^{a}, CHR^{a}, N, and NR^{a};
X² is selected from the group consisting of CH and N;
X³ is selected from the group consisting of CH and N;
R¹ is selected from the group consisting of halogen, C₁₋₆ alkyl, C₁₋₆ alkoxy, C₃₋₈ cycloalkyl, and 3-8 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R^{a} is selected from the group consisting of H, halogen, and C₁₋₆ alkyl;
or R^{a} and R¹ are linked to each other to form 5-7 membered heterocycloalkyl, 5-7 membered cycloalkenyl, phenyl, 5-7 membered heterocycloalkenyl, or 5-6 membered heteroaryl;
R² is selected from the group consisting of C₁₋₆ alkyl, -OH, -OC₁₋₆ alkyl, -OC₃₋₆ cycloalkyl, -SH, -SC₁₋₆ alkyl, -SC₃₋₆ cycloalkyl, -NH₂, -NH(C₁₋₆ alkyl), -NH(C₃₋₆ cycloalkyl), -NH(3-8 membered heterocycloalkyl), -N(C₁₋₆ alkyl)₂, -N(C₁₋₆ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), -N(C₁₋₆ alkyl)₂, C₃₋₆ cycloalkyl, and 3-8 membered heterocycloalkyl;
R³, R⁴, and R⁵ are each independently selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂, wherein the C₁₋₆ alkyl, -OC₁₋₆ alkyl, -SC₁₋₆ alkyl, -NH(C₁₋₆ alkyl), or -N(C₁₋₆ alkyl)₂ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
o, p, and q are each independently selected from the group consisting of 0, 1, and 2;
L is selected from the group consisting of -NH- and -CH₂-, wherein L is optionally substituted with one or more groups selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂;
ring A is selected from the group consisting of 3-8 membered heterocycloalkyl, wherein ring A optionally contains 1-3 heteroatoms independently selected from the group consisting of N, O, and S in addition to the N atom linked to L, and ring A is optionally substituted with one or more groups selected from the group consisting of D, halogen, -OH, -C₁₋₆ alkyl, -OC₁₋₆ alkyl, -SH, -SC₁₋₆ alkyl, -NH₂, -NH(C₁₋₆ alkyl), -N(C₁₋₆ alkyl)₂, -C₁₋₄ alkylene-OC₁₋₆ alkyl, -C₁₋₄ alkylene-SC₁₋₆ alkyl, -C₁₋₄ alkylene-NH(C₁₋₆ alkyl), and -C₁₋₄ alkylene-N(C₁₋₆ alkyl)₂;
ring B is selected from the group consisting of an aromatic ring or a partially saturated ring;
Y¹, Y², and Y³ are each independently selected from the group consisting of C, CH, N, O, and S.

2. The compound, the stereoisomer thereof, or the pharmaceutically acceptable salt thereof according to claim 1, wherein the compound of formula (II) is selected from the group consisting of a compound of formula (I), a stereoisomer thereof, and a pharmaceutically acceptable salt thereof, 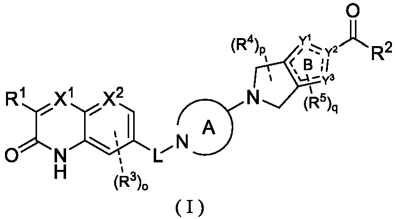 wherein,
X¹ is selected from the group consisting of CR^{a} and N;
X² is selected from the group consisting of CH and N;
R¹ is selected from the group consisting of halogen, C₁₋₆ alkyl, C₁₋₆ alkoxy, C₃₋₈ cycloalkyl, and 3-8 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R^{a} is selected from the group consisting of H, halogen, and C₁₋₆ alkyl;
or R^{a} and R¹ are linked to each other to form 5-7 membered cycloalkenyl, phenyl, 5-7 membered heterocycloalkenyl, or 5-6 membered heteroaryl;
R² is selected from the group consisting of C₁₋₆ alkyl, -OH, -OC₁₋₆ alkyl, -OC₃₋₆ cycloalkyl, -SH, -SC₁₋₆ alkyl, -SC₃₋₆ cycloalkyl, -NH₂, -NH(C₁₋₆ alkyl), -NH(C₃₋₆ cycloalkyl), -NH(3-8 membered heterocycloalkyl), -N(C₁₋₆ alkyl)₂, -N(C₁₋₆ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
R³, R⁴, and R⁵ are each independently selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂, wherein the C₁₋₆ alkyl, -OC₁₋₆ alkyl, -SC₁₋₆ alkyl, -NH(C₁₋₆ alkyl), or -N(C₁₋₆ alkyl)₂ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
o, p, and q are each independently selected from the group consisting of 0, 1, and 2;
L is selected from the group consisting of -NH- and -CH₂-, wherein L is optionally substituted with one or more groups selected from the group consisting of C₁₋₆ alkyl, D, halogen, -OH, -OC₁₋₆ alkyl, -SH, -SC₁₋₆ alkyl, -NH₂, -NH(C₁₋₆ alkyl), and -N(C₁₋₆ alkyl)₂;
ring A is selected from the group consisting of 3-8 membered heterocycloalkyl, wherein ring A optionally contains 1-3 heteroatoms independently selected from the group consisting of N, O, and S in addition to the N atom linked to L, and ring A is optionally substituted with one or more groups selected from the group consisting of D, halogen, -OH, -C₁₋₆ alkyl, -OC₁₋₆ alkyl, -SH, -SC₁₋₆ alkyl, -NH₂, -NH(C₁₋₆ alkyl), and -N(C₁₋₆ alkyl)₂;
ring B is selected from the group consisting of an aromatic ring or a partially saturated ring;
Y¹, Y², and Y³ are each independently selected from the group consisting of C, CH, N, O, and S.

3. The compound, the stereoisomer thereof, or the pharmaceutically acceptable salt thereof according to claim 1 or 2, wherein X¹ is selected from the group consisting of CR^{a} and N; or X¹ is selected from the group consisting of CHR^{a} and NR^{a};
optionally, X¹ is selected from the group consisting of CR^{a}, and X² is selected from the group consisting of CH; or X¹ is selected from the group consisting of CH, and X² is selected from the group consisting of N; or X¹ is selected from the group consisting of N, and X² is selected from the group consisting of CH; or X¹ is selected from the group consisting of NR^{a}, and X² is selected from the group consisting of CH;
optionally, X¹ is selected from the group consisting of CR^{a}, X² is selected from the group consisting of CH, and X³ is selected from the group consisting of CH; or X¹ is selected from the group consisting of CH, X² is selected from the group consisting of N, and X³ is selected from the group consisting of CH; or X¹ is selected from the group consisting of N, X² is selected from the group consisting of CH, and X³ is selected from the group consisting of CH; or X¹ is selected from the group consisting of X¹ is selected from the group consisting of CH, X² is selected from the group consisting of CH, and X³ is selected from the group consisting of N; or X¹ is selected from the group consisting of NR^{a}, X² is selected from the group consisting of CH, and X³ is selected from the group consisting of CH.

4. The compound, the stereoisomer thereof, or the pharmaceutically acceptable salt thereof according to any one of claims 1-3, wherein R¹ is selected from the group consisting of halogen, C₁₋₄ alkyl, C₁₋₄ alkoxy, C₃₋₆ cycloalkyl, and 3-6 membered heterocycloalkyl containing 1-3 heteroatoms independently selected from the group consisting of N, O, and S, wherein R¹ is optionally substituted with one or more groups selected from the group consisting of D, halogen, -OH, -OC₁₋₆ alkyl, -SH, -SC₁₋₆ alkyl, -NH₂, -NH(C₁₋₆ alkyl), and -N(C₁₋₆ alkyl)₂;
or R¹ is selected from the group consisting of halogen, C₁₋₄ alkyl, and C₃₋₆ cycloalkyl, wherein R¹ is optionally substituted with one or more groups selected from the group consisting of D and halogen;
or R¹ is selected from the group consisting of methyl, ethyl, propyl, cyclopropyl, cyclobutyl, cyclopentyl, cyclohexyl, oxetanyl, azetidinyl, thietanyl, tetrahydropyrrolyl, tetrahydrofuranyl, tetrahydrothienyl, piperidinyl, tetrahydropyranyl, tetrahydrothiopyranyl, morpholinyl, and piperazinyl, wherein R¹ is optionally substituted with one or more groups selected from the group consisting of D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂;
or R¹ is selected from the group consisting of chloro, methyl, ethyl, propyl, trifluoromethyl, 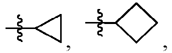 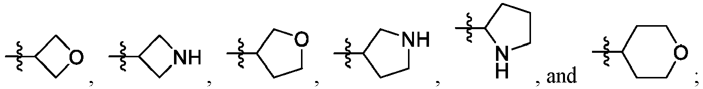
or R¹ is selected from the group consisting of chloro, methyl, ethyl, trifluoromethyl, and

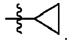

5. The compound, the stereoisomer thereof, or the pharmaceutically acceptable salt thereof according to any one of claims 1-4, wherein R^{a} is selected from the group consisting of H, F, Cl, methyl, ethyl, and propyl; or R^{a} is selected from the group consisting of H and methyl; or R^{a} is selected from the group consisting of H and F;
optionally, R^{a} and R¹ are linked to each other to form 5-6 membered heterocycloalkyl, 5-6 membered cycloalkenyl, phenyl, or 5-6 membered heterocycloalkenyl or 5-6 membered heteroaryl containing 1-3 heteroatoms independently selected from the group consisting of N, O, and S; or R ^{a} and R¹ are linked to each other to form 5-6 membered cycloalkenyl, or 5-6 membered heterocycloalkenyl containing 1-3 heteroatoms independently selected from the group consisting of N, O, and S; or R ^{a} and R¹ are linked to each other to form 5-6 membered heteroaryl containing 1-3 heteroatoms independently selected from the group consisting of N, O, and S; or R^{a} and R¹ are linked to each other to form cyclopentenyl, cyclohexenyl, dihydrofuranyl, furanyl, pyrrolyl, pyrazolyl, imidazolyl, thienyl, or thiazolyl; or R^{a} and R¹ are linked to each other to form cyclopentenyl, cyclohexenyl, or dihydrofuranyl.

6. The compound, the stereoisomer thereof, or the pharmaceutically acceptable salt thereof according to any one of claims 1-5, wherein R is selected from the group consisting of 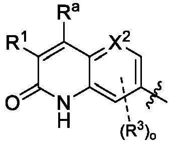 and 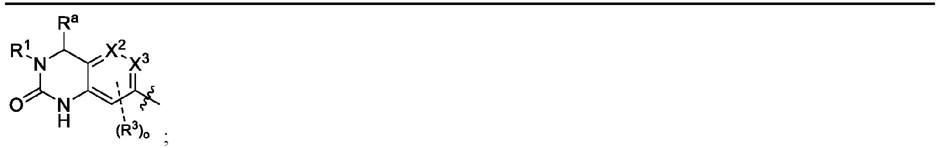 or R is selected from the group consisting of 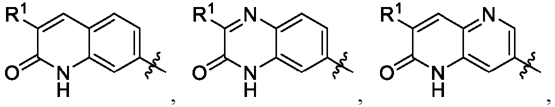 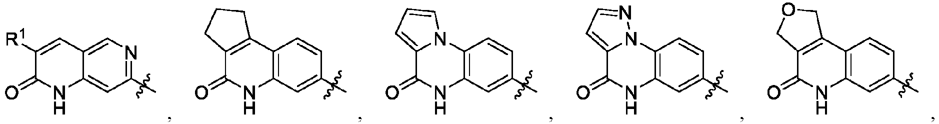 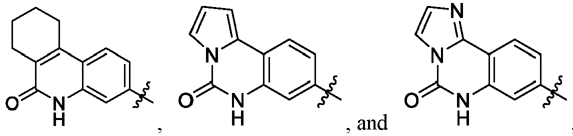 , and R is substituted with 0, 1, or 2 R³; or R is selected from the group consisting of

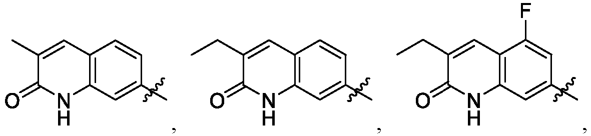

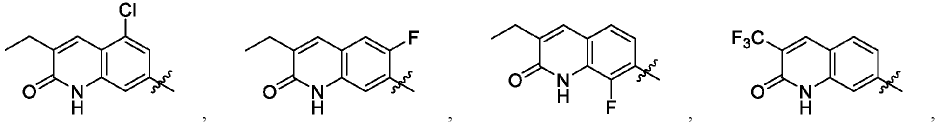

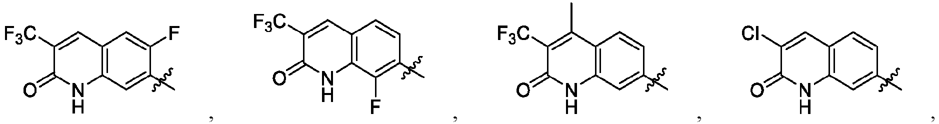

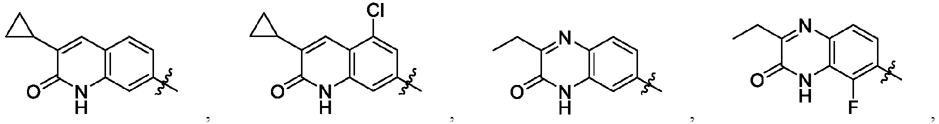

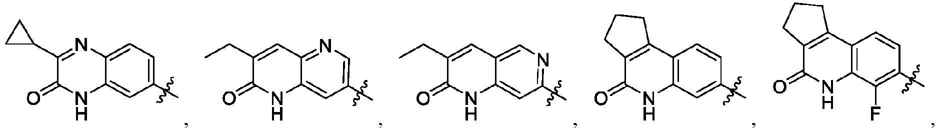

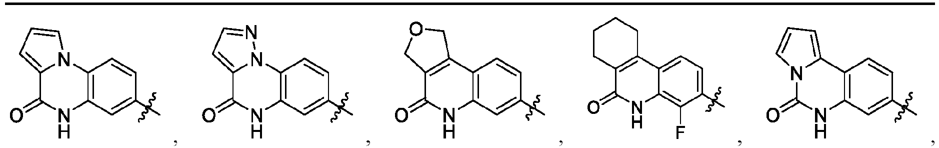

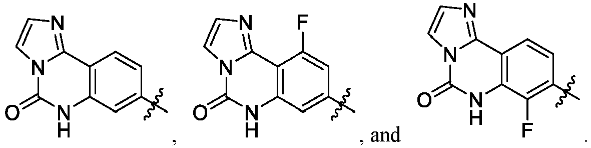

7. The compound, the stereoisomer thereof, or the pharmaceutically acceptable salt thereof according to any one of claims 1-6, wherein R² is selected from the group consisting of C₁₋₄ alkyl, -OH, -OC₁₋₄ alkyl, -OC₃₋₆ cycloalkyl, -SH, -SC₁₋₄ alkyl, -SC₃₋₆ cycloalkyl, -NH₂, -NH(C₁₋₄ alkyl), -NH(C₃₋₆ cycloalkyl), -NH(3-8 membered heterocycloalkyl), -N(C₁₋₄ alkyl)₂, -N(C₁₋₄ alkyl)(C₃₋₆ cycloalkyl), and -N(C₃₋₆ cycloalkyl)₂, wherein R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-8 membered heterocycloalkyl;
or R² is selected from the group consisting of -NH(C₁₋₄ alkyl), -NH(C₃₋₆ cycloalkyl), and -NH(3-8 membered heterocycloalkyl), wherein R² is optionally substituted with one or more groups selected from the group consisting of D, halogen, -OH, -OC₁₋₄ alkyl, -SH, -SC₁₋₄ alkyl, -NH₂, -NH(C₁₋₄ alkyl) or -N(C₁₋₄ alkyl)₂, C₃₋₆ cycloalkyl, and 3-6 membered heterocycloalkyl;
or R² is selected from the group consisting of -NHCH₃, -NHCH(CH₃)₂, 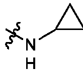 , -NHCD₃, -NHCH₂CH₃, -NHCH₂CF₃, 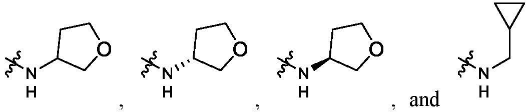 ; or R² is selected from the group consisting of -NHCH₃, 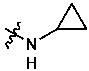 , and -NHCH₂CH₃.

8. The compound, the stereoisomer thereof, or the pharmaceutically acceptable salt thereof according to any one of claims 1-7, wherein R³ is selected from the group consisting of C₁₋₄ alkyl, D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂, wherein the C₁₋₄ alkyl, -OC₁₋₄ alkyl, -SC₁₋₄ alkyl, -NH(C₁₋₄ alkyl), or -N(C₁₋₄ alkyl)₂ is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂; or R³ is selected from the group consisting of methyl, ethyl, propyl, F, and Cl, wherein the methyl, ethyl, or propyl is optionally substituted with one or more groups selected from the group consisting of D, F, Cl, -OH, and -NH₂; or R³ is selected from the group consisting of methyl, F, and Cl;
optionally, R⁴ is selected from the group consisting of C₁₋₄ alkyl, D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂, wherein the C₁₋₄ alkyl, -OC₁₋₄ alkyl, -SC₁₋₄ alkyl, -NH(C₁₋₄ alkyl), or -N(C₁₋₄ alkyl)₂ is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂; or R⁴ is selected from the group consisting of methyl, ethyl, propyl, F, and Cl, wherein the methyl, ethyl, or propyl is optionally substituted with one or more groups selected from the group consisting of D, F, Cl, -OH, and -NH₂;
optionally, R⁵ is selected from the group consisting of C₁₋₄ alkyl, D, F, Cl, Br, -OH, -OC₁₋₄ alkyl, -SH, -SC₁₋₄ alkyl, -NH₂, -NH(C₁₋₄ alkyl), and -N(C₁₋₄ alkyl)₂, wherein the C₁₋₄ alkyl, -OC₁₋₄ alkyl, -SC₁₋₄ alkyl, -NH(C₁₋₄ alkyl), or -N(C₁₋₄ alkyl)₂ is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂; or R⁵ is selected from the group consisting of methyl, ethyl, propyl, F, and Cl, wherein the methyl, ethyl, or propyl is optionally substituted with one or more groups selected from the group consisting of D, F, Cl, -OH, and -NH₂.

9. The compound, the stereoisomer thereof, or the pharmaceutically acceptable salt thereof according to any one of claims 1-8, wherein o is selected from the group consisting of 0, 1, and 2; or o is selected from the group consisting of 0 and 1;
optionally, p is selected from the group consisting of 0 and 1; or p is selected from the group consisting of 0; optionally, q is selected from the group consisting of 0 and 1; or q is selected from the group consisting of 0.

10. The compound, the stereoisomer thereof, or the pharmaceutically acceptable salt thereof according to any one of claims 1-9, wherein L is selected from the group consisting of -NH- and -CH₂-, wherein L is optionally substituted with one or more groups selected from the group consisting of methyl, D, and F;
or L is selected from the group consisting of -CH₂-.

11. The compound, the stereoisomer thereof, or the pharmaceutically acceptable salt thereof according to any one of claims 1-10, wherein ring A is selected from the group consisting of 3-6 membered heterocycloalkyl, wherein ring A optionally contains 1-3 heteroatoms independently selected from the group consisting of N and O in addition to the N atom linked to L, and ring A is optionally substituted with one or more groups selected from the group consisting of D, F, Cl, -OH, -C₁₋₄ alkyl, -OC₁₋₄ alkyl, -SH, -SC₁₋₄ alkyl, -NH₂, -NH(C₁₋₄ alkyl), and -N(C₁₋₄ alkyl)₂; or ring A is optionally substituted with one or more groups selected from the group consisting of -C₁₋₃ alkylene-OC₁₋₄ alkyl, -C₁₋₃ alkylene-NH(C₁₋₄ alkyl) and -C₁₋₃ alkylene-N(C₁₋₄ alkyl)₂;
or ring A is selected from the group consisting of azetidinyl, tetrahydropyrrolyl, piperidinyl, diazetidinyl, imidazolinyl, piperazinyl, oxazolinyl, and morpholinyl, wherein ring A is optionally substituted with one or more groups selected from the group consisting of D, F, Cl, -OH, -OC₁₋₄ alkyl, -SH, -SC₁₋₄ alkyl, -NH₂, -NH(C₁₋₄ alkyl), and -N(C₁₋₄ alkyl)₂; or ring A is optionally substituted with one or more -C₁₋₄ alkyl; or ring A is optionally substituted with one or more groups selected from the group consisting of -C₁₋₃ alkylene-OC₁₋₄ alkyl;
or ring A is selected from the group consisting of 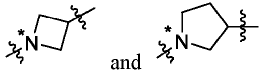 , wherein ring A is optionally substituted with one or more groups selected from the group consisting of -OH and -C₁₋₄ alkyl; or ring A is optionally substituted with one or more groups selected from the group consisting of -OH, -C₁₋₄ alkyl, and -C₁₋₃ alkylene-OC₁₋₄ alkyl;
or ring A is selected from the group consisting of 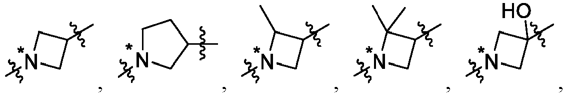 and 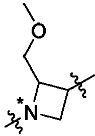 , wherein the nitrogen atom marked with * is linked to L on one side and to the structural fragment on the other side.

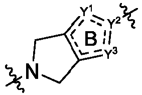

12. The compound, the stereoisomer thereof, or the pharmaceutically acceptable salt thereof according to any one of claims 1-11, wherein ring B is selected from the group consisting of an aromatic ring, wherein the aromatic ring contains 1, 2, or 3 heteroatoms selected from the group consisting of N, O, and S; or ring B is selected from the group consisting of a 5-membered heteroaromatic ring, wherein the 5-membered heteroaromatic ring contains 1, 2, or 3 heteroatoms selected from the group consisting of N, O, and S;
or ring B is selected from the group consisting of a pyrazole ring, a pyrrole ring, a thiazole ring, an oxazole ring, an isoxazole ring, a furan ring, an imidazole ring, and a thiophene ring;
or ring B is selected from the group consisting of 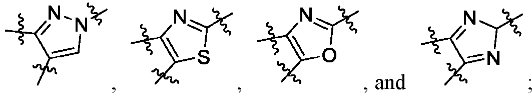 or
ring B is selected from the group consisting of

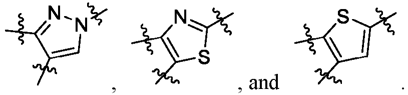

13. A compound of the following formula, a stereoisomer thereof, or a pharmaceutically acceptable salt thereof:

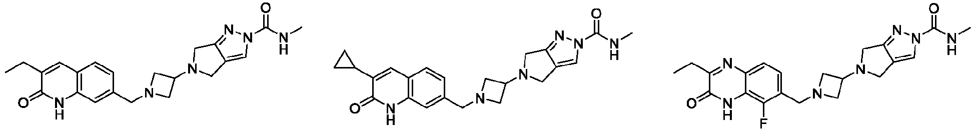

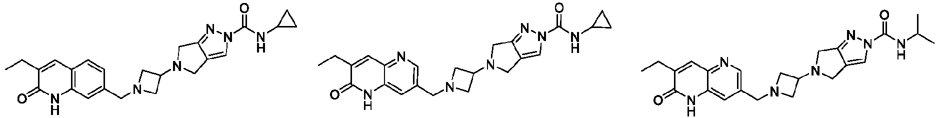

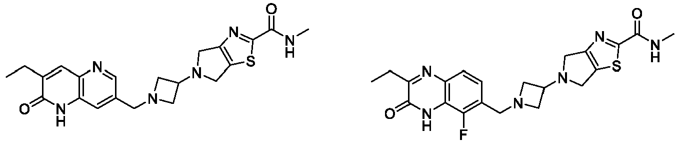

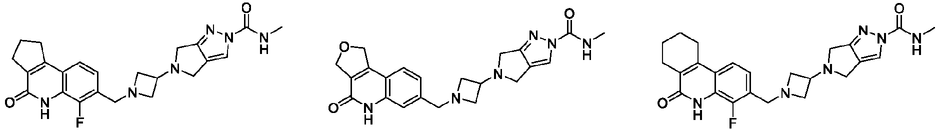

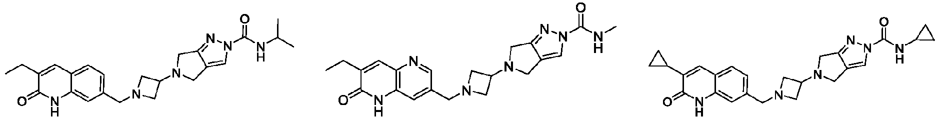

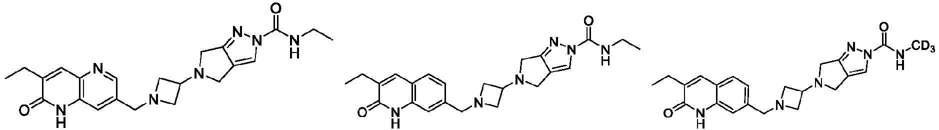

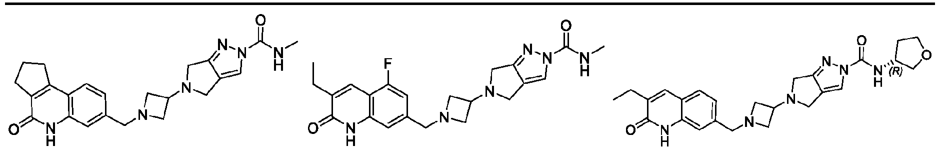

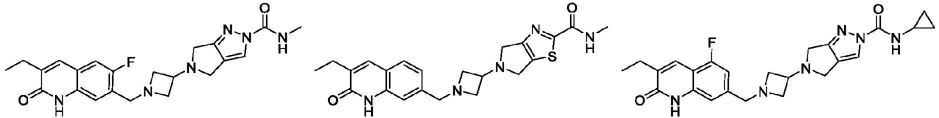

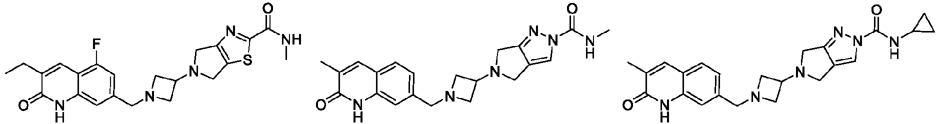

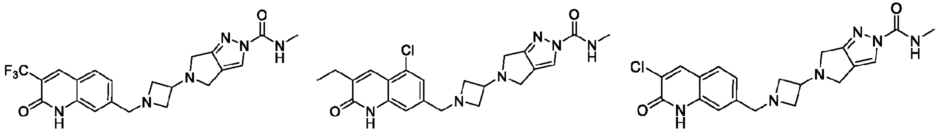

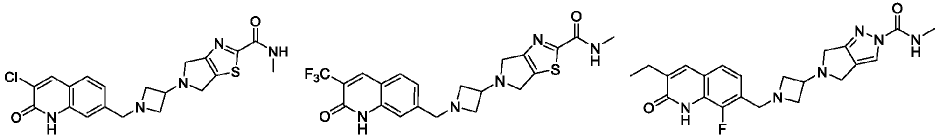

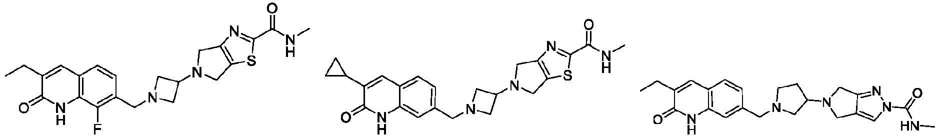

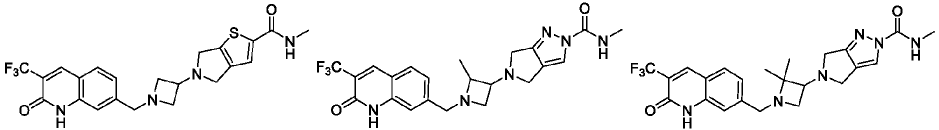

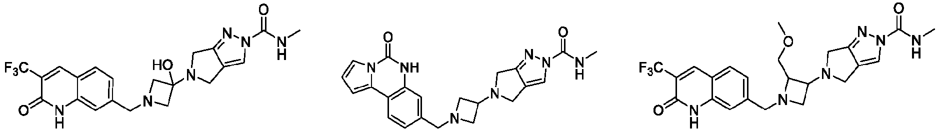

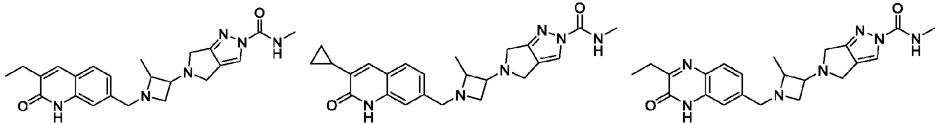

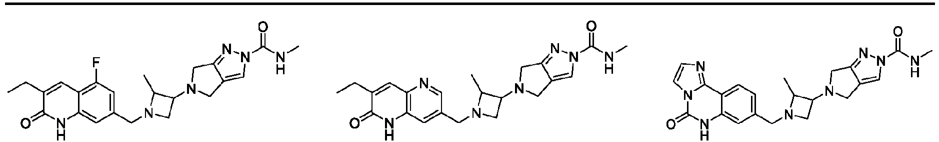

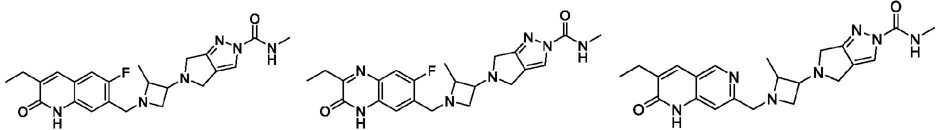

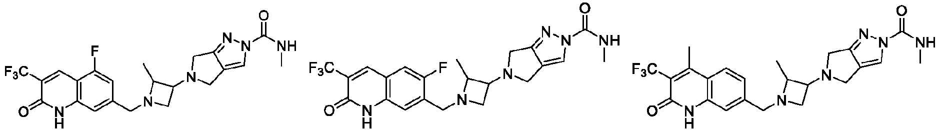

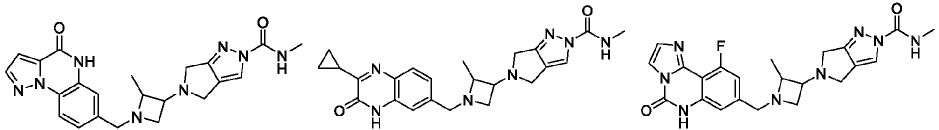

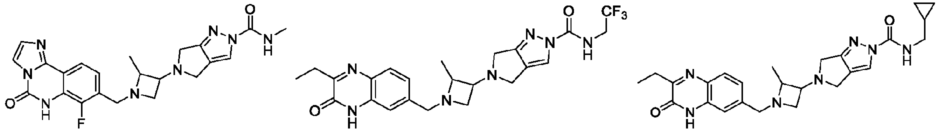

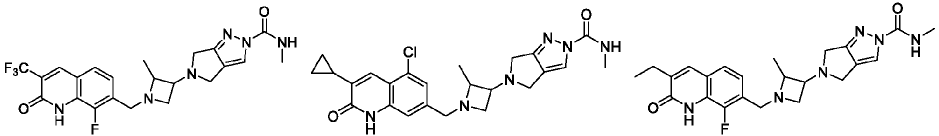

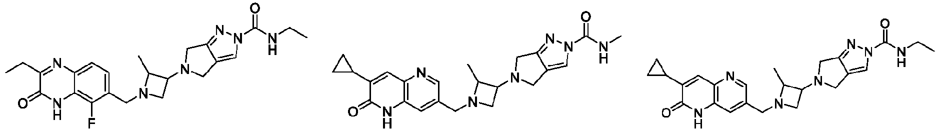

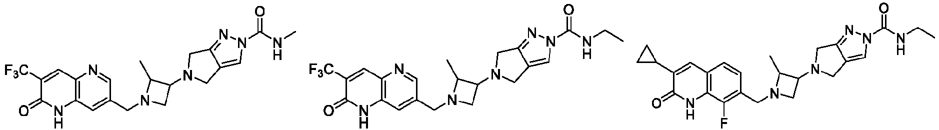

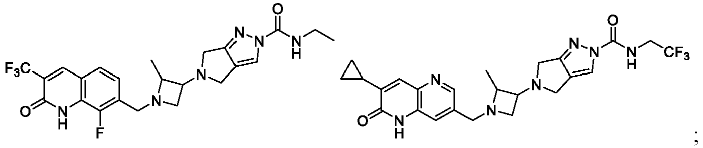

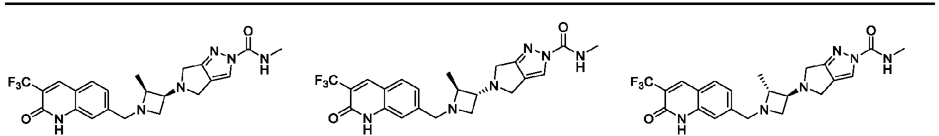

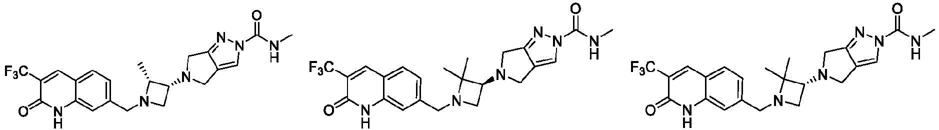

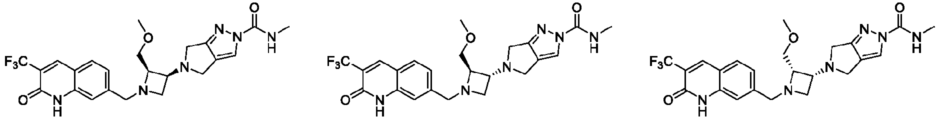

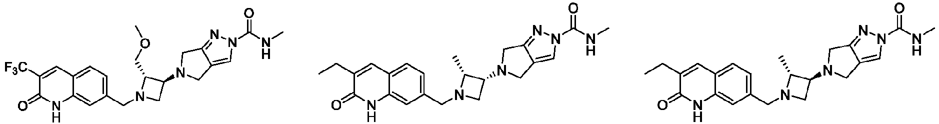

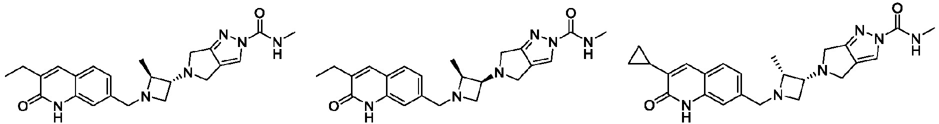

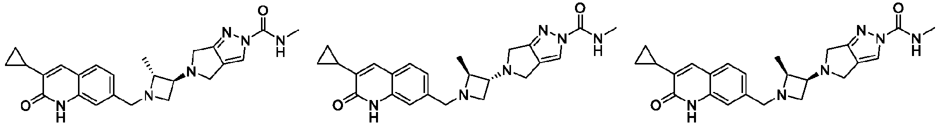

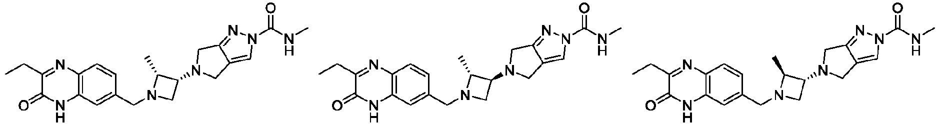

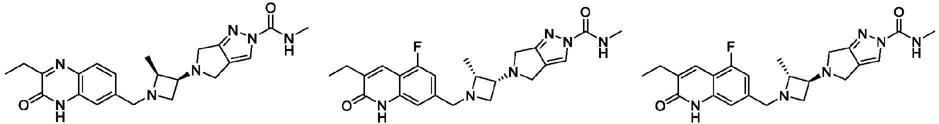

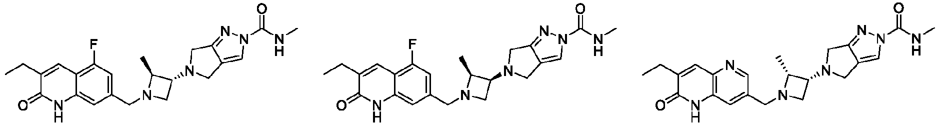

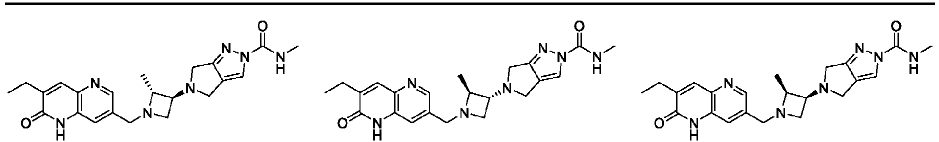

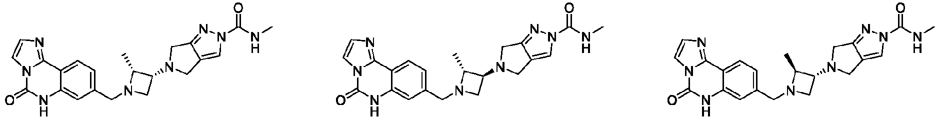

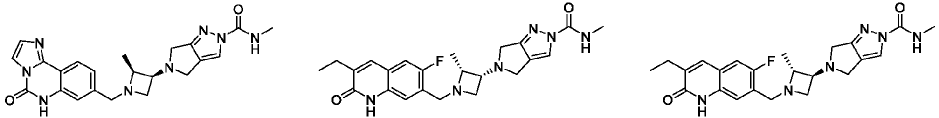

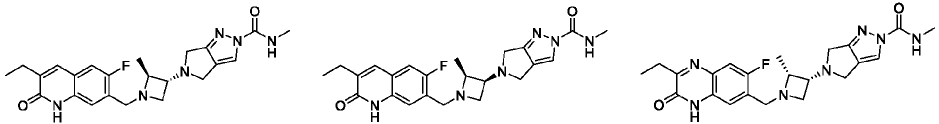

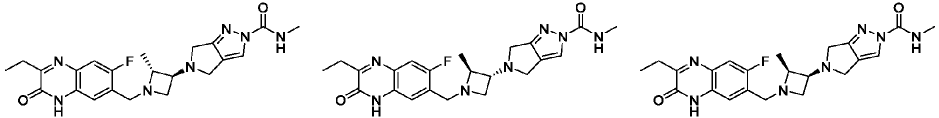

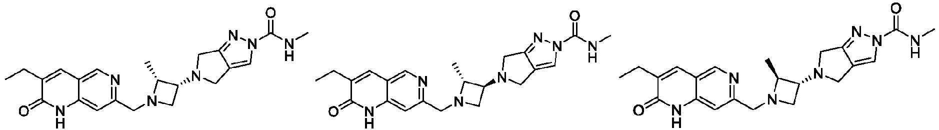

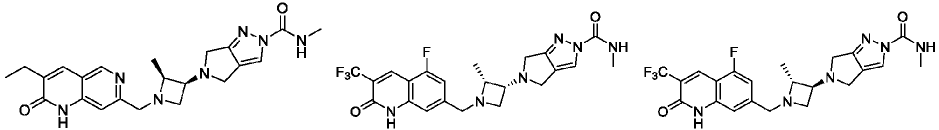

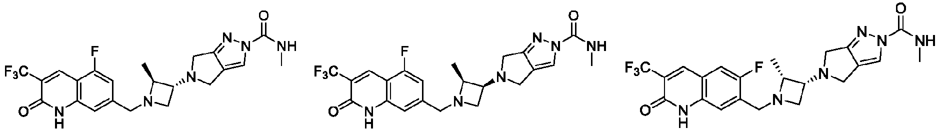

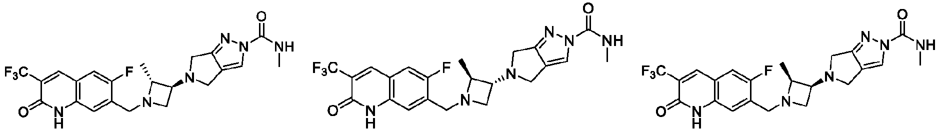

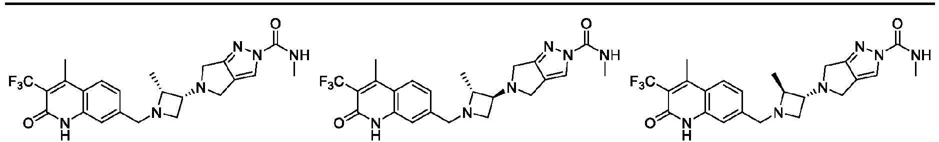

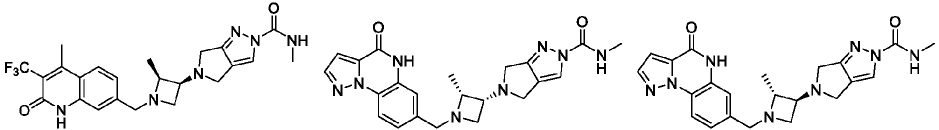

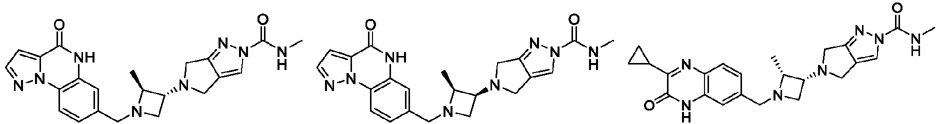

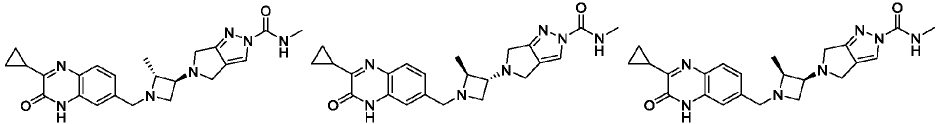

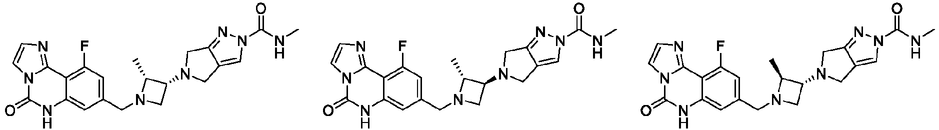

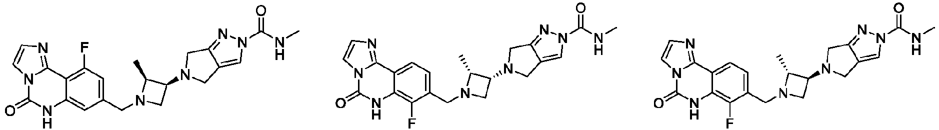

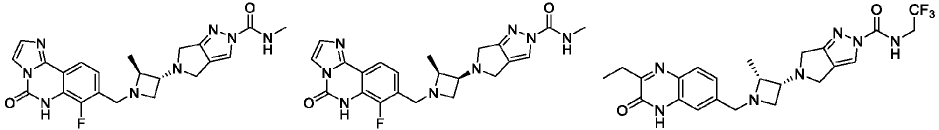

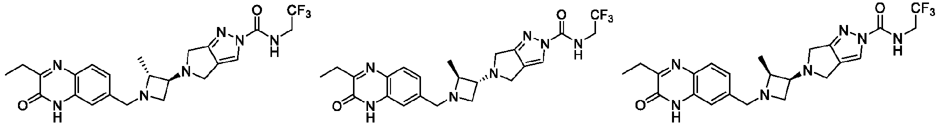

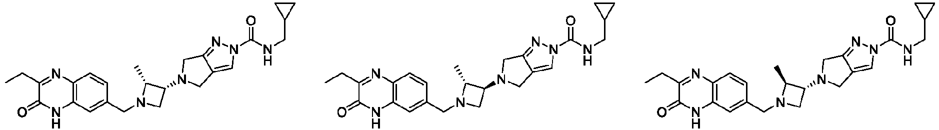

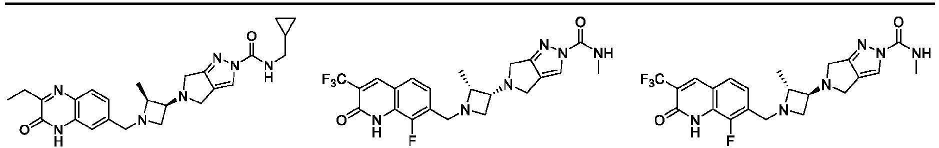

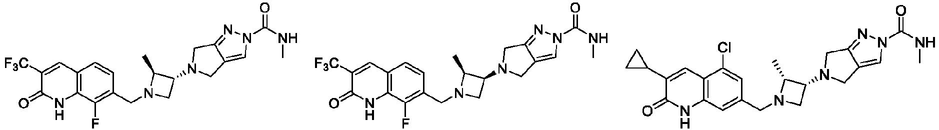

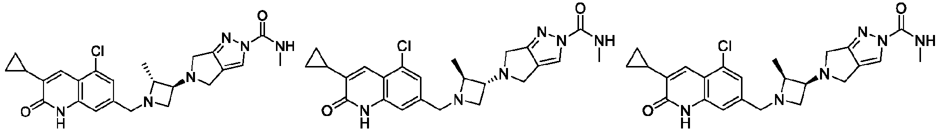

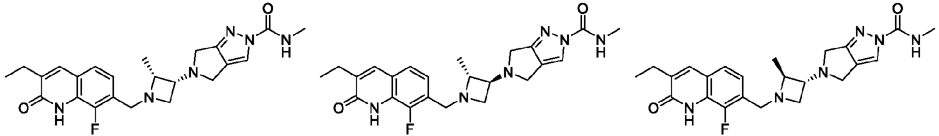

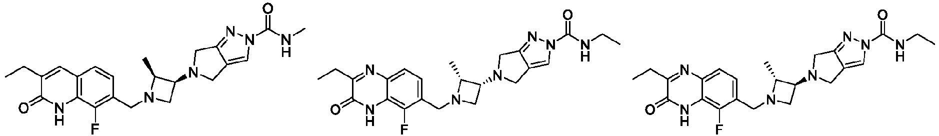

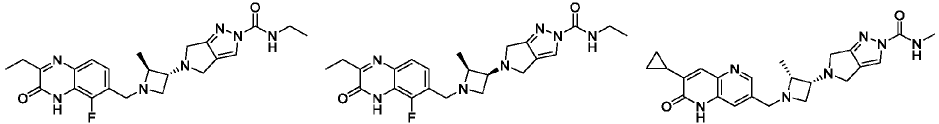

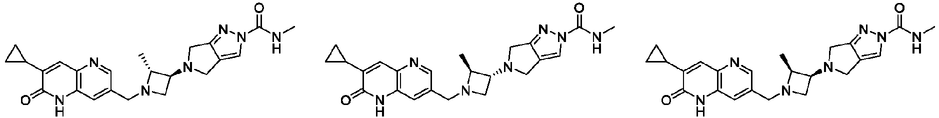

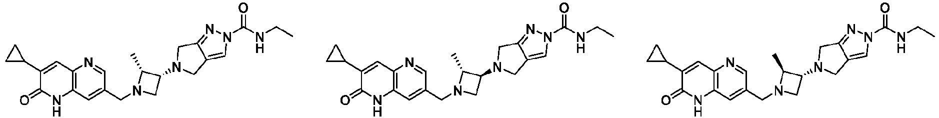

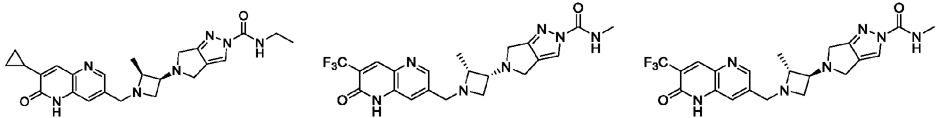

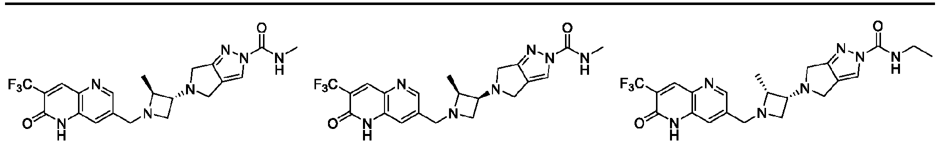

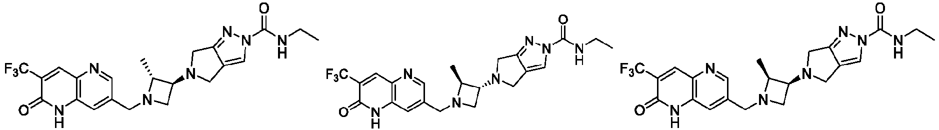

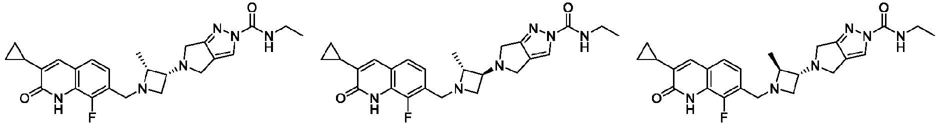

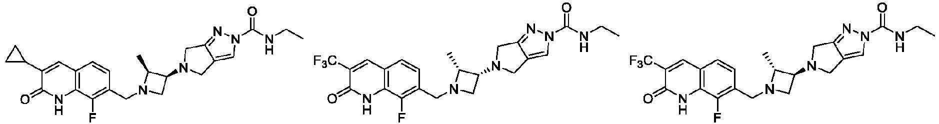

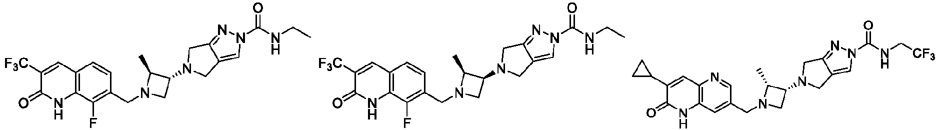

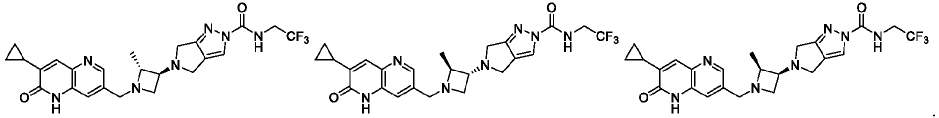

14. A pharmaceutical composition, comprising the compound, the stereoisomer thereof, or the pharmaceutically acceptable salt thereof according to any one of claims 1-13.

15. Use of the compound, the stereoisomer thereof, or the pharmaceutically acceptable salt thereof according to any one of claims 1-13, or the pharmaceutical composition according to claim 14 for preparing a medicament for treating a PARP1-related disease, wherein optionally, the PARP1-related disease is selected from the group consisting of a tumor or a cancer; optionally, the cancer is selected from the group consisting of breast cancer, ovarian cancer, colon cancer, pancreatic cancer, and prostate cancer.
